(19) **Europäisches Patentamt** / **European Patent Office** / **Office européen des brevets**

(11) **EP 2 358 218 B1**

(12) **EUROPEAN PATENT SPECIFICATION**

(45) Date of publication and mention
of the grant of the patent:
**12.04.2017 Bulletin 2017/15**

(21) Application number: **09828168.6**

(22) Date of filing: **18.11.2009**

(51) Int Cl.:
*A23L 33/20* (2016.01)     *A23L 33/24* (2016.01)

(86) International application number:
**PCT/US2009/064988**

(87) International publication number:
**WO 2010/059725 (27.05.2010 Gazette 2010/21)**

(54) **METHODS AND COMPOSITIONS FOR WEIGHT MANAGEMENT AND FOR IMPROVING GLYCEMIC CONTROL**

VERFAHREN UND ZUSAMMENSETZUNGEN FÜR GEWICHTSMANAGEMENT UND VERBESSERTE GLYKÄMISCHE KONTROLLE

MÉTHODES ET COMPOSITIONS POUR LA GESTION DU POIDS ET POUR AMÉLIORER LE CONTRÔLE GLYCÉMIQUE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **11.02.2009 US 151745 P**
**07.04.2009 US 167291 P**
**18.11.2008 US 115759 P**

(43) Date of publication of application:
**24.08.2011 Bulletin 2011/34**

(73) Proprietor: **Gelesis LLC**
**Boston MA 02116 (US)**

(72) Inventors:
• **SANNINO, Alessandro**
**73100 Lecce 25 (IT)**
• **AMBROSIO, Luigi**
**80044 Ottaviano (Napoli) (IT)**
• **RON, Eyal**
**Lexington MA 02420 (US)**
• **ZOHAR, Yishai**
**Boston MA 02116 (US)**
• **DEMITRI, Christian**
**73010 San Pietro in Lama (IT)**

(74) Representative: **Coles, Andrea Birgit et al**
**Kilburn & Strode LLP**
**20 Red Lion Street**
**London WC1R 4PJ (GB)**

(56) References cited:
**WO-A2-2009/021701     US-A- 5 415 864**
**US-A1- 2001 006 677     US-A1- 2004 192 582**
**US-A1- 2005 100 603     US-A1- 2005 118 326**
**US-A1- 2006 102 483     US-A1- 2008 095 911**
**US-A1- 2008 227 944**

Remarks:
The file contains technical information submitted after the application was filed and not included in this specification

**Description**

**FIELD OF THE INVENTION**

The present invention is in the field of prevention and treatment of obesity, management of weight and diabetes and general wellness, including gut and heart health.

**BACKGROUND OF THE INVENTION**

Public health efforts and current anti-obesity agents have not controlled the obesity epidemic. This disorder is increasingly prevalent in industrialized nations because of the abundance of food and the reduced activity levels that accompany the movement of populations from rural to urban settings. Obesity is loosely defined as an excess of body fat over that needed to maintain health.

Obesity is a condition in which excess body fat has accumulated to such an extent that health may be negatively affected. (World Health Organization (2000). Technical report series 894: Obesity: Preventing and managing the global epidemic). It is commonly defined as a Body Mass Index (BMI = weight divided by height squared) of 30 kg/m$^2$ or higher. Overweight is distinguished and defined as a BMI between 25-29.9 kg/m$^2$ (Obes Res. 1998 Sep; 6 Suppl 2:51S-209S. Clinical Guidelines on the Identification, Evaluation, and Treatment of Overweight and Obesity in Adults--The Evidence Report. National Institutes of Health).

Excessive body weight is associated with various diseases, particularly cardiovascular diseases, diabetes mellitus type 2, obstructive sleep apnea, certain types of cancer, and osteoarthritis (National Heart, Lung, and Blood Institute. Clinical Guidelines on the Identification, Evaluation, and Treatment of Overweight and Obesity in Adults NIH Publication No. 98-4083 September 1998 National Institutes of Health). As a result, obesity has been found to reduce life expectancy. The primary treatment for obesity is dieting and physical exercise. If diet and exercise fails, anti-obesity drugs and bariatric surgery may be recommended in severe cases (National Institute for Health and Clinical Excellence. Clinical Guideline 43: Obesity: The prevention, identification, assessment and management of overweight and obesity in adults and children. London, 2006).

The pathogenesis of obesity is multi-factorial and includes the control of feeding behavior, mechanisms of fat storage, the components of energy intake and expenditure, and genetic and psychological influences. Likewise, the treatment of obesity is generally multi-factorial. Unfortunately, the mechanisms of fat storage and genetic influences are not, generally speaking, amenable to treatment. Moreover, the control of feeding behavior and psychological influences require prolonged treatment. Although the components of energy intake and expenditure are treatable, many obese individuals are resistant to or incapable of engaging in activities which significantly increase their energy expenditure. Therefore, controlling energy intake is an attractive approach for the treatment of obesity.

Inclusion of low energy density foods with significant volume results in reduction of overall caloric intake in a single meal (Bell et al. Am J Clin Nutr, 67:412-20, 1998; Rolls et. al. Am J Clin Nutr, 70: 448-455, 1999). Given the success in reducing caloric intake in one meal, a longer term approach of including low energy density foods in diets has been demonstrated to increase long-term weight loss (Ello-Martin et. al Am J Clin Nutr , 85:1465-7, 2007; Greene et. al. Obesity, 14: 1795-1801,2006). The concept of eating low energy foods to induce satiety by taking up stomach volume has sometimes been called the "volumetrics diet" and non-technical books have been written for those wishing to follow this approach (see Barbara Rolls, "Volumetrics Eating Plan" Harper Collins, 2007). The volumetrics diet has suffered from limited food choices, leading to poor compliance.

The sensation of satiety as a means of suppressing of appetite is known in the art and is linked to both obesity treatment and effecting weight loss. For example, U.S. Patent No. 5,336,486 to Acharya et al. describes the false sensation of satiety induced by filling the stomach with heavy digestible vegetable fibers. Consuming large amounts of fiber, however, requires the patient to expel large quantities of fiber which can cause gastrointestinal discomfort. Others are unable tolerate such high volumes of fiber for other reasons such as flatulence as a result of the colon bacteria's digestion of fiber. To diminish the discomfort caused by a full stomach retaining vegetable fibers for a relatively longer duration, diet recipes based on vegetable fibers have been refined by the addition of easily digestible products with low number of calories. U.S. Patent No's. 5,063,073 to Kratochvil; 5,654,028 to Christensen et al.*;* and 6,426,077 to Grace et al.*;* U.S. Patent No's. 5,405,616 and 6,103,269 to Wounderlich et al. describe a material composed of gelatin or collagen hydrolysate, one or more active agents, and one or more excipients (i.e. plasticizers, odorants, etc.). Low calorie products for controlling body weight can be obtained by using collagenic biopolymers, such as soluble collagen, gelatin or collagen hydrolysate. (See U.S. Patent No's. 5,100,688; 5,211,976; 5,219,599; 5,665,234; and 5,665,419). Commercial products, such as "Dietary Supplement -CALORAD®", produced by EYI-Essentially Yours Industries, Inc.-USA, have been used for weight-loss control and as a muscular stimulant, as well as for osteoporosis and for arthritis treatment.

Increased elasticity (G') of foods has been linked to increased satiety and therefore could be used for weight management [I.T. Norton, W.J. Frith and S. Ablett; Fluid gels, mixed fluid gels and satiety; Food Hydrocolloids; Volume 20, Issues 2-3, March-May 2006, Pages 229-239]. This study and others demonstrated that foods with higher elastic response created higher levels of satiety. In a similar manner, viscosity was associated with satiety as well; satiety and fullness were higher for high- compared with low-viscosity meals. [Marciani, L., Gowland, P. A., Spiller, R. C., Manoj, P., Moore, R. J. Young, P., & Fillery-Travis, A. J. (2001) Effect of meal viscosity and nutrients on satiety, intragastric dilution, and emptying assessed by MRI. American Journal of Physiology Gastrointestinology and Liver Physiology, 280: G1227-G1233]. Furthermore, increased viscosity was correlated with short-term gut hormone response, implying the importance of food structure in the modulation of postprandial satiety-related physiology [Juvonen, K. R. et al. Viscosity of Oat Bran-Enriched Beverages Influences Gastrointestinal Hormonal Responses in Healthy Humans; Journal of Nutrition, Vol. 139, No. 3, 461-466, 2009]. In addition, satiety was linked to gastric emptying rates in which higher viscosity was related to slower emptying times and increased satiation [Hlebowicz, J. et al. Effect of commercial breakfast fiber cereals compared with corn flakes on postprandial blood glucose, gastric emptying and satiety in healthy subjects: a randomized blinded crossover trial; Nutrition Journal 2007, 6:22

The obesity rate has been climbing steadily over the last several years. Carrying extra weight increases the chances of developing serious health problems, such as heart disease, stroke, certain kinds of cancers, as well as diabetes. The incidence of Type 2 diabetes in our country is increasing concurrently with the rise in obesity. The American Diabetes Association estimates about 21 million people have diabetes, with another 54 million people diagnosed with pre-diabetes. Pre-diabetes is a condition in which fasting blood glucose levels are elevated, but not yet to the level indicated for Type 2 diabetes.

Type 2 diabetes is associated with insulin resistance. Insulin is an important hormone that delivers glucose (sugar) to our cells. When a person is overweight, the cells in the body become less sensitive to the insulin that is released from the pancreas. There is some evidence that fat cells are more resistant to insulin than muscle cells. If a person has more fat cells than muscle cells, then the insulin becomes less effective overall, and glucose remains circulating in the blood instead of being taken in to the cells to be used as energy.

Glycemic control is a medical term referring to the typical levels of blood sugar (glucose) in a person with diabetes mellitus. Much evidence suggests that many of the long-term complications of diabetes, especially the microvascular complications, result from many years of hyperglycemia (elevated levels of glucose in the blood). Good glycemic control, in the sense of a "target" for treatment, has become an important goal of diabetes care, although recent research suggests that the complications of diabetes may be caused by genetic factors[Tarnow, L; Groop; Hadjadj; Kazeem; Cambien; Marre; Forsblom; Parving et al. (2008). "European rational approach for the genetics of diabetic complications-EURAGEDIC: patient populations and strategy". Nephrology, dialysis, transplantation 23 (1): 161-8] or, in type 1 diabetics, by the continuing effects of the autoimmune disease which first caused the pancreas to lose its insulin-producing ability.[Adams, D.D. (2008). "Autoimmune destruction of pericytes as the cause of diabetic retinopathy". Clinical ophthalmology 2 (2): 295-8].

"Perfect glycemic control" would mean that glucose levels were always normal (70-130 mg/dl, or 3.9-7.2 mmol/L) and indistinguishable from a person without diabetes. In reality, because of the imperfections of treatment measures, even "good glycemic control" describes blood glucose levels that average somewhat higher than normal much of the time. In addition, one survey of type 2 diabetics found that they rated the harm to their quality of life from intensive interventions to control their blood sugar to be just as severe as the harm resulting from intermediate levels of diabetic complications.[Huang, ES; Brown; Ewigman; Foley; Meltzer (2007). "Patient perceptions of quality of life with diabetes-related complications and treatments". Diabetes care 30 (10): 2478-83].

There have been several attempts to control the absorption of carbohydrates especially after meals. Emerging data indicates that modulation of postprandial plasma glucose levels plays an important role in overall glycemic control. Early in the development of type 2 diabetes, the initial burst of insulin release in response to food intake is compromised, allowing postprandial hyperglycemia to develop. Meal-associated hyperglycemia further contributes to increase insulin resistance and decrease insulin production. Evidence of a strong correlation between high postprandial glycemic levels and the development of vascular complications underscores the significance of treating mealtime glycemia.

One method to measure the absorption rate of carbohydrates is defined by the Glycemic Index Scale [http://www.glycemicindex.com/]. Heaton et al. has reported that glycemic index is controlled by differences in particle sizes of wheat, maize, and oat (e,g, Heaton K W, Marcus S N, Emmett P M, Bolton C H: Particle size of wheat, maize, and oat test meals: effects on plasma glucose and insulin responses and on the rate of starch digestion in vitro, Am. J. Clin. Nutr., Vol. 47, 675-682 (1988)). Moreover, it has been known that the glycemic index of a food depends on the form in which it is presented. For example, the glycemic index of boiled rice is lower than that of powdered rice; the glycemic index of a whole apple is lower than that of a "pureed" apple (see, for example, Kunihiro Doi and Keisuke Tsuji Eds., Shokumotsu Sen-i (Dietary Fiber), p.412-420 (Asakura-shoten, Tokyo, 1997)). In addition, methods utilizing a polysaccharide with gel formation ability, such as guar gum, pectin, or glucomannan have been known. These are methods for lowering postprandial glucose levels and improving glycemic control. The use of certain polysaccharides in foods extends the endogastric residence time of glucose due to gel formation (see, for example, "Kagaku to Seibutsu (Chemistry and Biology)," Vol. 18, p95-105, 1980).

U.S. Pat. No. 7,601,705 and references within, teaches controlled induced viscosity fiber system for blunting the postprandial glycemic response, comprises neutral soluble fiber source such as guar gum, pectin, locust bean gum, methylcellulose, and lightly hydrolyzed starch. The invention also describes a method of incorporating soluble fiber into a liquid product without the typical negative organoleptic or physical stability issues. The invention also relates to a method of inducing the feeling of fullness and satiety by feeding the induced viscosity fiber system.

U.S. Pat. No. 5,776,887 teaches a nutritional product having controlled absorption of carbohydrate. The product taught in U.S. Pat. No. 5,776,887 comprises protein, fat, carbohydrate, fiber and disaccharides. U.S. Pat. No. 5,695,803 teaches nutritional products containing acid treated starches to improve the insulin response of foods.

Absorbent materials for water and aqueous media, including fluids secreted by the human body, are well known in the literature. These materials are typically polymer-based and are produced in the form of powders, granules, microparticles, fibers or films. Upon contact with an aqueous medium, these edible polymer hydrogels swell by absorbing the liquid phase into their structure without dissolving. When the swelling reaches equilibrium, a gel, typically referred to as a "hydrogel", is formed. Hydrogels capable of absorbing a quantity of water in excess of 95% of their overall weight are defined as "superabsorbent" (SAP).

Chen Jun et al. in "Gastric retention properties of superporous hydrogel composite" J. Controlled Release, 64, 39-51, 2000, and in U.S. Pat. No. 6,018,033 and Park K. et al. in U.S. Pat. No. 5,750,585 and U.S. Pat. No. 6,271,278 disclose that hydrogels obtained by grafting and cross-linking a mixture of acrylic acid, acrylamide, potassium salt of 3-sulfopropyl acrylate and N,N'-methylenebisacrylamide in the presence of AcDi-Sol® (small cross-linked polysaccharide), swell in the stomach after oral administration and can be used as an auxiliary in diet control. Burnett D.R. et al. in US 2004/192582 discloses an ingestible formulation for transient, noninvasive reduction of gastric volume comprising of polymeric formulations capable of being retained in the stomach for a certain period of time followed by rapid degradation upon entering an small intestine. The concept of using polymers for taking up stomach volume to induce satiation is also disclosed by others (see, for example, US Patent Application Publication Nos. 20050245957 and 20060142794; and PCT Published Application Nos. WO 2006/047882 and WO 2006/070337).

Other nonbiodegradable polymers may swell in the stomach and act as stomach-fillers. Yet because these polymers are non-degradable, they will increase the risk of impaction, defined as the presence of putty-like or hardened feces in the rectum or sigmoid (syndrome of moderate toxemia, an absence of fecal movements and straining). In certain cases, polymers can act as laxatives - another undesirable affect. Laxatives (or purgatives) are foods, compounds, or drugs taken to induce bowel movements or to loosen the stool, and are most often taken to treat constipation. Certain types of laxatives are bulking agents that produce bulkier stool and retain more water. Additionally, these laxatives may form an emollient gel, making it easier for peristaltic action to propel stool along the gastrointestinal system. These bulking agents include dietary fiber and synthetic hydrogels such as polyacrylic acids, including calcium polycarbophyl (such as Noveon AA-1 CA-1 or CA-2, Lubrizol, OH). Some products containing this type of polymers are: Equalactin™, FiberCon™, Fiber-Lax™, FiberNorm™, Konsyl™, Mitrolan™; these all recommend a dose of about 1-1.5. g per administration. Other products contain similar non-degradable polymers, such as cross-linked polyacrylic acid hydrogel homopolymers (such as Carbopol 971P, 71G, 974P, Lubrizol, OH).

Both natural non-digested fibers and the synthetic hydrogels absorb water and act as stomach fillers because of the bulking effect, and yet they do not degrade in the GI tract.

WO 2009/021701 discloses edible polymer hydrogels, which are disclosed as useful in methods for treating obesity or other medical disorders or diseases where calorie restriction has a health benefit.

Insertion and inflation of balloons into the small intestine of rats resulted in decreased fluid intake, but also appeared to evoke a painful reaction when the balloons were inflated past a certain point (Bardos, Behav Neurosci., 111:834-844, 1997). Likewise, balloon insertion into the small intestine was perceived negatively by rats as shown in a taste aversion paradigm (Bardos, Physiol Behav., 74:407-413, 2001). Use of a balloon in people would be highly invasive and difficult to insert and maintain. In addition, an inserted balloon would result in continual stimulation of the small intestine, producing habituation and adaptation, as well as pain, which does not occur with the episodic stimulation produced naturally by food.

## SUMMARY OF THE INVENTION

The present invention provides compositions, foods and methods for enhancing satiety, for lowering the amount of food intake, and for improving glycemic control.

In one embodiment, the invention provides an edible polymer hydrogel that swells in the stomach and small intestine to provide or enhance satiety by mechanical stimuli and/or increased viscosity.

In one embodiment, the invention provides an edible polymer hydrogel formulation which swells in the small intestine, but not in the stomach.

In one embodiment, the edible polymer hydrogel swells in the stomach, collapses and enters the small intestine, swells in the small intestine and is degraded in the colon.

In one embodiment, the invention provides methods of inducing weight loss, maintaining weight or enhancing or providing glycemic control in a subject, comprising the step of orally administering prior to or with a meal to the subject an edible polymer hydrogel which swells in the stomach and/or the small intestine. The edible polymer hydrogel is preferably administered in an amount sufficient to slow gastric emptying and absorption of carbohydrates and fats in the small intestine.

In one embodiment, the invention provides modified foods and foodstuffs which comprise an edible polymer hydrogel and which have a reduced energy density compared to conventional or unmodified foods.

In one embodiment, the invention provides a food comprising an edible polymer hydrogel in which the edible polymer hydrogel is swollen in the food. In this embodiment, the edible polymer hydrogel is added as an ingredient during food preparation in the swollen state, or it is added in a dehydrated state and then swells during food preparation. In another embodiment, the edible polymer hydrogel is formed during food preparation. In this embodiment, the polymer(s) and cross-linking agent components of the edible polymer hydrogel are added to one or more other ingredients of the food during food preparation, resulting in formation of the edible polymer hydrogel.

In an embodiment, the invention provides a food comprising an edible polymer hydrogel, in which the edible polymer hydrogel is present in the food in a dehydrated state. In this embodiment, the edible polymer hydrogel swells in the stomach and/or small intestine following ingestion.

In an embodiment, the invention provides a method of preparing a food or foodstuff comprising an edible polymer hydrogel, comprising contacting a polymer with an cross-linking agent in the presence of one or more additional ingredients, thereby forming the food or foodstuff comprising the edible polymer hydrogel.

In an embodiment, the invention provides an edible polymer hydrogel coated with a moisture barrier. The moisture barrier can comprise, for example, proteins, fats, sugars or a combination thereof. Preferably, the edible polymer hydrogel is in the form of particles and the particles are coated with the moisture barrier.

In an embodiment, the invention provides an edible polymer hydrogel composition which is coated with an enteric coating. The edible polymer hydrogel is preferably present in the composition in the dehydrated state and the enteric coating is sufficient to inhibit swelling of the edible polymer hydrogel in the stomach. Degradation of the enteric coating in the small intestine then leads to swelling of the edible polymer hydrogel in the small intestine.

In an embodiment, the invention provides a food or beverage comprising an anionic edible polymer hydrogel and a pH reducing agent. The pH reducing agent is preferably capable of reducing the pH of the food or beverage to a pH at which swelling of the edible polymer hydrogel is inhibited or delayed.

In an embodiment, the invention provides a beverage comprising an edible polymer hydrogel and gas bubbles or one or more agents which induce effervescence. The effervescence is preferably capable of inhibiting or delaying the swelling of the edible polymer hydrogel.

In an embodiment, the invention provides an edible polymer hydrogel in a form which can be used in cooking.

## Brief Description of the Drawings

Figure 1 is a schematic of a beverage capable of providing long-lasting water and mineral delivery to the small intestine for prolonged hydration.

Figure 2 is a schematic of a beverage of the invention, showing how the container under the cap is broken to release the edible polymer hydrogel into the liquid where it begins to swell.

Figure 3 is a schematic of a beverage of the invention comprising coated xerogel particles.

Figure 4 is a graph comparing the viscosity of citric acid-cross-linked carboxymethylcellulose with viscosities of certain food fibers.

Figure 5 is a graph comparing the viscosity of citric acid-cross-linked carboxymethylcellulose with the viscosities of guar gum and psyllium.

Figure 6 is a graph comparing the elastic response of citric acid-cross-linked carboxymethylcellulose and certain food fibers.

Figure 7 is a summary of a study of citric acid-cross-linked carboxymethylcellulose in rats.

Figure 8 is a graph illustrating the effect of citric acid-cross-linked carboxymethylcellulose on food intake in rats.

Figure 9 is a graph showing swelling and collapse of an edible polymer hydrogel as it moves through the gastrointestinal tract.

## DETAILED DESCRIPTION OF THE INVENTION

The present invention relates to an edible polymer hydrogel comprising carboxymethylcellulose crosslinked with citric acid, for use in enhancing glycemic control in a subject, wherein said subject is diabetic, insulin resistant or at risk for developing insulin resistance or diabetes, and wherein the edible polymer hydrogel is orally administered to the subject.

In another aspect the invention relates to A modified food or foodstuff comprising an edible polymer hydrogel, wherein said hydrogel comprises carboxymethylcellulose crosslinked with citric acid, and is in the form of dehydrated particulates coated with a moisture barrier.

The term "food bolus", as used herein, refers to a mass of masticated and/or partially digested food which is present in one region of the digestive tract, e.g., the mouth, the stomach, the small intestine or the colon, following the ingestion of food.

The subject to be treated can be in need of weight- and or shape-management with a BMI of less than 25. The subject to be treated can be in need of weight loss or weight maintenance. The subject can be overweight, with a BMI of 25 to 30, or obese, with a BMI greater than 30. The subject can also be of normal weight, with a BMI less than 25, but at risk for weight gain. The subject can also be in need of glycemic control. Such a subject can be overweight, obese or of normal weight or below (BMI less than 25). The subject can be diabetic or pre-diabetic. The subject can also be at risk for developing diabetes, particularly Type II diabetes. For example, the subject can suffer from insulin resistance or metabolic syndrome. The method can be used to prevent, inhibit or delay the development of insulin resistance, metabolic syndrome or diabetes.

Herein described are methods of lowering cholesterol and reducing the risk of colon cancer in a subject. These methods comprise the step of orally administering to the subject in need thereof an effective amount of an edible polymer hydrogel comprising a cross-linked cellulosic polymer. Following oral administration, the edible polymer hydrogel travels from the subject's stomach, through the small intestine and into the colon, where it is fermented to produce short chain fatty acids, which have been shown to decrease risk of colon cancer by reduction of colonic pH and result in reduced serum cholesterol levels (Samelson SL, et al., J R Soc Med 1985; 78: 230 -233). The subject can be at risk of colon cancer or heart disease. For example, the subject can have a family history of colon cancer, or environmental exposure or a gene which increases the risk of colon cancer.

In certain embodiments, the edible polymer hydrogel of the invention swells in the stomach after administration. In the presence of ingested food, the edible polymer hydrogel, upon absorption of water or gastric fluids and/or upon mixing with the food in the stomach, will cause the volume of a food bolus to increase without substantially increasing the energy content of the bolus. In such embodiments, the increased size of the food bolus will result in satiation and decreased caloric intake. In certain embodiments, the edible polymer hydrogel remains swollen in the stomach for a period of time, then shrinks, degrades and/or collapses. In certain embodiments, the edible polymer hydrogel swells in the stomach and thereby slows gastric emptying to enhance the satiety effect of a limited calorie meal.

In certain embodiments, after administration, the edible polymer hydrogel swells in the small intestine but not in the stomach. In certain embodiments, the edible polymer hydrogel swells in the small intestine. In certain embodiments, the edible polymer hydrogel swells in the small intestine and thereby takes up volume and/or exerts pressure on the walls of the small intestine. In certain embodiments, the edible polymer hydrogel displaces liquid volume in the small intestine, resulting in improved glycemic control, satiation and decreased caloric intake. In certain embodiments, the edible polymer hydrogel exerts pressure on the small intestine walls, resulting in satiation and decreased caloric intake. In certain embodiments, the edible polymer hydrogel remains swollen in the small intestine for a period of time, after which it shrinks, degrades and/or collapses. Preferably, the edible polymer hydrogel degrades at least partially in the colon.

Herein described are methods which involve administering to a subject a composition comprising an edible polymer hydrogel which swells in the stomach, shrinks after a first period of time, passes into the small intestine, swells again in the small intestine, and shrinks in the small intestine after a second period of time. In another embodiment, the edible polymer hydrogel swells in the stomach and then passes into the small intestine, where it collapses, shrinks and/or at least partially degrades. In yet another embodiment of the invention, the edible polymer hydrogel swells in the stomach, passes through the small intestine and does not shrink in either the stomach or the small intestine. Preferably, the edible polymer hydrogel degrades at least partially in the colon, preferably enough to release most of the liquid it has absorbed.

Herein described are methods which involve administering to a subject a composition comprising an edible polymer hydrogel which swells in the stomach, shrinks after a first period of time, passes into the small intestine, swells again in the small intestine, passes to the colon and then shrinks, collapses and/or degrades. In an embodiment, the edible polymer hydrogel will swell in the stomach and then pass into the small intestine, and then to the colon where it collapses, shrinks and/or at least partially degrades. In yet another embodiment of the invention, the edible polymer hydrogel will swell in the stomach, pass through the small intestine and not shrink in either the stomach or the small intestine but will degrade, shrink and or collapse in the colon.

In preferred embodiments, the edible polymer hydrogel is an edible polymer hydrogel which, when swollen, has an elastic modulus of at least about 100 Pa and a viscosity of at least 20 $s^{-1}$ in the gastrointestinal environment, for example, in water, SGF/water 1:8 or SIF.

Data from the use of gastric balloons that occupy stomach volume, a procedure which is a common practice for weight loss in some parts of the world, indicates that at least 200 mL of volume, but preferably over 400 mL, is needed for efficacy. Animal studies have demonstrated that the amount of reduction of food intake caused by swollen hydrogel in the stomach is directly correlated with the amount of material that was administrated. Based on the *in vivo* data, it was also demonstrated that the amount of the reduction of food intake is also affected by the amount of swollen edible polymer hydrogel in the small intestine, which is also "volume driven."

Studies have demonstrated a direct correlation between viscosity of the gastrointestinal content and satiety. A material would preferably have rheological properties similar to that of digested food and be degradable before secretion to achieve efficacy, but minimize adverse events. The requirement for a degradable polymer is important, as a non-degradable polymer at the amounts needed to initiate satiety (preferably at least 200 mL when swollen) will cause adverse and /or undesired side effects like diarrhea, dehydration and constipation. Therefore, having materials that degrade in the gastrointestinal tract is important for safety and compliance. The edible polymer hydrogel is preferably at least partially degradable in the colon and not in the stomach or small intestine.

Accordingly, in a further embodiment, the edible polymer hydrogel increases its volume in the stomach or the small intestine. For example, the edible polymer hydrogel induces satiety following absorption of water and/or physiological fluids in the stomach and swells to a volume of at least 50, 100, 200, 300, 400, 600 and 800 mL, while in other embodiments, the edible polymer hydrogel swells to about 400 mL. The amount of edible polymer hydrogel administered depends upon the swollen volume desired and the degree to which the edible polymer hydrogel swells in the stomach, i.e., in the presence of gastric fluid. For example, to achieve a volume of 400 mL of swollen edible polymer hydrogel, 4 grams of an edible polymer hydrogel which swells 100-fold in the stomach is sufficient. Preferably, the edible polymer hydrogel administered swells at least about 20-, 40-, 60-, 80-,100-, 120-, 140- fold or more in SGF/water 1:8.

It is to be understood that unless otherwise stated, recited edible polymer hydrogel properties, such as swelling ratios, elastic modulus and viscosity, refer to the edible polymer hydrogel in neat or purified form, that is, prior to addition to food materials or coating.

The amount of edible polymer hydrogel administered depends upon the viscosity desired and the degree to which the edible polymer hydrogel viscosifies in the stomach and the small intestine, i.e., in the presence of gastric- or intestinal-fluids. For example, to achieve a food bolus viscosity above 10 sec$^{-1}$ and preferably above 50 sec$^{-1}$, the polymer material uptake should be at least 0.5% by weight of the total food and liquid consumed. Preferably, the edible polymer hydrogel administered increases the food bolus viscosity by two-fold in the presence of gastric and intestinal fluids. Preferably, the edible polymer hydrogel increases the viscosity of the food bolus in the small intestine sufficiently to significantly delay absorption of nutrients.

Preferably upon ingestion, the edible polymer hydrogel maintains a rheology (e.g., elastic modulus) similar to that of masticated or partially digested food to enhance satiety as measured by methods known in the art, for example a visual analog scale or to reduce food intake, for example, by at least 10%.

In certain embodiments, the edible polymer hydrogel composition reduces the peak bloodstream concentration of absorbed carbohydrates and fats and extends their absorption time into the bloodstream.

In certain embodiments, the composition comprises an edible polymer hydrogel which will only swell in the small intestine (i.e., it will not swell in any other part of the gastrointestinal (GI) tract). In certain embodiments, the edible polymer hydrogel is formulated so that it is only exposed in the pH environment of the small intestine (i.e. at a pH of about 6). For example, the edible polymer hydrogel can be coated by an enteric material which remains intact at stomach pH, but is degraded or removed at the higher pH of the small intestine. The edible polymer hydrogel can also be coated with a material which is removed enzymatically by enzymes found in the small intestine but not in the stomach.

In certain embodiments, the composition comprises an edible polymer hydrogel which swells in the small intestine, resulting in slower gastric emptying and prolonged satiety. For example, gastric emptying time can be 20% to 100% longer or more in the presence of the edible polymer hydrogel than in its absence.

In an embodiment, the invention relates to methods of treating obesity, inducing weight loss, and/or preventing weight gain by displacing volume of liquid and/or creating pressure on the walls of the small intestine of a subject in a non-invasive manner, preferably without creating significant pain or unreasonable discomfort in the subject. The methods comprise the step of orally administering to the subject an edible polymer hydrogel which swells in the small intestine and increases the viscosity of the intestinal contents. For example, the edible polymer hydrogel can increase the ratio of semi-solids to liquids in the intestinal contents. In this embodiment, the edible polymer hydrogel displaces a volume of liquid and/or induces pressure on the walls of the small intestine in order to induce a feeling of satiation either directly or by increasing gastric emptying time.

In one embodiment, the methods described herein involve administering to a subject a composition of the invention which causes the ileal brake (Maljaars PW, Peters HP, Mela DJ, Masclee AA., Ileal brake: a sensible food target for appetite control, Physiol Behav. 2008 Oct 20;95(3):271-81), thus releasing hormones and neurotransmitters that indice satiety. Such hormones and neurotransmitters can include cholecystokinins (CCK), leptin, obestatin, nesfatin$^{-1}$ and other neural signals that may induce satiety.

In some embodiments, the edible polymer hydrogel creates pressure on the wall of the small intestine, increases the volume of the small intestine's contents, or both. In certain embodiments, the edible polymer hydrogel reduces the contact between the lining of the small intestine and food particles by diluting the food within the bolus, thereby slowing nutrient uptake into the bloodstream.

In a preferred embodiment, the edible polymer hydrogel swells in the stomach following ingestion, moves into the small intestine and moves to the colon, where it is degraded. Preferably, degradation of the edible polymer hydrogel in the colon releases a substantial amount of its water content, for example, at least about 70, 80, 90 or 95% of the water content of the hydrogel, thereby maintaining the subject's fluid balance.

In a more preferred embodiment, the edible polymer hydrogel comprises a cross-linked anionic polymer which is not substantially absorbent at the pH of gastric fluid. Ingestion of food causes a rapid increase in stomach pH causing the edible polymer hydrogel in the stomach to swell. As the food is digested, stomach pH falls, causing collapse of the edible polymer hydrogel to a form which can move into the small intestine. At the pH of the small intestine, the edible polymer hydrogel swells again, then moves into the colon, where it is degraded, releasing at least about 70, 80, 90 or 95% of its water content.

In some embodiments, the edible polymer hydrogel has rheological properties substantially similar to those of masticated or partially digested foods. In some embodiments, the edible polymer hydrogel combines with an existing food bolus in the stomach or small intestine of the subject to increase the volume of the food bolus without a corresponding increase in energy content. Preferably the edible polymer hydrogel has substantially no energy content.

Another aspect of the present invention relates to edible polymer hydrogels with rheological properties substantially similar to those of fibers. In some embodiments, the composition combines with an existing food bolus in the subject to slow the emptying of the stomach, delay the absorption of some nutrients in the small intestine, and lower serum cholesterol. The composition can, for example, lower serum cholesterol, reduce chronic disease risk of cardiovascular disease (Jacobs DR, Jr., Meyer KA, Kushi LH, Folsom AR. Whole-grain intake may reduce the risk of ischemic heart disease death in postmenopausal women: the Iowa Women's Health Study. Am J Clin Nutr. 1998;68(2):248-257; Rimm EB, Ascherio A, Giovannucci E, Spiegelman D, Stampfer MJ, Willett WC. Vegetable, fruit, and cereal fiber intake and risk of coronary heart disease among men. JAMA. 1996;275(6):447-451; Keenan JM, Pins JJ, Frazel C, Moran A, Turnquist L. Oat ingestion reduces systolic and diastolic blood pressure in patients with mild or borderline hypertension: a pilot trial. J Fam Pract. 2002;51(4):369), colorectal cancer (Trock B, Lanza E, Greenwald P. Dietary fiber, vegetables, and colon cancer: critical review and meta-analyses of the epidemiologic evidence. J Natl Cancer Inst. 1990;82(8):650-661), decreased risk of diverticulosis (a relatively common condition that is characterized by the formation of small pouches (diverticula) in the colon) (Korzenik JR. Case closed? Diverticulitis: epidemiology and fiber. J Clin Gastroenterol. 2006;40 Suppl 3:S112-116).

In some embodiments, the compositions of the invention are fermented by bacteria that normally colonize the colon, resulting in the formation of beneficial short-chain fatty acids (acetate, propionate, and butyrate) (Kumar, C. M. et al. Modulatory effect of butyric acid-a product of dietary fiber fermentation in experimentally induced diabetic rats, The Journal of Nutritional Biochemistry, Volume 13, Issue 9, Pages 522-527). Such short chain fatty acids have been shown to reduce serum cholesterol level, induce satiety and protect against colon cancer.

In the foregoing methods, the edible polymer hydrogel can be administered prior to eating, for example, a meal or a snack, or with food. The edible polymer hydrogel can be administered for example within one or two hours of eating, or concurrently with food consumption. The edible polymer hydrogel can be administered in a variety of forms, for example, as a powder, in a capsule, tablet or sachet, or as a component of a food or beverage. Suitable dosage forms as well as modified foods and beverages are described herein.

## Foods and Foodstuffs

The present invention relates to modified foods and foodstuffs, including foods prepared with modified foodstuffs of the invention, with reduced energy density compared to corresponding conventional foods and foodstuffs. Thus when consumed in the same volume as the corresponding conventional foods, the modified foods of the invention provide fewer calories while effecting a substantially similar degree of satiety compared to conventional foods. Thus when consumed with a given amount of food the volume of the modified partially digested food in the stomach and the small intestine will increase and will result in enhanced satiety.

The term "food", as used herein, refers to an edible, palatable composition which can be ingested and may be in either cooked or uncooked form. Foods include hot and cold cereals, for example oatmeal and cornflakes; nutritional food bars, baked goods, pastas, syrups, purees, candies, beverages, shakes, processed meats, pet foods, dairy foods, frozen foods, such as ice cream, frozen yogurt; frozen confections, including ice pops; polenta, risotto, hummus, couscous, and so forth. A food can be intended for humans, companion animals and/or veterinary use, although modified food for humans is preferred.

The term "foodstuff", as used herein, refers to a material or composition which is used as an ingredient in preparing food. Examples of foodstuffs which can be modified as described herein include foodstuffs prepared from grains, cereals, starchy fruits and vegetables. Suitable examples include flours, such as flours prepared from wheat, rice, corn, oat, potato, sorghum, millet, rye, triticale and barley. Other flours include semolina flour, Atta flour, buckwheat flour, tapioca flour, brown rice flour, glutinous rice flour, noodle flour, pasta flour, chestnut flour, various nut flours, chickpea flour, bean flour, pea flour, spelt flour and potato starch flour. Foodstuffs which can be modified further include cornstarch, instant mashed potatoes, prepared mixes for baked goods including bread dough, cake mixes, pancake mixes, and so forth. Additional foodstuffs that can be modified according to the invention include preparations of bulgur, quinoa, triticale, parsnip, plantain, potato, pumpkin, acorn squash, butternut squash, summer squash, green peas, corn, yarns, taro, cassava, and breadfruit. Preferred foodstuffs for use in the present invention are carbohydrate-based foodstuffs.

The term "modified", as it is applied herein to foods and foodstuffs, refers to a food or foodstuff which includes an edible polymer hydrogel as an ingredient or component. A modified food or foodstuff can be compared to the corresponding "conventional" or unmodified food or foodstuff, that is, the corresponding food or foodstuff which does not include the edible polymer hydrogel. The edible polymer hydrogel has a lower energy density than the conventional food or foodstuff, and therefore dilutes the energy content of the modified food or foodstuff. Thus, the modified foods and foodstuffs of the invention have a lower energy density than their conventional counterparts. However, they can be consumed in the same volume as conventional foods, thereby achieving a substantially similar degree of satiety. Further, in certain embodiments, the edible polymer hydrogel is dehydrated in the food and swells once in contact with the contents of the stomach or small intestine, thereby inducing a greater sense of fullness relative to the volume of food consumed.

In one embodiment, the invention relates to an edible polymer hydrogel, such as those described herein, in a form that can be used in cooking. For example, the edible polymer hydrogel can be dried and milled to produce granules, grains or a fine powder. The edible polymer hydrogel can also be provided in a dehydrated, swollen or partially swollen state, or a combination of these, and in any form, such as powder, granules, grains, gel, and films. The edible polymer hydrogel can be packaged for sale and use, for example, in an air-tight container or bag, and is optionally packaged with instructions for use in cooking. In one embodiment, the instructions for use include recipes which utilize the edible polymer hydrogel.

Modified foods and foodstuffs of the invention preferably include a carbohydrate ingredient, such as a digestible carbohydrate ingredient. Preferably, in such modified foods and foodstuffs, the edible polymer hydrogel replaces at least a portion of at least one carbohydrate ingredient. It is particularly preferred that the edible polymer hydrogel replaces at least a portion of the digestible carbohydrate relative to the conventional food or foodstuff. Thus, in this embodiment, the modified food or foodstuff has a reduced digestible carbohydrate content compared to the corresponding conventional food or foodstuff.

In one embodiment, the modified food comprises a swollen or hydrated edible polymer hydrogel. In this embodiment, the edible polymer hydrogel is added as an ingredient during food preparation in the swollen state, or it is added in a dehydrated or partially swollen state and then swells during food preparation. In an embodiment, the edible polymer hydrogel is formed during food preparation. In this embodiment, the polymer(s) and cross-linking agent components of the edible polymer hydrogel are added to one or more other ingredients of the food during food preparation, resulting in formation of the edible polymer hydrogel during the preparation process, for example, while cooking.

In an embodiment, the invention provides a food comprising an edible polymer hydrogel, in which the edible polymer hydrogel is present in the food in a dehydrated state. In this embodiment, the edible polymer hydrogel swells in the stomach and/or small intestine following ingestion. The dehydrated edible polymer hydrogel is optionally coated with an moisture barrier to prevent or inhibit moisture uptake by the hydrogel during food preparation and/or storage.

In an embodiment, the invention provides a method of preparing a food or foodstuff comprising an edible polymer hydrogel, comprising contacting a polymer with an cross-linking agent in the presence of one or more additional ingredients, thereby forming the food or foodstuff comprising the edible polymer hydrogel.

In one embodiment, the invention provides a foodstuff which can be used to prepare a modified food of the invention, the foodstuff comprising an edible polymer hydrogel. For example, the edible polymer hydrogel can be dried and milled to a fine particle size and added to flour, for example, any of the flours described above, to yield modified flour. The edible polymer hydrogel can also be added to the flour in other forms, including granules, grains and films. The amount of edible polymer hydrogel in the modified flour can vary, but will typically be in the range of 5 to 55% by weight. The modified flour can be packaged together with instructions for use. The instructions for use can include recipes for preparing a modified food. Modified flours of the invention can be used in packaged mixes for baked goods, such as cake mixes, bread mixes, cookie mixes and pancake mixes, and in packaged doughs, such as bread dough and cookie dough. Alternatively, modified mixes and doughs can be prepared by adding conventional flour to the other ingredients in a reduced amount, for example from about 5% to about 55% less compared to a conventional mix, with the balance made up with milled or filmed edible polymer hydrogel.

The modified flours, mixes and doughs of the invention can be used to prepare any food item in which flour is used, including baked goods, such as breads, cakes, muffins, pastries, breakfast cereals, pastas, puddings and gravies.

Alternatively, such modified foods can be prepared by decreasing the amount of flour used in the corresponding conventional food, with the difference made up with the edible polymer hydrogel, present in a dehydrated, swollen, or partially swollen state, or a combination of these, and in any form, such as powder, granules, grains, films, etc.

The invention further provides modified foods which include an edible polymer hydrogel as an ingredient. Preferred modified foods include foods which have a carbohydrate base, including foods prepared from grains, cereals and/or starchy vegetables. In one embodiment, the edible polymer hydrogel replaces at least a portion, for example, from about 5% to about 55%, 5% to 40%, 5% to 30%, 5% to 20% or 5%to 10% of the carbohydrate content, relative to the corresponding conventional food. Modified foods with a carbohydrate base include baked goods, breads, cookies, crackers, pastas, cereals, including hot and cold breakfast cereals, potato-based foods, including mashed potatoes and fried potatoes, nutritional food bars, nutritional supplements, beverages, including nutritional drinks and shakes.

The invention also provides methods for producing a modified food or foodstuff. The method comprises replacing at least a portion of the carbohydrate content of a food or foodstuff with an edible polymer hydrogel, thereby forming the modified food or food stuff. The portion of the carbohydrate content can be replaced by removing the portion from the food or foodstuff or from one or more ingredients used to prepare the food or foodstuff and replacing it with the edible polymer hydrogel, preferably in a volume that is substantially similar to the volume of the portion removed. For example, modified bread can be prepared using modified flour of the invention or by replacing at least a portion of the flour in the conventional bread recipe with an edible polymer hydrogel.

In one embodiment, the modified food of the invention is a pet food, for example, a food for dogs or cats or other mammalian pets. The pet food can be a dry pet chow in the form of pieces or granules which include an edible polymer hydrogel as an ingredient. In another embodiment, the dry pet food is mixed with granules of the edible polymer hydrogel in either hydrated or dehydrated form. In another embodiment, the pet food is a wet food, such as a canned pet food, which comprises an edible polymer hydrogel. The invention also provides an edible polymer hydrogel in hydrated or dehydrated form, which is suitable for mixing with a pet food. For example, mixing a wet pet food with an edible polymer hydrogel increases the volume of the food without substantially increasing its caloric value.

In an embodiment, the modified food of the invention provides significant nutritional benefits in the form of soluble and/or insoluble fiber, carbohydrate, protein, vitamins, minerals and/or healthful fats and oils. The modified food is also preferably palatable with an appetizing flavor and texture.

In an embodiment, the modified food provides a convenient vehicle for replacement of a meal or a snack intended to be used by those seeking to lose weight. While consumers express a preference for snacks and other foods which are more healthful and which can assist them to manage their shape and weight and other health objectives, they show little inclination to sacrifice the organoleptic properties of their favorite foods or snacks. Therefore, preferred modified foods of the invention are palatable.

In one embodiment, the modified food of the invention is a nutritional food bar. The modified food bars of the invention represent an improvement over conventional food bars.

The modified foods of the invention, such as nutritional food bars, can include a variety of food ingredients in additional to an edible polymer hydrogel. Such food ingredients include carbohydrates, fiber, protein, fats and oils, sweeteners and flavorings and vitamins and minerals.

In an embodiment, the modified food is a hot or cold cereal or a nutritional food bar. The cereal can be a cold cereal comprising wheat, corn, oat small intestinerice or other grains, such as corn flakes and other cereals known in the art. The cereal can also be a hot cereal comprising wheat, corns, oats, rice or other grains, such as oat meal.

In other embodiments, the modified food is a dairy product. such as yogurt or cheese, including soft cheeses, such as cream cheese, cottage cheese and processed American cheese. The modified dairy products of the invention have reduced energy density than their conventional counterparts, while preserving the texture and/or the organoleptic properties of the conventional foods. The edible polymer hydrogel can be added to foods such as yogurt to provide flavor and/or texture, for example, as a replacement for fruit pieces. The edible polymer hydrogel can, for example, be swollen in an aqueous solution comprising a suitable flavouring agent, such as a fruit flavoring.

In another embodiment, the modified food is a dessert, such as a syrup, pudding, mousse, ice cream, frozen yogurt or custard.

The invention further provides methods of producing the modified foods of the invention. The modified foods can be prepared using conventional processes and recipes, but with the addition of the edible polymer hydrogel as an additional ingredient or as a substitute for all or part of another ingredient. The edible polymer hydrogel can be thoroughly mixed throughout the modified food, or it can be included in a discrete portion of the composition, for example, as a coating, or in particles or beads. The food can be uncooked or cooked, for example, by baking, frying, broiling or roasting.

In an embodiment, the edible polymer hydrogel is an ingredient in components of a food, such as cookies or chocolate pieces (e.g. chocolate chips or chocolate chunks). For example, the edible polymer hydrogel can be added as a powder to melted chocolate, which is then cooled to form chocolate pieces or coatings which comprise the edible polymer hydrogel.

In another embodiment, the hydrogel is one of the components of the food itself.

In another embodiment, the modified food, such as a food bar or a cookie, is prepared by cooking, preferably by baking. In this method a dough or batter is prepared which comprises the edible polymer hydrogel and other ingredients, such as a carbohydrate ingredient, an fat or oil, a protein ingredient and flavorings. This dough can be formed into individual cookies or food bars or into a larger form from which individual bars or cookies can be cut before or after baking. Following baking, the bars or cookies can be optionally coated with a conventional coating, such as a melted coating, including melted chocolate or vanilla, nuts, granola or other coatings known in the art.

In another embodiment, the method of producing a modified food of the invention does not involve cooking or heating. This method has the advantage of avoiding destruction of heat-sensitive vitamins and minerals. Additionally, energy requirements and processing times are reduced in this process. Such a process can be a batch process or a continuous process. In one embodiment for the production of a food bar, the process is a continuous one in which the ingredients are first combined. The ingredients can be combined by mixing, provided that when the ingredients include pieces of granola, cookies and so forth which are intended to remain intact, the mixing process substantially maintains the integrity of these pieces. The combined ingredients are transferred on a conveyor belt and hoppers to a conventional confectionary-type bar extruder, such as a Werner-Lahara bar extruder, which has opposing rollers which force the mixture through a die to form the extrudate or core. The extrusion is preferably performed at about room temperature. The preferred extruded shape is a rectangular bar, but other shaped bars, known in the snack bar art, such as cylindrical, and semicylindrical shaped bars can be made using appropriate extruder dies.

The extrudate is cut into individual serving size pieces using a suitable cutting means, such as a guillotine-type cutter or a wire cutter, for example, in a conventional manner. The extrudate is preferably cut so as to result in a bar of the desired size.

The process of preparing the food bars or cookies of the invention can further include the step of coating the bars or cookies, for example, by enrobing, spraying or dipping them in a coating material such as a melted coating material, for example, melted chocolate. The melted coating material may be the same or different from the coated bar. The surface coating is then allowed to cool, preferably by chilling in a cooling tunnel, to solidify the coating material. The coated product may be topped with a conventional topping, such as the granola or ground nuts in conventional manner.

The modified food, such as nutritional bars or cookies, can then be packaged, preferably in a conventional foil laminate food grade packaging film. Packaging in a foil laminate film preserves the moisture content of the product and prevents the edible polymer hydrogel from absorbing ambient moisture and swelling before ingestion upon storage over an extended period of time. The interior of the package can be flushed with an inert gas, such as nitrogen, in a conventional manner to reduce the oxygen content in the package.

In an embodiment, a modified food of the invention has low moisture content, for example, less than about 10% by weight, yet is chewy and moist tasting. In an embodiment, the food is shelf-stable for at least 6 to 12 months under non-refrigerated conditions.

Modified hot or cold breakfast cereals of the invention can be prepared by coating the cereal pieces, such as flakes, with the edible polymer hydrogel. The edible polymer hydrogel can also be added to the cereal as distinct pieces, optionally combined with one or more other food ingredients, such as nuts, sugars, and so forth. The edible polymer hydrogel can also be added as an integral component of the cereal, for example, in a baked cereal, being added to the dough or batter prior to baking. The edible polymer hydrogel can be coated with a moisture barrier, partially coated with a moisture barrier or uncoated.

For modified hot cereals of the invention, the edible polymer hydrogel can be coated on the cereal pieces and can be dehydrated, partially swollen or swollen to create greater volume and reduced

In one embodiment, the invention provides a beverage comprising an acid, such as citric acid, ascorbic acid, succinic acid, tartaric acid, phosphoric acid or monopotassium phosphate, and a pH-sensitive edible polymer hydrogel. Preferably the pH of the beverage is preferably 4 or less and more preferably between 2.5 and 4. Suitable edible polymer hydrogels include edible polymer hydrogels comprising a polyacidic polymer such as those described above. Such edible polymer hydrogels will not absorb significant quantities of water at the low pH of the beverage, but will absorb fluid in the stomach, particularly as stomach pH increases immediately during a meal. The beverage can be flavored, for example, with fruit flavoring or fruit juice. The beverage can also contain nutrients such as vitamins and minerals, protein, electrolytes, and/or sugars such as sucrose or glucose. Such nutrients can be provided by a fruit juice ingredient or added as purified nutrients or mixtures of nutrients. The beverage can comprise other flavourings, including artificial sweeteners, and natural and/or artificial colors. The beverages of the invention can be sold as ready-to-drink, or as a concentrate or powder to which water is added by the consumer.

In one embodiment, the present invention provides a beverage which is able to provide long-lasting water and mineral delivery to the small intestine for prolonged hydration. This result is achieved by adding swollen edible polymer hydrogel microspheres to the beverage. The edible polymer hydrogel is ingested together with the beverage, and once in the small intestine delivers the liquid and the salts under a gradient in concentration. The edible polymer hydrogel is then expelled with the feces.

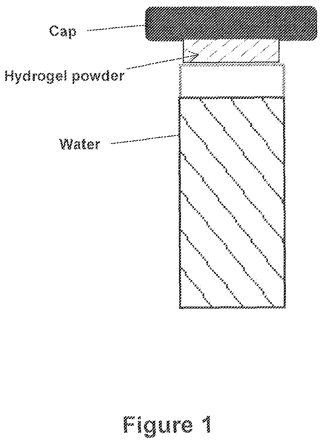

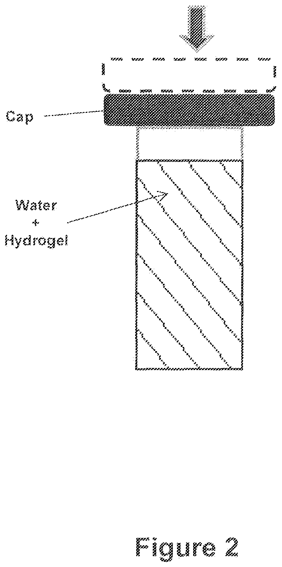

To provide this product, the addition of hydrogel particulates or microspheres in the dehydrated state are packaged protected from the liquid, for example under the cap (Figure 1). The hydrogel microspheres are optionally charged with additives, such as proteins, salts and/or molecules intended to be administered orally. Before drinking, the container under the cap is broken, releasing the edible polymer hydrogel into the liquid where it begins to swell (Figure 2). Release of the additives begins, first in the liquid mass, and then throughout the passage through the gastrointestinal tract.

The amount of edible polymer hydrogel stored changes as a function of the hydration time and salt and nutrients charge desired. However, the maximum quantity of edible polymer hydrogel stored in the bottle will be modulated in such a way that it will be not able to absorb all the liquid phase, in order to create a microbeads suspension rather than a bulk gel.

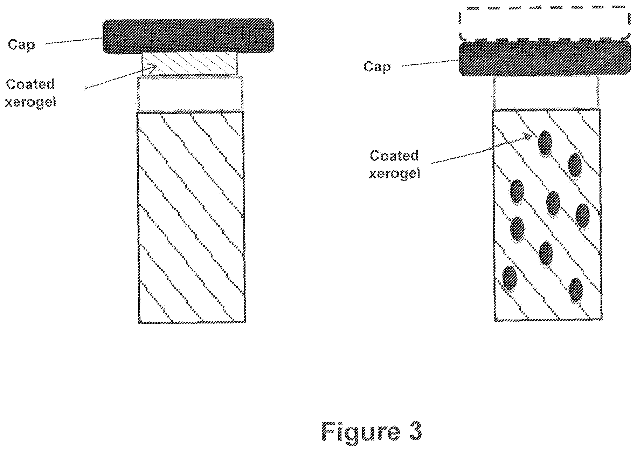

A second approach to this particular field of application consists in the use of a beverage, or other liquid, semi-liquid or frozen food as the carrier for the edible polymer hydrogel material, creating a bulking agent effect (Figure 3). Suitable foods include dairy products, such as yogurt, ice cream, frozen yogurt, frozen custard, and soups, to this aim, the edible polymer hydrogel, in dry form, is coated by a protein or macromolecular film or other suitable protective moisture barrier, which does not dissolve in water or water solutions, thus preventing the hydrogel from swelling in the liquid before ingestion. Once the edible polymer hydrogel reaches the stomach, the coating dissolves or is digested, and the edible polymer hydrogel starts to swell, thus increasing the viscosity of the liquid present in the stomach. Moreover, by means of this coating protection, the material can be ingested in high amounts, without the necessity to swallow large number of xerogel-filled capsules.

### Edible polymer hydrogels

The edible polymer hydrogels of the present invention are selected from a group consisting of homopolymers, copolymers, polymer blends, cross-linked polymers, polymer blends, superporous polymers, interpenetrating polymers, superabsorbent polymers and polymer composites. In certain embodiments, the edible polymer hydrogel is a superabsorbent edible polymer hydrogel. In certain embodiments, the edible polymer hydrogel has rheological properties similar to those of masticated or ground food mixed with gastric or intestinal fluid.

An "edible polymer hydrogel", as this term is used herein, is a cross-linked hydrophilic polymer capable of absorbing water and aqueous solutions in amounts which are many times the weight of the dry polymer. The term edible polymer hydrogel refers to any hydration state of the cross-linked polymer, from the dried or "xerogel" state to the fully hydrated gel state. One of skill in the art will understand that the desired hydration state of an edible polymer hydrogel depends upon its intended use. For example, in the methods described above in which the edible polymer hydrogel swells following oral administration, the edible polymer hydrogel is administered in a substantially dehydrated state, that is a state which retains substantially all of the absorption capacity of the edible polymer hydrogel. A "dehydrated" edible polymer hydrogel retains at least about 70, 80, 90, 95, 98 or 99% or more of its absorption capacity. A dehydrated edible polymer hydrogel, for example, is typically less than 25% water by weight, preferably less than about 10 % water by weight and most preferably about 5% or less water by weight.

The term "edible polymer hydrogel", as used herein, refers to a polymer hydrogel in any state of hydration, which is (1) produced by cross-linking a polymer, such as an edible polymer, with a cross-linking agent, for example, an edible cross-linking agent and/or (2) a hydrophilic polymer cross-linked with a polycarboxylic acid. Preferably, an edible polymer hydrogel is prepared from edible materials, such as food grade materials, or materials which are generally regarded as safe ("GRAS") as defined by the U.S. Food and Drug Administration or a food additive, as defined by the European Union. An edible polymer hydrogel, is prepared from edible materials if it results from crosslinking a food grade or GRAS polymer with an cross-linking agent. Preferably an edible polymer hydrogel degrades in the colon but does not degrade in the stomach or small intestine. An edible polymer hydrogel has biodegradable cross-links, a biodegradable backbone or, preferably, both.

An "edible polymer" is a polymer that has a biodegradable backbone.

An "edible cross-linking agent" is cross-linking agent that forms biodegradable cross-links with the polymer and the products of cross-link degradation are safe for consumption.

The term "biodegradable" as used herein, refers to material which degrades, partially or completely, within the gastrointestinal tract of a subject to which it has been orally administered. Such degradation occurs within the residence time of the material within the gastrointestinal tract and preferably occurs within the colon. Preferably, the extent of degradation is sufficient to release to the subject's gastrointestinal tract or colon, at least 70, 80 or 90% or more of the liquid absorbed in the edible polymer hydrogel.

It is not necessary that all materials used in the synthesis of the edible polymer hydrogel, for example solvents, be edible. However, it is preferred that any such non-edible materials be substantially absent from the edible polymer hydrogel. For example, any organic solvent which is not edible used in the preparation of the edible polymer hydrogel should be substantially removed from the materials prior to use. Certain low levels of residual non-materials may be acceptable depending on their identity, as is known in the art.

The polymer which is cross-linked to produce the edible polymer hydrogels of use herein is carboxymethylcellulose. The cross-linking agent is citric acid. The polymer can also be directly crosslinked, for example, as described in US Published Patent Application No. 2008/0227944.

Structurally, edible polymer hydrogels are two or three dimensional macromolecular configurations. They are produced through several methods including but not limited to: a) synthesis from monomers (cross-linking polymerization); b) synthesis from polymers and polymerization auxiliary (grafting and cross-linking polymerization); c) synthesis from polymers and non-polymerization auxiliary (cross-linking polymers); d) synthesis from polymers with energy sources (cross-linking polymers without auxiliaries) and e) synthesis from polymers (cross-linking by reactive polymer-polymer intercoupling). The raw materials and technology used in synthesis are main factors of hydrogels' key properties and their range of applications.

There are a number of methods known for obtaining high purity absorbent materials for aqueous media with three-dimensional polymeric configurations for potential applications in pharmaceutical and/or medical field: a) chemical methods: ionic and/or coordinative intercomplexing (i.e., U.S. Patent No. 4,570,629 to Widra and U.S. Patent No. 5,153,174 to Band et al.); cross-linking with oligomers or reactive polymers that have reactive groups with double bonds or rings (i.e., U.S. Patent No. 5,489,261 Franzblau et al and U.S. Patent No. 5,863,984 to Doillon et al.); cross-linking with radiation (i.e., U.S. Patent No. RE33,997 to Kuamz et al.; U.S. Patent No. 4,264,155 to Miyata; and U.S. Patent No. 5,948,429 to Bell et al.); and b) physical methods: cross-linking with microwaves (i.e., U.S. Patent Nos. 5,859,077 and 6,168,762 to Reichman et al.); freeze-drying (i.e., U.S. Patent Nos. 5,676,967 to Williams et al. and 5,869,080 to McGregor et al.); and dehydrothermo-cross-linking (i.e., U.S. Patent No. 4,837,285 to Berg et al.; U.S. Patent No. 4,950,485 to Akhtar et al.; and U.S. Patent No. 4,971,954 to Brodsky et al.).

In preferred embodiments, the edible polymer hydrogel is pH sensitive, i.e. its fluid capacity is a function of pH. Such edible polymer hydrogels include those formed from polybasic or polyacidic polymers. edible polymer hydrogels comprising polyacidic polymers will exhibit greater fluid capacity at high pH than at low pH. When consumed with or as a component of food, such edible polymer hydrogels will swell as stomach pH increases upon the introduction of the food, and then collapse at least partially as stomach pH drops upon digestion of the food. In one embodiment, the edible polymer hydrogel collapses in the stomach sufficiently to release at least 50% of its fluid content. Once the edible polymer hydrogel collapses it will be cleared to the small intestine by the clearance mechanism of the stomach. Preferably, the edible polymer hydrogel particles collapse in the stomach to a size less than 2 mm, enabling them to pass through the pylorus, the sphincter located at the junction of the stomach and small intestine. Due to the neutral pH of the upper gastrointestinal tract, such an edible polymer hydrogel will swell in the small intestine for a period of time sufficient to significantly decrease the absorption of sugars and fats and therefore enhance satiety and glycemic control, before shrinking sufficiently in the colon for excretion from the body. Such shrinking can occur, for example, by degradation of the edible polymer hydrogel through loss of cross-links, resulting in the release of fluid and sufficient volume decrease for excretion from the body. The release of water upon polymer degradation can help prevent diarrhoea and dehydration.

The edible polymer hydrogels useful in the products and methods of the invention preferably have a swelling ratio of at least about 40. The swelling ratio (SR) is a measure of the ability of the edible polymer hydrogel to absorb water. SR is obtained through swelling measurements at the equilibrium (using, for example, a Sartorius micro scale (Sartorius AG, Goettingen, Germany) with a sensitivity of $10^{-5}$) and it is calculated by the following formula:

$$SR = (W_s - W_d)/W_d$$

wherein $W_s$ is the weight of the edible polymer hydrogel after immersion in distilled water (SGF/water 1:8 or SIF) for 1 hour, and $W_d$ is the weight of the edible polymer hydrogel before immersion, the edible polymer hydrogel having been previously dried in order to remove any residual water. Unless otherwise stated, the term "swelling ratio" as used herein refers to a measurement made in distilled water as the swelling medium and is determined as described in Example 32C.

In preferred embodiments, the edible polymer hydrogel has an SR of at least about 40, about 50, about 60, about 70, about 80, about 90, or about 100. For example, in certain embodiments, the edible polymer hydrogel has an SR from about 10 to about 100, from about 20 to about 100, from about 30 to about 100, from about 40 to about 100, from about 50 to about 100, from about 60 to about 100, from about 70 to about 100, from about 80 to about 100, or from about 90 to about 100. In other embodiments, the edible polymer hydrogel has a SR of about 40 to about 200, about 40 to about 250, 40 to about 300 or 100 to about 500. In certain embodiments, the edible polymer hydrogel has an SR up to 150, 200, 250, 300, 400, 500 or greater. All SR ranges bounded by any of the lower limits and any of the upper limits set forth herein are contemplated by this invention.

In certain embodiments, the edible polymer hydrogel can absorb an amount of intestinal fluid or gastric fluid which is at least about 30, 40, 50, 60, 70, 80, 90, 100, 120 or more times its dry weight. The ability of the edible polymer hydrogel to absorb such fluids can be tested using conventional means including testing with samples of bodily fluids obtained from one or more subjects or with simulated bodily fluids, such as simulated gastric fluid. In certain preferred embodiments, the edible polymer hydrogels can absorb significant amounts of SIF or a fluid prepared by combining one volume of simulated gastric fluid (SGF) with eight volumes of water. SGF and SIF can be prepared using USP Test Solutions procedures which are known in the art. In some embodiments, the edible polymer hydrogels of the invention can absorb at least about 10, 20, 30, 40, 50, 60, 70, 80, 90, 100, 120 or more times their dry weight of SGF/water 1:8 and/or SIF.

The elastic modulus of a material is a mathematical description of an object or substance's tendency to be deformed elastically (i.e., non-permanently) when a force is applied to it. Unless otherwise stated, the term "elastic modulus" as used herein refers to a measurement made in distilled water as the medium and is determined as described in Example 32A. Preferred edible polymer hydrogels of use herein have an elastic modulus at least about 100 Pa, 200 Pa, 300 Pa, 400 Pa or greater in distilled water as measured by the method of Example 32A.

Viscosity is a measure of the resistance of a fluid which is being deformed by either shear stress or extensional stress (a stress which is applied parallel or tangential to a face of a material). Unless otherwise stated, the term the term "viscosity" of a material, such as an edible polymer hydrogel, refers to a value determined in distilled water using the protocol described in Example 32B. Preferred edible polymer hydrogels of use herein have a viscosity of at least about $15$ s$^{-1}$, $30$ s$^{-1}$, $50$ s$^{-1}$ or $100$ s$^{-1}$ as measured using the method of Example 32.

In one embodiment, the edible polymer hydrogel of use herein has a swelling ratio of more than 40, an elastic modulus of at least 200 Pa and a viscosity of at least $30$ s$^{-1}$.

The edible polymer hydrogel is preferably used in particulate or powder form. The edible polymer hydrogel particles can be a variety of sizes, but will typically be in the range of about 1-1000 $\mu$m. Preferably, the particle sizes will be in the range of about 10-800 $\mu$m and more preferably about 50- 600 $\mu$m. An appropriate particle size range can be selected for a particular use by one of skill in the art.

When swollen, the edible polymer hydrogel can have varying rheological properties depending on the nature of the modified food. For example, in a solid composition such as a food bar or baked good, the swollen polymer can be firm to match the rheological properties of the food. The rheological properties of the edible polymer hydrogel can be tuned by controlling the extent of cross-linking. For example, a highly cross-linked hydrogel will be stiffer and will typically also have reduced water absorbency compared to a lightly cross-linked hydrogel. Thus, the edible polymer hydrogel can be engineered to provide a balance of desired rheological properties and desired absorbency.

In an embodiment, the dehydrated edible polymer hydrogel is coated with a moisture barrier before it is used in preparation of the modified foods of the invention. Thus, the invention provides an edible polymer hydrogel which is coated with a moisture layer. This layer is impermeable or resistant to prevent or inhibit water absorption and swelling of the edible polymer hydrogel upon storage, either alone or as a component of a modified food of the invention and/or upon contact with saliva. It has been found that transport of water between components with a different moisture content in a composite food can be prevented, at least inhibited, by using a coating layer between the two components, As the edible polymer hydrogels of the invention are hygroscopic, it is desirable to coat them.

Extensive research in the field of moisture-barriers for coating pills has been conducted by the pharmaceutical industry. For example, British patent application 756082 discloses that the moisture sensitivity of tablets can be reduced by mixing moisture- sensitive powdered ingredients with a solution of a prolamin in alcohol and then processing the coated powders into tablets.

Shellac is a commonly used biopolymer in the application of a moisture-barrier coating on foods, and is often used in combination with hydroxypropylcellulose (US 4,820,533). The combination of shellac with prolamin has been used for this purpose as well (EP 0 090 559) In patent application WO 95/23520, an ice cream composition is described in which sugar particles are present, encapsulated in a layer of butterfat. The sugar particles are very small (>100 um). Owing to the layer of butterfat, the sugar is prevented from dissolving in the ice cream. US 2006/0286264 details the coating of particles with triglyceride with specific fatty acid chain length and solids content. US 2002/0146495 describes moisture barrier composition for forming an moisture barrier for food products, especially for baking applications, comprises edible, low melting oil and edible, high melting fat. EP0471558 describes the creation of a moisture-barrier from a biopolymer such as sodium caseinate, and lipids.

Other coatings for food products are free of the "waxy" mouth feel, which remain solid at room temperature but melt sharply at body temperature and with a melting range which may be controlled within narrow limits. In one embodiment, the coating comprises one or more of the other ingredients such as oils, proteins or fats, used in the preparation of the food product.

All the above techniques and others known in the art can be employed to coat the edible polymer hydrogel particles utilizing techniques that are known in the art such as spray coating, prilling (spray congealing), fluid bed coating, panning, spreading, spraying, spouting, atomizing, immersing, brushing and/or rolling.

**Preparation of Edible Polymer hydrogels**

In preferred embodiments, the edible polymer hydrogels for use in the invention can be prepared by a method comprising of cross-linking an aqueous solution comprising carboxymethylcellulose with citric acid thereby producing the edible polymer hydrogel.

The cross-linking reaction is preferably conducted at an elevated temperature, for example, at a temperature greater than room temperature (25°C). The reaction can be conducted at a temperature from about 30°C to about 300°C or higher, preferably from about 50°C to about 140°C. In one embodiment, while the cross-linking reaction is conducted at an elevated temperature, the reaction solution is concentrated by the removal of water. The removal of water can be accomplished, for example, by evaporation. In one embodiment, a fraction of the water is removed. In another embodiment, substantially all of the water is removed, thereby producing a dry residue. Optionally, the reaction mixture is maintained at elevated temperature for a period of time following removal of water to dryness.

The carboxymethylcellulose can be used in the acid form, or as a salt with a suitable cation, such as sodium, potassium or calcium.

The method can further include the steps of purifying the edible polymer hydrogel, for example, by washing the edible polymer hydrogel in a polar solvent such as water, a polar organic solvent, for example, an alcohol such as methanol or ethanol, or a combination thereof. The edible polymer hydrogel immersed in the polar solvent swells and releases any component, such as by-products or unreacted polycarboxylic acid that was not incorporated into the polymer network. Water is preferred as the polar solvent, distilled water is still more preferred. The volume of water required to reach the maximum swelling degree of the gel is approximately 10- to 20-fold greater than the initial volume of the gel itself. The importance of avoiding the presence of any toxic by-products in the synthetic process becomes evident when the substantial amounts of water involved on an industrial scale as well as the disposal and/or recycling of the washes during this step is taken into account. The edible polymer hydrogel washing step may be repeated more than once, optionally changing the polar solvent employed. For example, the edible polymer hydrogel can be washed with methanol or ethanol followed by distilled water, with these two steps optionally repeated one or more times. The washing step can be also performed using a mixture of water/methanol, in a composition which can vary from 1/10 to 10/1 methanol/water (volume/volume); in one preferred embodiment, this composition can be comprised in the range of 1/5 to 5/2 methanol/water; in a particular preferred embodiment this composition will be 1/3 methanol/water.

The method can further include drying of the edible polymer hydrogel. The drying step is carried out by immersing the fully swollen edible polymer hydrogel in a cellulose nonsolvent, a process known as phase inversion. Suitable cellulose non-solvents include, for example, acetone and ethanol. Drying the edible polymer hydrogel by phase inversion results in a final microporous structure which improves the absorption properties and absorption rate of the edible polymer hydrogel by capillarity. Moreover, if the porosity is interconnected or open, i.e. the micropores communicate with one another, the absorption/desorption kinetics of the gel will be improved as well. When a completely or partially swollen gel is immersed into a non-solvent, the gel undergoes phase inversion with the expulsion of water, until the gel precipitates in the form of a vitreous solid as white coloured particles. Various rinses in the non-solvent may be necessary in order to obtain the dried gel in a short period of time. For example, when the swollen edible polymer hydrogel is immersed in acetone as the non-solvent, a water/acetone mixture is formed which increases in water content as the edible polymer hydrogel dries; at a certain acetone/water concentration, for example, about 55% in acetone, water is no longer able to exit from the edible polymer hydrogel, and thus fresh acetone has to be added to the edible polymer hydrogel to proceed with the drying process. The higher the acetone/water ratio during drying, the faster the drying process. Pore dimensions (i.e. the dimension of the pores generated in the bulk matrix of the hydrogel due to the particular drying method) are affected by the rate of the drying process and the initial dimensions of the edible polymer hydrogel particles: larger particles and a faster process tend to increase the pore size. Pore dimensions in the microscale range are preferred, as pores in this size range exhibit a strong capillary effect, resulting in the higher absorption and water retention capacity.

When used after the washing stage with the use of a water/methanol mixture, this acetone phase inversion procedure requires a substantially lower amount of acetone (up to 15 times lower). This is because the hydrogel does not swell completely in amethanol/water mixture, even if it is still washed of residuals. Thus the volume of product to be desiccated by phase inversion is substantially lower, requiring a smaller amount of non solvent to be desiccated. Industrially, this is important due to expenses associated with acetone use in terms of safety control procedures and waste management.

The edible polymer hydrogels of the invention can also be dried by other processes, such as air drying, freeze drying or oven drying. These drying methods can be used alone, in combination, or in combination with the non-solvent drying step described above. For example, the edible polymer hydrogel can be dried in a non-solvent, followed by air drying, freeze drying, oven drying, or a combination thereof to eliminate any residual traces of non-solvent. Oven drying can be carried out at a temperature of approximately 30-45°C until the residual non-solvent is completely removed. The washed and dried edible polymer hydrogel can then be used as is, or can be milled to produce edible polymer hydrogel particles of a desired size.

The cross-linking solution can optionally include a compound which serves as a molecular spacer. A "molecular spacer", as this term is used herein, is a polyhydroxylated compound which, although not taking part in the reaction resulting in the formation of the cross-linked edible polymer hydrogel network to a significant extent, results in an edible polymer hydrogel with an increased absorption capacity. Although in certain cases the molecular spacer may participate in the cross-linking reaction to a small extent, it is believed that molecular spacers function by sterically blocking access to the polymer chains, thereby increasing the average distance between the polymer chains during the cross-linking reaction. Cross-linking, therefore, can occur at sites which are not close together, thereby enhancing the ability of the polymer network to expand and greatly increase the edible polymer hydrogel absorption properties. From the molecular standpoint, this corresponds to a decrease of the elastic (entropic in nature) contribution to polymer swelling, associated to a lower degree of network cross-linking. Suitable compounds for use as molecular spacers in the methods of the present invention include monosaccharides, disaccharides and sugar alcohols, including sucrose, sorbitol, plant glycerol, mannitol, trehalose, lactose, maltose, erythritol, xylitol, lactitol, maltitol, arabitol, glycerol, isomalt and cellobiose. The molecular spacer is preferably included in the cross-linking solution in the amount of about 0.5% to about 30% by weight relative to the solvent, or 1 - 5 fold relative to the polymer, more preferably about 10% to about 20% and more preferably about 18% by weight relative to the solvent.

According to a preferred embodiment of the invention, the molecular spacer used to synthesise the edible polymer hydrogel is selected from the group consisting of sorbitol, sucrose and plant glycerol.

According to a particularly preferred embodiment of the method of the invention, sorbitol is used as the molecular spacer, at a concentration within the range of 0.5 to 24% by weight referred to the weight of water, preferably within the range of 10 to 20% by weight referred to the weight of water, still more preferably at a concentration of 18% by weight referred to the weight of water.

In one embodiment, the aqueous solution includes an ionic polymer, preferably an anionic polymer, and most preferably, carboxymethylcellulose. In a particularly preferred embodiment the anionic polymer is carboxymethylcellulose and the polycarboxylic acid is citric acid.

In another embodiment, the aqueous solution includes an ionic polymer and a non-ionic polymer. The ionic polymer is preferably an anionic polymer, and most preferably, carboxymethylcellulose. The non-ionic polymer is preferably substituted cellulose, more preferably a hydroxyalkylcellulose or a hydroxyalkyl alkylcellulose, and most preferably hydroxyethylcellulose ("HEC"). The preferred polycarboxylic acid is citric acid.

The weight ratios of the ionic and non-ionic polymers (ionic : nonionic) can range from about 1:10 to about 10:1, preferably from about 1:5 to about 5:1. In preferred embodiments, the weight ratio is greater than 1:1, for example, from about 2 to about 5. In a particularly preferred embodiment, the ionic polymer is carboxymethycellulose, the non-ionic polymer is hydroxyethylcellulose, and the weight ratio (ionic:nonionic) is about 3:1.

In a preferred embodiment, the total precursor concentration in the aqueous solution is at least 2% by weight referred to the weight of the water of the starting aqueous solution, and the amount of the cross-linking agent is between about 0.5% and about 5% by weight referred to the weight of the precursor. In the present description, the term "precursor" indicates the hydrophilic polymer(s) used as the precursors for the formation of the edible polymer hydrogel polymer network. In certain embodiments, the "weight of the precursor" is the weight of CMC used or the combined weights of CMC and HEC used. The aqueous solution preferably includes sorbitol in an amount of about 18% by weight relative to the weight of water.

The cross-linking reaction is preferably carried out at a temperature between about 50°C and 140°C. Varying the temperature during this stage of the process will enable one to increase or decrease the cross-linking degree of the polymer network. A cross-linking temperature of about 80 °C is preferred. In one embodiment, the hydrophilic polymer is carboxymethylcellulose, preferably as the sodium salt ("CMCNa") (2-10%), the cross-linking agent is citric acid (0.01 to 5%), the molecular spacer is sorbitol (6 to 24 %), the cross-linking temperature is in the range of 65 to 100 °C and cross-linking time is from about 0.5 to about 48 hours.

**Coatings**

In certain embodiments, the composition will comprise individually-coated polymeric particles. In other embodiments, the composition will contain polymeric particles that are encapsulated with coating. In certain embodiments, the coating will prevent swelling in the stomach.

In certain embodiments, the coating will prevent swelling in the mouth and/or in the food. Such coatings, preferably, degrade in the stomach, thereby exposing the edible polymer hydrogel to the stomach contents and allowing swelling of the edible polymer hydrogel in the stomach. Suitable coatings include moisture barrier coatings comprising proteins, fats, sugars or a combination thereof.

In certain embodiments, the composition will comprise edible polymer hydrogel with an enteric coating. The term "enteric coating" is defined as a barrier applied to oral medication that controls the location of absorption in the digestive system. Enteric refers to the small intestine, thus enteric coatings prevent release of medication before it reaches the small intestine. Most enteric coatings work by presenting a surface that is stable at the highly acidic pH of the stomach, but breaks down rapidly at a less acidic (relatively more basic) pH. For example, they will not dissolve in the acidic stomach environment (pH from 1.5 to 5), but they will in the higher pH (pH above about 5.5) of the small intestine environment. Materials used for enteric coatings include fatty acids, waxes, and shellac as well as plastics. In one embodiment, the enteric coating is not digestible in the stomach of the subject, thereby preventing release of the edible polymer hydrogel in the subject's stomach. In one embodiment, the enteric coating is designed to dissolve under digestive conditions after a time period. This time period is preferably not less than about 50 minutes, thereby preventing exposure of the edible polymer hydrogel in the subject until after the material has been emptied from the stomach.

Examples of such enteric coatings include cellulosics, vinyl, acrylic derivatives, cellulose acetate phthalate, polyvinyl acetate phthalate, derivatives of hydroxypropyl methylcellulose such as hydroxypropyl methylcellulose phthalate or hydroxypropyl methylcellulose acetate succinate, copolymers of methyl methacrylate and ethyl acrylate and combinations thereof. More specifically suitable coatings include cellulose derivative include carboxymethylethylcellulose, cellulose acetate phthalate, cellulose acetate succinate, methylcellulose phthalate, hydroxymethylethylcellulose phthalate, hydroxypropylmethylcellulose phthalate, hydroxypropylmethylcellulose acetate succinate and the like; polyvinyl derivative include polyvinyl alcohol phthalate, polyvinyl butylate phthalate, polyvinyl acetoacetal phthalate and the like, maleic acid-vinyl compound copolymer include poly(vinyl acetate, maleic acid anhydride), poly(vinyl butyl ether, maleic acid anhydride), poly(styrene, maleic acid monoester), and the like; acrylic copolymer include poly(ethyl acrylate, methacrylic acid), poly(styrene, acrylic acid), poly(methyl acrylate, methacrylic acid, octyl acrylate), poly(methacrylic acid, methylmethacrylate) (e.g. Eudragit L and Eudragit S, each being trade name, available from Rohm Pharma, Germany), and combinations thereof as well as similar enteric coatings known to one in the art.

In certain embodiments, the composition will comprise a coating that will dissolve at a predetermined rate based on the thickness and composition of the coating. Such coatings could include cellulose ethers (such as ETHOCEL and METHOCEL and their mixtures), Instacoat Aqua (which includes HPMC and PVA based systems), and mixtures of acrylic resin (such as ethyl acrylate/methyl methacrylate copolymers).

**Methods of Formulation and Administration**

In certain embodiments, the composition is orally administered. Suitable oral dosage forms include tablets, capsules, caplets, chewable compositions, powders, syrups, solutions, suspension and shakes. In one embodiment, the composition is compressed with one or more excipients, and optionally with one or more pH modifying agents and/or one or more active agents to form a tablet. Suitable excipients used to prepare tablets include binding agents, preservatives, lubricants, antioxidants, glidants, flavorants, colorants, and combinations thereof.

In one embodiment, the edible polymer hydrogel is encapsulated in a hard or soft gelatin capsule. The capsule fill material contains the material and optionally one or more pH modifying agents and/or active agents. The fill material may also contain one or more excipients. As described above, suitable excipients include but are not limited to, plasticizers, crystallization inhibitors, wetting agents, bulk filling agents, aggregation prevention agents, solubilizers, glidants, bioavailability enhancers, solvents, and combinations thereof.

In certain embodiments, the buffering agent is selected from a group consisting of ammonium bicarbonate, ammonium carbonate, ammonium hydroxide, sodium bicarbonate, calcium carbonate, calcium hydroxide, magnesium carbonate, potassium bicarbonate, potassium carbonate, potassium hydroxide, sodium carbonate, sodium hydroxide, or combinations thereof.

Other examples of excipients include saccharides such as sucrose, lactose, mannitol or glucose, starch, partially pregelatinized starch, crystalline cellulose, calcium phosphate, calcium sulfate, precipitated calcium carbonate, hydrated silicon dioxide and the like. Examples of binders include an oligosaccharide or a sugar alcohol such as sucrose, glucose, lactose, maltose, sorbitol or mannitol; a polysaccharide such as dextrin, starch, sodium alginate, carrageenan, guar gum, arabic gum or agar; a natural polymer such as tragacanth, gelatin or gluten; a cellulose derivative such as methylcellulose, ethylcellulose, sodium carboxymethylcellulose or hydroxypropylmethylcellulose; a synthetic polymer such as polyvinylpyrrolidone, polyvinylalcohol, polyvinylacetate, a polyethyleneglycol, polyacrylic acid or polymethacrylic acid; and the like.

In certain embodiments, the dosage form is incorporated into a semi-solid base to form a spoonable delivery system. The semi-solid base may be comprised of pectin, guar gum, xanthan gum, gum arabic, gum acacia, locust bean gum, carageenan gum, alginic acid, psyllium hydrocolloid, oat flour gum, rice flour gum, glucomannan, tragacanth gum, karaya gum, tapioca, corn starch, cellulose gums, agar, gelatin, polyacrylates, polysaccharides, polyvinylpyrrolidones, pyrrolidones, polyols, collagen, polyethylene glycols, polyvinylalcohols, polyethers, polyesters, natural or synthetic oils, liquid paraffin, beeswax, silicon waxes, natural or modified fatty acids, or combinations thereof. Additionally, viscous fruit purees such as apple, prune, apricot, pear, pineapple, banana, grape, strawberry, raspberry, blackberry, boysenberry, loganberry, dewberry, gooseberry, cranberry, mulberry, elderberry, blueberry, fig, currant, kiwi may be used.

In certain embodiments, the dosage forms may be a sachet containing the polymeric powder which may be consumed as a dry powder or added into a semi-solid base to form a spoonable delivery system. The semi-solid base can comprise a viscous fruit puree such as apple, prune, apricot, pear, pineapple, banana, grape, strawberry, raspberry, blackberry, boysenberry, loganberry, dewberry, gooseberry, cranberry, mulberry, elderberry, blueberry, fig, currant, kiwi or combinations thereof.

In certain embodiments, the composition is administered with an appetite suppressant or anti-obesity agent. In certain embodiments, the composition and the appetite suppressant or anti-obesity agent are administered simultaneously or sequentially (i.e. in separate formulations). In certain embodiments, the composition and the appetite suppressant, anti-obesity nutraceutical, or anti-obesity agent are in the same formulation.

In certain embodiments, the appetite suppressant, anti-obesity nutraceutical or anti-obesity agent is selected from a group consisting of sibutramine hydrochloride, orlistat, rimonabant, benzphetamine, diethylpropion, mazindol phendimetrazine, phentermine, amphetamine, fenfluramine, nalmetrene, Phentermine (Fastin, Adipex, Ionamin and others); Diethylpropion (Tenuate); Sibutramine (Meridia, Reductil); Rimonabant (Acomplia); benfluorex; butenolide; diethylpropion; FG 7142 (N-methyl-9H-pyrido[5,4-b]indole-3-carboxamide); norpseudoephedrine; phenmetrazine; phentermine; phenylpropanolamine; pyroglutamyl-histidyl-glycine; sibutramine; Phendimetrazine (Prelu-2, Bontril); Benzphetamine (Didrex); Oxyntomodulin; Methylphenidate; (Concerta) (Ritalin); Phenylethylamine (Trimspa), pyruvate, DHEA, B-hydroxy-B-methylbutyrate, chitosan, conjugated linoleic acid (CLA), hoodia gordonii, bitter orange (citrus naringin), kava, usnic acid, ephedra, and combinations thereof.

In certain embodiments, the composition is administered in conjunction with a surgical intervention for obesity. In certain embodiments, the surgical intervention to treat obesity is selected from the group consisting of gastric banding, gastric bypass surgery, intragastric balloon, implantable gastric stimulator and gastric electrical stimulation.

## EXAMPLES

The invention now being generally described, it will be more readily understood by the following examples, which are included merely for purposes of illustration of certain aspects and embodiments of the present invention and are not intended to limit the invention.

### Example 1 Citric Acid Cross-linking of Carboxymethylcellulose/Hydroxyethylcellulose Mixtures

Materials

Carboxymethylcellulose sodium salt (CMCNa, MW 700 kDa, DS 0.9, food grade), HEC (MW 250kDa, food grade) were purchased from Eigenmann e Veronelli S.p.A. Milano and citric acid was supplied by Dal Cin S.p.A. Sesto San Giovanni Milano and used as received.

Edible polymer hydrogel Synthesis

Edible polymer hydrogel samples were obtained by reacting CMCNa and HEC with citric acid as a cross-linking agent in water according the following procedure. First, a total polymer concentration of 2% by weight of water, using a mixture of CMCNa and HEC with weight ratio equal to 3/1 was dissolved in distilled water by gently stirring at room temperature until a clear solution was obtained. (Poor cross-linking efficiency has been reported if only CMCNa is used, due both to the electrostatic repulsion between polyelectrolyte chains and to the high degree of substitution of hydroxyl groups at C6, the most reactive position [1]). CMCNa dissolution is slow at the concentration adopted; thus, HEC was added first to water until a clear solution was obtained with a slight increase in viscosity after 5 minutes. Then, CMCNa was added with continual stirring until a clear solution was obtained (24h), with a significant increase of viscosity. Finally, critic acid (CA) was added at different concentrations (1.75%, 2.75%, 3.75%, 10% and 20% w/w polymer) in order to obtain samples with various degrees of cross-linking. This final solution was used to mold 10 mm thick samples. All samples were first pre-dried at 30°C for 24 h to remove a large portion of the absorbed water and then kept at 80°C for the cross-linking reaction (24h with intermediate control).

Moreover, samples containing neat HEC or neat CMCNa samples cross-linked with CA were also prepared following exactly the same experimental conditions used for HEC/CMCNa mixtures.

All samples were analyzed by FT IR measurements. Anhydride formation was detected by monitoring its characteristic stretching band in the carbonyl region at 1738 cm$^{-1}$ [2].

Swelling Ratio

For this example, equilibrium swelling measurements for all the samples were carried out in distilled water using a Sartorius microbalance ($10^{-5}$ sensitivity). The swelling ratio was measured by weighing samples before and after their immersion in distilled water for about 24 h. The swelling ratio (SR) is defined as following:

$$SR= (W_s-W_d)/W_d$$

where $W_s$ is the weight of the swollen edible polymer hydrogel and $W_d$ is the weight of the dried sample [3].

Differential Scanning Calorimeter

A differential scanning calorimeter (Mettler-Toledo 822[e] Mettler DSC) was used for thermal analysis. The scanning temperature range and the heating rate were 10-200°C and 5°C/min, respectively.

The thermal cycle that was used was: (1) heating 10-100°C; (2) isotherm at 100°C for 3 minutes; (3) cooling from 100°C to 10°C; (4) heating from 10°C to 200°C; (5) isotherm at 200°C; (6) cooling until room temperature. An empty pan was used as a reference.

Fourier Transform Infrared Spectroscopy

All FT IR spectra were recorded on a JASCO FT IR 660 plus spectrometer equipped with an attenuated total reflectance (ATR) crystal sampler. Film samples were used directly on a ATR crystal sampler at a resolution of 4 cm$^{-1}$, by 300 scans, at absorbance range from 4000 cm$^{-1}$ to 600 cm$^{-1}$.

**Results and discussion**

A differential scanning calorimeter (DSC) thermogram of neat citric acid showed a peak at about 60°C, attributable to a water loss process associated with anhydride dehydration. A complete degradation, starting at about 160°C, was observed in the second scan.

DSC analysis of neat CMCNa and HEC powders indicated that some water was still absorbed in the polymers. Above 100°C, a possible degradation peak of CMCNa was detected. Both CMCNa and HEC showed thermal stability below 100°C.

A film of edible polymer hydrogel obtained with a 3:1 ratio of CMCNa/HEC and 3.75% by weight of polymer of citric acid was analyzed by DSC after drying the sample at 30°C for 24 h and reducing to powder. A large endothermic peak associated with the evaporation of the water produced by the anhydrification process was evident. A small exothermic peak that is superimposed on the first, larger peak was attributed to esterification. In the second heating cycle the glass transition ($T_g$=38°C) of the cross-linked cellulose mixture was observed.

After this preliminary DSC study, different edible polymer hydrogel samples were prepared according the following procedures. After mixing reagents in water, the reaction vessel was kept at 30°C for 24 h in dry conditions to remove water. The temperature was then raised above 60°C, calculating from the results of the first DSC analysis, obtain the citric acid anhydride. Anhydride is available to cross-link with cellulose hydroxyl groups above 60°C. Different reaction conditions such as temperature and CA concentration were utilized to optimize the synthetic procedure as summarized in Table 1. Two different reaction temperatures (80°C and 120°C) for the cross-linking process were attempted. A reaction temperature of 80°C was subsequently chosen to either prevent degradation risk or limit the reaction rate. Moreover, very high concentrations (10% and 20% by weight) of CA were initially used in order to amplify the FT IR signals associated with each chemical reaction step. Neat CMCNa and HEC were first cross-linked with CA in order to investigate its reactivity with each of the polymers.

Table 1. The effect of different reaction conditions, such as temperature and CA concentration, on the synthetic procedure

| Reaction label | Initial polymer | Citric acid concentration (% w/w polymer) |
|---|---|---|
| A10 | CMCNa | 10 |
| A20 | CMCNa | 20 |
| B10 | HEC | 10 |
| B20 | HEC | 20 |
| C10 | CMCNa/HEC (3/1) | 10 |
| C20 | CMCNa/HEC (3/1) | 20 |

FT IR spectra were recorded of the citric acid, the A10 reaction mixture before heating and the A10 reaction mixture after 5 h of heating. In the CA spectrum it is possible to observe a strong C=O band centred at 1715 cm$^{-1}$ due to carboxylic acid. The FT IR spectrum of sample A10 shows a strong absorption band at 1590 cm$^{-1}$ characteristic of cellulose [4].

After heating, the absorbance band at about 1590cm$^{-1}$ is still observed and additionally a new band at 1738 cm$^{-1}$ appears. Anhydrides display two stretching bands in the carbonyl region around 1758 cm$^{-1}$ and 1828 cm$^{-1}$. The higher frequency band was more intense in acyclic anhydrides. Cyclic anhydrides show the lower frequency (C=O stretching band) stronger than the stretching band at higher frequency [2]. The new peak observed at 1738 cm$^{-1}$ can be attributed to the characteristic stretching band of the carbonyl group at lower frequency related to anhydride formation, an intermediate reaction necessary for reaction of CA with cellulose hydroxyl groups. In contrast, the carbonyl peak expected at higher frequency is not detectable probably due to its weak intensity.

FT-IR spectra were recorded of citric acid, B10 reaction mixture before heating and B10 reaction mixture after 6.5 h of heating. The HEC spectrum again shows the band at 1590 cm$^{-1}$ before and after heating while the absorbance of the carbonyl group at 1738 cm$^{-1}$ appears only after heating at 80°C as observed for the sample A10.

Although FT-IR analysis is generally considered a qualitative technique, a literature study carried out by Coma and co-workers demonstrated that infrared spectroscopy could be used at first approximation for the determination of the cross-linking rate in cross-linked cellulosic derivatives [4]. Starting from this premise, the evolution of the different reactions leading to cross-linking at 80°C was monitored by recording FT IR spectra at different reaction times.

The area under the absorbance peak at 1738 cm$^{-1}$ (A$_1$), representing the carbonyl group, was compared to the area under the reference absorbance peak at 1592 cm$^{-1}$ (A2) which is invariant in all spectra. The evolution of the anhydride was evaluated as the ratio of A$_1$/A$_2$ as a function of the reaction time. FTIR spectra of CMCNa polymer when the reaction is performed at 80°C with 20% CA or 10% CA both displayed a similar trend: the anhydride band that is absent before heating reaches a maximum almost immediately after the first hour, successively decreases to a minimum after 3 h, then increases again to reach a second maximum after 5 h. Finally, a slower process reduced the band area to zero after 24 h. It is worth noting that in the spectrum of the 20% CA reaction, the second maximum matched a value (A$_1$/A$_2$ = 0.10) higher than those observed in the 10% CA reaction (A$_1$/A$_2$ = 0.04).

It is assumed that the peak at around 1738 cm$^{-1}$ is due to the anhydrification process involving free CA followed by the first condensation of this anhydride with cellulose hydroxyl leading to loss of the anhydride carbonyl groups. Then the now-linked carboxylate groups on the polymer are able to form an anhydride again, leading to an increase of the 1738 cm$^{-1}$ peak. The second reaction of this anhydride is responsible for the cross-linking, and results in further elimination of the anhydride group and consequent reduction of the peak at 1738 cm$^{-1}$. This second reaction is slower since it involves groups linked to large macromolecules and hence is more sterically hindered, as has also been reported for other cellulose cross-linking processes [1]. This possible reaction mechanism is confirmed by the swelling measurements.

FTIR spectra were also recorded for reactions of HEC polymer when the reaction was performed at 80°C with either 20% CA or 10% CA. In the 10% CA scenario, the anhydride band intensity increased from 0 to 0.098 when the reaction time increased from 0 h to 6.5 h, but dropped to 0 when the reaction time reached 24 h. The 20% CA reaction paralleled the same trend and provided a maximum value of 0.079 at 5h. Assuming that the cross-linking mechanism is the same as described for CMCNa, the anhydrification and esterification reactions appear superimposed. Therefore, in the FTIR spectra, the HEC polymer shows a single peak. This latter result was in accordance with conclusion of Xie and co-workers [5]. They studied the degree of substitution by evaluating cross-linking esterification on starch thermally reacted with CA at different reaction time and found a maximum after a few hours.

To explain the data observed in all FTIR spectra recorded after 24 hours, we posit that the polymer is unstable when kept in the oven for 24 hours because of unidentified secondary reactions. These reactions modify the polymer structure and also involve ester functions. Xie and co-workers [5] work hypothesized that the degree of substitution reached a maximum and then decreased since dissociation of the substituents from starch occurred when the reaction time was longer than 7 h.

Finally, polymer mixtures of CMCNa and HEC were cross-linked. CMCNa contains carboxylic acid functional groups that increase the volume variation process in solution. A preliminary attempt to follow the reaction pathway failed. It is likely that the reaction systems considered are too complex and have many different reaction centres. FT IR spectra of C10 reaction registered before, after 8 h, and after 13 h of heating were compared. Reaction sample C20 showed similar spectra. Moreover, it is worth noting that when polymer mixtures were used (C10 and C20), a broad signal appeared at about 1715 cm$^{-1}$, especially when a higher CA concentration was used in the reaction. In fact, with 20% of CA, the signal of CA at 1715 cm$^{-1}$ was very broad and overlapped to the polymer signal at 1590 cm$^{-1}$, make a clear band undetectable. However, it should be pointed out a band around 1715cm$^{-1}$ was detected before heating. The C10 reaction mixture before heating showed a band around 1715 cm$^{-1}$ covering the absorbance region monitored previously for the other reactions (A10, A20, B10, B20); thus a clear assignment to the carbonyl group is difficult. However, the other two spectra indicated that this band moved to higher wavenumbers during the cross-linking reaction. In particular , , the FT IR spectrum showed a broad band in the range of 1711 cm$^{-1}$ - 1736 cm$^{-1}$ after 8 h and after 13h this band appeared more clearly as a narrow absorbance band at 1737 cm$^{-1}$, which is typical of carbonyl groups. Spectra of C20 reaction provide similar results. Although a quantitative analysis of carbonyl groups is not possible when C10 and C20 samples are cross-linked, an evaluation of the carbonyl peak similar to those observed for the reaction of the neat polymers can be assumed.

The cross-linking kinetics were also monitored by studying the swelling behaviour during the reaction progress. Swelling ratio was calculated as a function of the reaction time for: (a) CMCNa with 10% or 20% of CA concentration; (b) HEC with 10% or 20% of CA concentration; (c) the mixture of CMCNa and HEC (3/1) with 10% or 20% CA concentration; (d) the mixture of CMCNa and HEC (3/1) with 1.75%, 2.75% or 3.75% CA concentration.

The results indicated that the swelling of CMCNa cross-linked with 10% of citric acid was higher than HEC with the same citric acid concentration after 24 h. When 20% of citric acid was added to the celluloses, the shape of the swelling curves was similar for HEC and CMCNa. In this case, as cross-linking proceeded, the swelling of HEC based samples decreased faster than CMCNa samples. This indicated a higher rate of reaction between CA and HEC. This probably occurs because HEC is less sterically hindered than CMCNa and can react more quickly than CMCNa chains. In addition, HEC has more OH groups than CMCNa (3 vs 2) in each repeating unit.

The maximum swelling of CMC/CA sample is observed at the gelation onset, after 3 h. This corresponds to the beginning of the second esterification reaction. As the cross-linking process increases, the corresponding equilibrium water absorption decreases, confirming the results of FTIR analysis.

The same reaction mechanism can be assumed for neat HEC cross-linked with CA. In this case, however, the overall behaviour is slightly different due to the absence of carboxylic groups bonded to the polymer. The results of swelling experiments must be interpreted taking into account that the CA introduces the high hydrophilic carboxylic groups that are responsible for the formation of a polyelectrolyte network. Therefore water absorption is significantly increased as carboxylic groups are linked first to the HEC chains and then to the gelled network. This effect cannot be appreciated in CMC edible polymer hydrogels since a large amount of -COOH groups, those linked to the CMCNa chains, is already bonded to the network at the onset of gelation. A similar trend is observed for the mixtures of HEC and CMCNa.

Edible polymer hydrogels of practical use presenting a high degree of swelling were obtained with a reduced concentration of citric acid (1.75%, 2.75% and 3.75% by weight of polymer). With a citric acid concentration of 3.75%, the swelling ratio can reach 900. This edible polymer hydrogel, after swelling, is characterized by adequate stiffness and it is able to keep the same shape of the synthesis vat. edible polymer hydrogels formerly synthesized [1] using divinyl sulfone, a toxic reagent, as cross-linking agents and the same ratio between CMCNa and HEC were characterized by a maximum swelling ratio of 200. In this case, a higher swelling ratio is obtained using an environmentally friendly cross-linking agent. At concentrations lower than 1.75% CA, a weak cross-linking associated with insufficient mechanical property is observed.

## Conclusions

This work shows for the first time that CA can be successfully used as cross-linking agent of CMCNa/HEC mixtures. An esterification mechanism based on an anhydride intermediate formation is proposed to explain the reaction of cellulose polymers with CA.

The cross-linking reaction for CMCNa/HEC system was observed either by DSC or FTIR analysis. The evolution of the different cross-linking reactions was monitored by means of FT IR spectra collected at different reaction times using an excess of citric acid. The swelling ratio, monitored at different reaction times, confirmed the reaction path figured out from FTIR analysis. An optimal degree of swelling (900 fold) for practical applications were achieved using low CA concentrations. The edible polymer hydrogel obtained through the method described in this Example 1 has the great advantage of reducing primary and production costs and avoiding toxic intermediates during its synthetic process.

## Example 2 Citric Acid Cross-Linking of Carboxymethylcellulose and Carboxymethylcellulose/Hydroxyethylcellulose Mixtures in the Presence of a Molecular Spacer

### Materials and methods

All the materials employed were provided by Aldrich Italia and used without any further modification. The devices used in the characterization, , were a scanning electron microscope (SEM) JEOL JSM-6500F, a precision $10^{-5}$g Sartorius scale, an Isco mixer and an ARES rheometer, in addition to the standard laboratory glassware, cupboards and counters for standard synthesis.

The edible polymer hydrogels were prepared by cross-linking an aqueous solution of carboxymethylcellulose sodium salt (CMCNa) and hydroxyethylcellulose (HEC), using citric acid (CA) as the cross-linking agent and sorbitol as the molecular spacer. The composition of a gel is given by the nominal amount of the reagents in the starting solution. The parameters used to define said composition are the following:

(i)

(i) the precursor weight concentration (%) = the total mass of polymers in the solution (e.g. CMCNa + HEC) (g) x 100/mass of water (g);

(ii)

(ii) the CMCNa to HEC weight ratio = mass of CMCNa (g) in the solution/mass of HEC in the solution (g);

(iii)

(iii) the cross-linking agent (CA) weight concentration (%) = mass of CA in the solution (g) x 100/mass of the precursors in the solution (g);

(iv)

(iv) the molecular spacer (e.g. sorbitol) weight concentration (%) = mass of molecular spacer (g) x 100/mass of water (g).

The laboratory tests demonstrated that polymer concentrations lower than 2% and CA concentrations lower than 1% do not achieve cross-linking or lead to the synthesis of a gel having very poor mechanical properties. On the other hand, CA concentrations higher than about 5% significantly increase the degree of cross-linking and polymer stabilization, but excessively reduce the absorption properties of the superabsorbent gel.

Since CMCNa is the ionic polymer species, it is possible to achieve the desired absorption properties by adjusting the weight ratio of carboxymethylcellulose sodium salt (CMCNa) to hydroxyethylcellulose (HEC). A CMCNa/HEC weight ratio of between 0/1 and 5/1, preferably between 1/1 and 3/1, was observed to enable the synthesis of an edible polymer hydrogel having optimum absorption properties.

Examples relating to the synthesis of different edible polymer hydrogels according to the invention, differing from one another in the weight percent (wt%) of citric acid and in the composition of the polymeric precursor, are provided below.

Preparation of edible polymer hydrogel A: In a beaker containing distilled water, sorbitol at a concentration of 4% by weight was added and mixed until it was completely solubilized within a few minutes. The CMCNa and HEC polymers were added at a total concentration of 2% by weight, with a CMCNa/HEC weight ratio of 3/1. Mixing proceeded until solubilisation of the whole quantity of polymer was achieved and the solution became clear. At this stage, citric acid at a concentration of 1% by weight was added to the solution, whose viscosity had greatly increased. The solution obtained was poured into a vessel and dried at 48°C for 48 hours. During this process, the macromolecules are stabilized into a polymeric network which is the backbone of the edible polymer hydrogel. At the end of the cross-linking process, the edible polymer hydrogel was washed with distilled water for 24 hours at room temperature. During this phase, the edible polymer hydrogel swelled up, thereby eliminating the impurities. In order to obtain the maximum degree of swelling and elimination of all impurities, at least 3 distilled water rinses were performed during the 24 hours washing step. At the end of this washing step, the edible polymer hydrogel was dried by phase inversion in acetone as the nonsolvent, until a glassy white precipitate was obtained. The precipitate was then placed into an oven at 45°C for about 3 hours to remove any residual trace of acetone.

Preparation of edible polymer hydrogel B: edible polymer hydrogel B was prepared as edible polymer hydrogel A, except that the polymer was made only of CMCNa, and that the CMCNa concentration is 2% by weight referred to the weight of distilled water.

Preparation of edible polymer hydrogel C: edible polymer hydrogel C was prepared as edible polymer hydrogel B, except that the citric acid concentration was 0.04% by weight referred to the weight of distilled water.

Preparation of edible polymer hydrogel D: edible polymer hydrogel D was prepared as edible polymer hydrogel B, except that the citric acid concentration was 0.01% by weight referred to the weight of distilled water.

Preparation of edible polymer hydrogel D: edible polymer hydrogel D was prepared as edible polymer hydrogel B, with the only exception that the citric acid concentration is 0.5% by weight referred to the weight of CMCNa.

<u>Preparation of edible polymer hydrogel E:</u> edible polymer hydrogel D was prepared as edible polymer hydrogel A, with the only exception that the CMCNa and HEC polymers are added at a total concentration of 4% by weight referred to the weight of distilled water.

<u>Preparation of edible polymer hydrogel F:</u> edible polymer hydrogel F was prepared as edible polymer hydrogel A, with the only exception that the citric acid concentration is 0.5% by weight referred to the combined weight of CMCNa and HEC.

<u>Absorption measurements</u>

The absorption properties of the edible polymer hydrogels as described above were tested by absorption measurements in distilled water. The absorption measurements essentially consisted of placing the dry sample, obtained from the drying step, in distilled water and left to swell until an equilibrium condition was reached.

The absorption properties of the gel were assessed based on its swelling ratio (SR), defined according to the formula illustrated above. In order to minimise the influence of experimental errors, each test was performed on three samples from each gel, and the mean value of the results of the three measurements was taken as the effective value.

Three dry samples were taken from each of the test gels, each having different weights and sizes. After recording the weights, the samples were swollen in abundant quantities of distilled water at room temperature. Upon reaching equilibrium after 24 hours, the samples were weighed once more in order to determine the swelling ratio.

**Results**

Table 2 below reports some of the results obtained, in terms of the swelling ratio, varying the concentrations of the reagents and the cross-linking times (6 hours, 13 hours, 18 hours, 24 hours).

Table 2. The effect of reagent concentration and cross-linking time on Swelling Ratio

| Sample | CMCNa | HEC | Citric Acid | Sorbitol | Cross-linking time/swelling ratio | | | |
|---|---|---|---|---|---|---|---|---|
| - | 75% | 25% | - | - | 6 hours | 13 hours | 18 hours | 24 hours |
| g16 | 2% | | 0.02% | 4% | nr | 50 | 30 | 20 |
| g17 | 4% | | 0.04% | 4% | nr | 25 | 10 | 5 |
| nr = not cross-linked | | | | | | | | |

The increase in the polymer concentration exerted a negative effect on the swelling properties of the final product and the cross-linking time exerted a significant effect of the absorbing properties.

Thus, further experiments were carried out by holding the polymer concentration constant at 2% and varying the citric acid concentration. The results are reported in Table 3.

Table 3. The Effect of varying citric acid concentration on Swelling Ratio

| Sample | CMCNa | HEC | Citric Acid | Sorbitol | Cross-linking time/swelling ratio | | | |
|---|---|---|---|---|---|---|---|---|
| - | 75% | 25% | - | - | 6 hours | 13 hours | 18 hours | 24 hours |
| g21 | 2% | | 0.04% | 4% | 40 | 25 | 20 | 10 |
| g22 | 2% | | 0.02% | 4% | Nr | 50 | 30 | 20 |
| g23 | 2% | | 0.01% | 4% | Nr | nr | 50 | 30 |
| nr = not cross-linked | | | | | | | | |

Table 3 shows that sample g22 with a CA concentration of 0.02% had the best swelling ratio.

Further experiments were performed where HEC was removed completely from the solution. This should have rendered the edible polymer hydrogel to be more hydrophilic, thereby leading to an increase of the swelling ratio. Table 4 shows some of the results obtained.

Table 4. The Effect of Elimination of HEC on Swelling Ratio

| Sample | CMCN a | HEC | Citric Acid | Sorbitol | Cross-linking time/swelling ratio | | | |
|---|---|---|---|---|---|---|---|---|
| | 100% | 0% | - | - | 6 hours | 13 hours | 18 hours | 24 hours |
| g30 | 2% | | 0.04% | 4% | Nr | 85 | 55 | 30 |
| g31 | 2% | | 0.02% | 4% | Nr | 100 | 75 | 40 |
| g32 | 2% | | 0.01% | 4% | Nr | nr | 70 | 50 |
| nr = not cross-linked | | | | | | | | |

The highest swelling ratio is associated with a cross-linking time of 13 hours and a citric acid concentration of 0.02%. Additionally, higher citric acid concentrations together with shorter cross-linking times lead to equally satisfactory swelling ratios, although the reaction is very fast and less easy to control.

Finally, the possibility of increasing the swelling ratio by creating porosity into the material to promote absorption properties was evaluated. For that purpose, the sample g31, subjected to cross-linking for 12 hours, was allowed to swell in distilled water for 24 hours and then dried by phase inversion in acetone. With this technique, a swelling ratio of 200 was obtained.

## Example 3 Swelling of an edible polymer hydrogel in Simulated Gastric Fluid (SGF) and SGF/Water Mixtures

This example describes an evaluation of the superabsorbent edible polymer hydrogel denoted edible polymer hydrogel B in Example 2 in *in vitro* swelling and collapsing experiments in various media at 37°C.

Swelling Kinetics (in 100% SGF) at 37°C

100 mg of the dried edible polymer hydrogel was immersed in either simulated gastric fluid ("SGF") or a mixture of SGF and water and allowed to swell until an equilibrium condition was reached. SGF was prepared according to USP Test Solutions procedures. The swelling ratio in each fluid was determined at various time points. The results are set forth in Tables 5 and 6.

Table 5. Swelling of dry edible polymer hydrogel B in 100 % SGF at 37°C.

| Swelling Time (min.) | Swelling Ratio (g/g) |
|---|---|
| 15 | 15.4 |
| 30 | 15.6 |
| 60 | 16.2 |
| 90 | 15.1 |

Table 6. Swelling of Dry edible polymer hydrogel B in a mixture of SGF and Water (1:8) at 37°C.

| Swelling Time (min.) | Swelling Ratio (g/g) |
|---|---|
| 15 | 78.8 |
| 30 | 84.6 |
| 60 | 88.6 |
| 90 | 79.3 |

Collapsing Kinetics (with addition of SGF) at 37°C

To simulate the effect of digestion on a hydrated edible polymer hydrogel, to the swollen edible polymer hydrogel from above (Table 6, SGF/water) after 60 minutes, 100% SGF was slowly added to collapse the gel particles. Swelling ratio was monitored as a function of cumulative volume of added SGF. The results are set forth in Table 7.

Table 7. Swelling ratio as a function of cumulative volume of added SGF

| SGF added (mL) | Swelling Ratio (g/g) |
|---|---|
| 0 | 88.6 |
| 8 | 23.1 |
| 30 | 22.6 |
| 50 | 23.1 |
| 75 | 17.1 |

Kinetics of Swelling (in 1:8 SGF/water), Collapsing (in SGF) and Re-Swelling (in Simulated Intestinal Fluid)

Experiments were conducted by monitoring the swelling ratio through a full cycle of swelling in 1:8 SGF/water, collapsing in SGF, and re-swelling (then degradation) in simulated intestinal fluid (SIF), all at 37°C. Experiments performed and results are provided in Table 8, for the re-swelling/degradation kinetics. pH values are given when available.

Table 8. Kinetics of swelling in SGF/water, collapsing in SGF, and re-swelling in SIF

| Expt. # | 60-min Swell in SGF/water | Collapse in 70-mL SGF | Re-swelling / Degradation in SIF | | | |
|---|---|---|---|---|---|---|
| | Swell Ratio | Swell Ratio | 30 min | 45 min | 90 min | 120 min |
| 1 | 95.5 pH 4.82 | 20.7 pH 1.76 | | 71.2 | 87.3 | |
| 2 | 95.3 | 19.5 pH 1.75 | 72.6 | | | 80.5 |

## Conclusions

This edible polymer hydrogel swells approximately 15 fold in simulated gastric fluids (pH 1.5), and 85x in a simulated gastric fluids/water mixture (pH 3). This indicates that the edible polymer hydrogel has a pH/swelling correlation where at pH below 3 (pKa of CMC is ~3.1) there will be limited swelling of the edible polymer hydrogel due to absence of the Donnan effect. The polymer can also swell in the increased pH of simulated intestinal fluid.

## Example 4 General Preparative Method for Citric Acid Cross-linked Carboxymethylcellulose

Alternate syntheses of an edible polymer hydrogel consisting of carboxymethylcellulose cross-linked with citric acid were investigated. These preparative methods differed from those set forth above with respect to starting polymer concentrations; cross-linking reaction procedure (changed from 100 °C under vacuum to 80 °C in air atmosphere); washing procedures; and drying procedures. In this example, the general synthesis procedure is described, followed by a number of examples.

Raw Materials

All of the materials used are food grade and are currently in use for a wide range of food preparation. A list of the most common applications for the raw materials used in this preparation is provided below:

1. Cellulose (CAS # 9004-32-4, E466):

The major areas of applications for cellulose are in frozen dairy products, pet food, bakery products, beverages, low calorie food, instant products and salad dressings. Cellulose is also used in pharmaceutical and cosmetics and personal care products. It allows the control of viscosity and rheology and is used as a suspending and binding agent. Due to its hydrophilic properties, cellulose is also used for water retention in food. It further inhibits crystal growth, and in film form it is strong and resistant. In the following examples, the cellulosic polymer used is Carboxymethylcellulose Sodium Salt (CMC Na), which is a food additive.

2. Citric Acid (CAS# 77-92-9, E330):

As a food additive, citric acid is used as a flavouring and preservative in food and beverages, especially soft drinks. Citric acid is recognized as safe for use in food by all major national and international food regulatory agencies. It is naturally present in almost all forms of life, and excess of citric acid is readily metabolized and eliminated from the body.

3. Sorbitol (CAS# 50-70-4, E420):

Sorbitol is a water soluble polyhydric alcohol with a sweet taste and high stability (besides properties of humectancy and plasticizing). It is used in manufacture of toothpaste, tonics/liquid pharmaceutical formulations, cosmetic products like face creams and lotions. It has a wide range of applications. The major uses are in dentifrice, cosmetics creams, lotions and colognes, which have become daily consumer product of the modern society. In the pharmaceutical sector it is used in vitamin syrups, cough syrups, tablet compounding, among others. Sorbitol is also a raw material for production of Vitamin C and also has applications in food products, tobacco conditioning, high quality papers, etc.

Solution Preparation

The first step of hydrogel synthesis is the mixing of raw materials. The raw materials are sodium carboxymethylcellulose (CMCNa; polymer), citric acid (cross-linker) and sorbitol (molecular/physical spacer). While the solubility of citric acid and sorbitol is very high in aqueous solutions, problems occur with sodium carboxymethylcellulose. There are many procedures which can be used to accelerate CMCNa dissolution and a few of these are described below.

1. Wet the raw materials (in particular, CMCNa) with an alcohol (ethanol, methanol or isopropylic alcohol) before the addition of water. This procedure reduces the hydration rate in the first step of the mixing and avoids clot formation). When a homogeneous solution (between alcohol and water) is achieved inside the grains, the CMCNa starts to absorb water and dissolves quickly.
2. Wet the CMCNa with water by mixing quickly to avoid clot formation. Addition of a small amount of acetic acid in water can improve the cellulose dissolution rate (pH of 3.74 is achieved by adding 25 ml of glacial acetic acid into 100 ml of purified water).
3. Keeping the tank at 10°C under constant mixing allows rapid CMCNa dissolution.

Only a few hours (typically around 6 h) are required to completely dissolve the CMCNa by using a combination of techniques 1 and 4 above (without the use of acetic acid).

Solution Drying Process

The solution prepared in the preceding stage is dried into a humid film. The drying stage is important to control the final properties of the hydrogel. The cross-linking of cellulose occurs by means of an equilibrium reaction with a production of water as a byproduct of the reaction. This means that the reaction takes place only when the moisture inside the material is below a certain value. For this reason, the initial solution is poured in a flat container to cast into a film. The film thickness is another important parameter to control for the modulation of water evaporation velocity and material cross-linking kinetics. The drying temperature should be below 45°C, and a water condenser (to eliminate moisture from the drying chamber) can help to speed up the process.

Cross-linking stage

The film cross-linking reaction takes place when the material temperature rises above the temperature of the intra-lactone formation of the citric acid (around 60°C). Important parameters are: film thickness, material and air humidity, time and temperature.

Washing stage

The washing stage, in combination with the material drying, is an important part of the process. The term "washing" usually indicated the action of removing impurities from a material, but it assumes a different meaning in the case of a hydrogel. In fact, it is during this stage that the final properties of a hydrogel are controlled. When a cross-linked hydrogel is placed in a water solution, it starts to swell up to the equilibrium with the surrounding solution. The hydrogel network is able to release all of the unreacted starting materials. For this reason, the washing medium should be changed several times (approximately 3). Washing the hydrogel in a mixture of water and an alcohol (ethanol or methanol) accelerates the washing stage and significantly reduces the volume of solvent requested. This significantly affects the safety management cost on the production line.

Drying stage

The drying stage significantly affects the final properties of hydrogel (yield and swelling ratio). A number of drying methods can be applied. One is water extraction by means of phase inversion procedure in a non-solvent (e.g. acetone) for the hydrogel network. Several studies confirm that the phase inversion method is likely the most suitable in the production of a hydrogel with enhanced swelling properties. On the other hand, this method is less efficient in terms of operation costs related to safety control procedure. The water evaporation is less expensive, but the final swelling capacity of the material is, in general, lower. This different behavior has been attributed to a different capillary water retention effect associated with a different microporosity, which in the case of phase inversion is higher and interconnected (sponge-like material), while with air drying is much lower (bulk material).

A third possibility is the partial washing of the cross-linked film, with a mixture of water and methanol, and the subsequent drying by phase inversion in acetone. This procedure has the double advantage of obtaining a high performance hydrogel (in terms of swelling capacity) and a low processing cost due to a lower volume of the partially swollen hydrogel to be dried.

**Examples 5-15 Hydrogel Production**

Examples 5- 15 below refer to several groups of hydrogels, which differ in terms of one or more of starting CMCNa concentration, sorbitol concentration, cross-linking time, washing and drying procedures. The syntheses within each example refer to the same synthesis, where only the cross-linking time is changed.

**Example 5**

A: 3% CMCNa; 6% Sorbitol; 0.15% Citric Acid (5% w/w of CMCNa)
Cross-linked for 60 min @ 80°C (ambient pressure)
Washed in tap water (700ms) overnight
Dried in acetone

Swelling Ratio in simulated gastric fluid (SGF) after 15 min = 33.16
Swelling Ratio in SGF after 30 min = 30.46
Swelling Ratio in SGF after 60 min = 49.38
Swelling Ratio in SGF after 120 min = 33.98

B: 3% CMCNa; 6% Sorbitol; 0.15% Citric Acid (5% w/w of CMCNa)
Cross-linked for 90 min @ 80°C (ambient pressure)
Washed in tap water (700ms) overnight
Dried in acetone

Swelling Ratio in SGF after 15 min = 32.38
Swelling Ratio in SGF after 30 min = 29.5
Swelling Ratio in SGF after 60 min = 28.4
Swelling Ratio in SGF after 120 min = 26.2

C: 3% CMCNa; 6% Sorbitol; 0.15% Citric Acid (5% w/w of CMCNa)
Cross-linked for 120 min @ 80°C (ambient pressure)
Washed in tap water (700ms) overnight
Dried in acetone

Swelling Ratio in SGF after 15 min = 23.14
Swelling Ratio in SGF after 30 min = 24.46
Swelling Ratio in SGF after 60 min = 18.94
Swelling Ratio in SGF after 120 min = 17.7

D: 3% CMCNa; 6% Sorbitol; 0.15% Citric Acid (5% w/w of CMCNa)
Cross-linked for 150 min @ 80°C (ambient pressure)
Washed in tap water (700ms) overnight

Dried in acetone

Swelling Ratio in SGF after 15 min = 24.54
Swelling Ratio in SGF after 30 min = 23.22
Swelling Ratio in SGF after 60 min = 26.16
Swelling Ratio in SGF after 120 min = 23.06

**Discussion**

It can be observed that increasing the cross-linking time, and thus the extent of cross-linking , decreases the swelling ratio on average as expected. It is worth noting that when the cross-linking temperature is reduced from 100°C under vacuum to 80°C in air atmosphere, the sensitivity of the difference in swelling capacity among samples cross-linked at different times (60, 90, 120, 150 min) changes slightly. It is worth noting that tap water was used to synthesize and wash all the hydrogel samples in this example. Tap water washing generally decreases the final product swelling capacity by about 20%, and the same synthesis can be performed using deionized water for better performances in terms of swelling capacity of the final product.

## Example 6

**A:** 3% CMCNa; 9% Sorbitol; 0.15% Citric Acid (5% w/w of CMCNa)
Cross-linked for 60 min @ 80°C (ambient pressure)
Washed in tap water (700ms) overnight
Dried in acetone

Swelling Ratio in SGF after 15 min = 60.1
Swelling Ratio in SGF after 30 min = 63.8
Swelling Ratio in SGF after 60 min = 71.42
Swelling Ratio in SGF after 120 min = 65.26

**B:** 3% CMCNa; 9% Sorbitol; 0.15% Citric Acid (5% w/w of CMCNa)
Cross-linked for 90 min @ 80°C (ambient pressure)
Washed in tap water (700ms) overnight
Dried in acetone

Swelling Ratio in SGF after 15 min = 73.5
Swelling Ratio in SGF after 30 min = 81.62
Swelling Ratio in SGF after 60 min = 64.6
Swelling Ratio in SGF after 120 min = 63.14

**C:** 3% CMCNa; 9% Sorbitol; 0.15% Citric Acid (5% w/w of CMCNa)
Cross-linked for 120 min @ 80°C (ambient pressure)
Washed in tap water (700ms) overnight
Dried in acetone

Swelling Ratio in SGF after 15 min = 47.86
Swelling Ratio in SGF after 30 min = 42.14
Swelling Ratio in SGF after 60 min = 49.5
Swelling Ratio in SGF after 120 min = 42.58

**D:** 3% CMCNa; 9% Sorbitol; 0.15% Citric Acid (5% w/w of CMCNa)
Cross-linked for 150 min @ 80°C (ambient pressure)
Washed in tap water (700ms) overnight
Dried in acetone

Swelling Ratio in SGF after 15 min = 53.32
Swelling Ratio in SGF after 30 min = 63.96

Swelling Ratio in SGF after 60 min = 61.28
Swelling Ratio in SGF after 120 min = 68.88

**Discussion**

The syntheses in this example are equal to those of Example 5, except for the amount of spacer (sorbitol) used, which was increased from twice to 3 times the amount of CMCNa (9%). An increase in swelling capacity was observed for all the samples for all cross-linking times, thus confirming that an increase in the average distance between the polymer chains during the chemical stabilization decreases the elastic (entropic) response to swelling of the macromolecular network. Best results in terms of swelling capacity were obtained by samples cross-linked for 60 and 90 min. Sample 8, while showing a slightly lower swelling capacity, seemed to be very stable after a long time in SGF (120 min), always increasing its swelling capacity in time. Rheological properties seemed to be good and no gel leaching was observed.

**Example 7**

**A:** 3% CMCNa; 12% Sorbitol; 0.15% Citric Acid (5% w/w of CMCNa)
Cross-linked for 90 min @ 80°C (ambient pressure)
Washed in tap water (700ms) overnight
Dried in acetone

Swelling Ratio in SGF after 15 min = 98.3
Swelling Ratio in SGF after 30 min = 98.68
Swelling Ratio in SGF after 60 min = 109.46
Swelling Ratio in SGF after 120 min = 91.42

**B:** 3% CMCNa; 12% Sorbitol; 0.15% Citric Acid (5% w/w of CMCNa)
Cross-linked for 120 min @ 80°C (ambient pressure)
Washed in tap water (700ms) overnight
Dried in acetone

Swelling Ratio in SGF after 15 min = 55.5
Swelling Ratio in SGF after 30 min = 64.42
Swelling Ratio in SGF after 60 min = 70.12
Swelling Ratio in SGF after 120 min = 92.94

**C:** 3% CMCNa; 12% Sorbitol; 0.15% Citric Acid (5% w/w of CMCNa)
Cross-linked for 150 min @ 80°C (ambient pressure)
Loss on drying = 7-10 %
Washed in tap water (700ms) overnight
Dried in acetone

Swelling Ratio in SGF after 15 min = 47.14
Swelling Ratio in SGF after 30 min = 55.84
Swelling Ratio in SGF after 60 min = 59.84
Swelling Ratio in SGF after 120 min = 60.9

**Discussion**

Syntheses of this example were characterized by increased spacer concentration (12% sorbitol). This was done having assessed a significant sensitivity, in terms of swelling capacity of the final product, to spacer concentration in the reacting mixture. Sample A, Cross-linked for 90 min, showed the best performance, going up to almost 109 swelling ratio in SGF after 60 min. Further increasing the cross-linking time to 120 and 150 minutes significantly decreased the swelling capacity of the final products, thus showing a higher sensitivity to cross-linking time for syntheses in this example when compared to the Examples 5 and 6.

### Example 8

**A:** 6% CMCNa; 12% Sorbitol; 0.3% Citric Acid (5% w/w of CMCNa)
+ glacial acetic acid 25ml / 100ml of water (pH 3.76)
Cross-linked for 60 min @ 80°C (ambient pressure)
Washed in tap water (700 mL) overnight
Dried in acetone

Swelling Ratio in SGF after 15 min = 62.4
Swelling Ratio in SGF after 30 min = 61.74
Swelling Ratio in SGF after 60 min = 72.92
Swelling Ratio in SGF after 120 min = 65.58

**B:** 6% CMCNa; 12% Sorbitol; 0.3% Citric Acid (5% w/w of CMCNa)
+ glacial acetic acid 25ml / 100ml of water (pH 3.76)
Cross-linked for 90 min @ 80°C (ambient pressure)
Washed in tap water (700ms) overnight
Dried in acetone

Swelling Ratio in SGF after 15 min = 52.62
Swelling Ratio in SGF after 30 min = 56.70
Swelling Ratio in SGF after 60 min = 59.9
Swelling Ratio in SGF after 120 min = 55.54

**C:** 6% CMCNa; 12% Sorbitol; 0.3% Citric Acid (5% w/w of CMCNa)
+ glacial acetic acid 25ml / 100ml of water (pH 3.76)
Cross-linked for 120 min @ 80°C (ambient pressure)
Washed in tap water (700ms) overnight
Dried in acetone

Swelling Ratio in SGF after 15 min = 31.16
Swelling Ratio in SGF after 30 min = 37.96
Swelling Ratio in SGF after 60 min = 39.72
Swelling Ratio in SGF after 120 min = 35.54

**D:** 6% CMCNa; 12% Sorbitol; 0.3% Citric Acid (5% w/w of CMCNa)
+ glacial acetic acid 25ml / 100ml of water (pH 3.76)
Cross-linked for 150 min @ 80°C (ambient pressure)
Washed in tap water (700ms) overnight
Dried in acetone

Swelling Ratio in SGF after 15 min = 26.42
Swelling Ratio in SGF after 30 min = 30.26
Swelling Ratio in SGF after 60 min = 27.1
Swelling Ratio in SGF after 120 min = 25.32

**Discussion**

Acetic acid has been added to the starting reacting mixture of syntheses of this example, to change the pH of the solution to 3.76 and better dissolve the higher polymer concentration (6% cellulose). The amount of sorbitol used in this synthesis is always double with respect to the CMCNa (12%), and the citric acid concentration was always 5% of CMCNa for all the samples.

The first relevant result was that not only was the dissolution of CMCNa complete and easy to achieve, but also a stable cross-linked network was obtained. Moreover, the swelling ratio of the material was significant, with a maximum of approximately 73 for the sample cross-linked for 60 minutes and kept in the SGF for 60 minutes. Of course, increasing the cross-linking time produces a concomitant decrease in the swelling ratio; this reduction seemed to be quite smooth, thus not displaying a high sensitivity to variations of the cross-linking time.

## Example 9

**A:** 3% CMCNa; 6% Sorbitol; 0.15 % Citric Acid
Cross-linked for 60 min @ 80°C (ambient pressure)

Swelling Ratio in SGF after 15 min = 22.66
Swelling Ratio in SGF after 30 min = 22.08
Swelling Ratio in SGF after 60 min = 22.56
Swelling Ratio in SGF after 120 min = 20.74

**B:** 3% CMCNa; 6% Sorbitol; 0.15% Citric Acid
Cross-linked for 90 min @ 80°C (ambient pressure)

Swelling Ratio in SGF after 15 min = 17.3 ($\pm$ 5%)
Swelling Ratio in SGF after 30 min = 16.38 ($\pm$ 5%)
Swelling Ratio in SGF after 60 min = 16.76 ($\pm$ 5%)
Swelling Ratio in SGF after 120 min = 15.8 ($\pm$ 5%)

**C:** 3% CMCNa; 6% Sorbitol; 0.15% Citric Acid
Cross-linked for 120 min @ 80°C (ambient pressure)

Swelling Ratio in SGF after 15 min = 13.06
Swelling Ratio in SGF after 30 min = 13.4
Swelling Ratio in SGF after 60 min = 14.26
Swelling Ratio in SGF after 120 min = 12.94

**D:** 3% CMCNa; 6% Sorbitol; 0.15% Citric Acid
Cross-linked for 150 min @ 80°C (ambient pressure)

Swelling Ratio in SGF after 15 min = 12.74
Swelling Ratio in SGF after 30 min = 13.26
Swelling Ratio in SGF after 60 min = 13.8
Swelling Ratio in SGF after 120 min = 13.02

**Discussion**

With the aim of evaluating the removal of both water washing and acetone drying stages, samples of Example 9 have been produced with the same composition of Example 5 but eliminating these two stages. Thus, samples were cross-linked at different times, and the resulting dry powder was directly used for the swelling studies. The result was not good, with a swelling ratio not exceeding 22 in the best case. This suggests that at least one of the two stages, washing and acetone drying, is necessary to obtain a product with desired swelling properties. This is most probably due to a combination of effects, including the microstructure (from a connected microporosity to a bulky material), the presence of unreacted impurities (which can also have an additional side effect of the increasing of cross-linking during the storage time, due to solid state reactions), etc.

## Example 10

**A:** 3% CMCNa; 6% Sorbitol; 0.15% Citric Acid
Cross-linked for 60 min @ 80°C (ambient pressure)

Washed in tap water (700 □s) overnight
Dried in air atmosphere at 45°C

Swelling Ratio in SGF after 15 min = 43.52
Swelling Ratio in SGF after 30 min = 41.44
Swelling Ratio in SGF after 60 min = 59.06
Swelling Ratio in SGF after 120 min = 58.36

**B:** 3% CMCNa; 6% Sorbitol; 0.15% Citric Acid
Cross-linked for 90 min @ 80°C (ambient pressure)
Washed in tap water (700 □s) overnight
Dried in air atmosphere at 45°C

Swelling Ratio in SGF after 15 min = 57.45
Swelling Ratio in SGF after 30 min = 45.72
Swelling Ratio in SGF after 60 min = 50.7
Swelling Ratio in SGF after 120 min = 55.86

**C:** 3% CMCNa; 6% Sorbitol; 0.15% Citric Acid
Cross-linked for 120 min @ 80°C (ambient pressure)
Washed in tap water (700 □s) overnight
Dried in air atmosphere at 45°C

Swelling Ratio in SGF after 15 min = 51.94 ($\pm$ 5%)
Swelling Ratio in SGF after 30 min = 74.4 ($\pm$ 5%)
Swelling Ratio in SGF after 60 min = 74.76 ($\pm$ 5%)
Swelling Ratio in SGF after 120 min = 85.9 ($\pm$ 5%)

**D:** 3% CMCNa; 6% Sorbitol; 0.15% Citric Acid
Cross-linked for 150 min @ 80°C (ambient pressure)

Washed in tap water (700 □s) overnight
Dried in air atmosphere at 45°C

Swelling Ratio in SGF after 15 min = 91.5
Swelling Ratio in SGF after 30 min = 96.54
Swelling Ratio in SGF after 60 min = 98.24
Swelling Ratio in SGF after 120 min = 95.98

**Discussion**

The samples of this example were prepared to evaluate removing just the acetone drying stage, as it is the most expensive both in terms of cost and manufacturing-related safety issues. Samples were prepared again in the same composition and with the same procedure of Example 5, but the action drying stage was removed. Samples were then washed in water after cross-linking and desiccated in air atmosphere at 45°C. Results seem to be very interesting. In fact, swelling capacity is surprisingly high, with a maximum swelling ratio higher than 90 for example 23, still obtained using just tap water for washing and only a double concentration of sorbitol with respect to CMCNa. Moreover, it is worth noting that the air drying procedure will add an energy cost to the whole process, related to the heating and humidity removal, which in the acetone drying was not present, having been replaced by the thermodynamic of the phase inversion. The additional costs of the energy consumption is lower than that of acetone management, both in terms of associated costs and safety procedures involved including control of the solvent traces in the dry final product.

**Example 11**

**A:** 3% CMCNa; 6% Sorbitol; 0.15% Citric Acid
Cross-linked for 90 min @ 80°C (ambient pressure)

Washed in methanol / distilled water (70%/30%) three times (for $\approx$ 24h)
Dried overnight in oven at 45°C

Swelling Ratio in SGF after 15 min = 19.12
Swelling Ratio in SGF after 30 min = 23.96
Swelling Ratio in SGF after 60 min = 23.12
Swelling Ratio in SGF after 120 min = 24.30

**B:** 3% CMCNa; 6% Sorbitol; 0.15% Citric Acid
Cross-linked for 150 min @ 80°C (ambient pressure)
Washed in methanol / distilled water (70%/30%) three times (for ≈ 24h)
Dried overnight in oven at 45°C

Swelling Ratio in SGF after 15 min = 21.06
Swelling Ratio in SGF after 30 min = 20.28
Swelling Ratio in SGF after 60 min = 19.09
Swelling Ratio in SGF after 120 min = 21.76

**Discussion**

Samples from this example were synthesized without the acetone drying. Methanol was also added to the water during the washing stage in order to significantly reduce the volume of water used during the washing stage and still purify the material before the final desiccation stage. Swelling capacity in SGF was found to be quite low. However, it can be improved by changing the mixture composition. The relevant issue is the achievement of a hydrogel with good mechanical properties and a stable network configuration.

**Example 12**

**A:** 3% CMCNa; 12% Sorbitol; 0.15% Citric Acid
Cross-linked for 90 min @ 80°C (ambient pressure)
Washed in methanol / distilled water (70%/30%) three times (for ≈ 24h)
Dried overnight in oven at 45°C

Swelling Ratio in SGF after 15 min = 24.02
Swelling Ratio in SGF after 30 min = 24.70
Swelling Ratio in SGF after 60 min = 24.11
Swelling Ratio in SGF after 120 min = 25.73

**B:** 3% CMCNa; 12% Sorbitol; 0.15% Citric Acid
Cross-linked for 150 min @ 80°C (ambient pressure)
Washed in methanol / distilled water (70%/30%) three times (for ≈ 24h)
Dried overnight in oven at 45°C

Swelling Ratio in SGF after 15 min = 22.80
Swelling Ratio in SGF after 30 min = 27.10
Swelling Ratio in SGF after 60 min = 26.50
Swelling Ratio in SGF after 120 min = 28.11

**Discussion**

These samples from this example were obtained with the same procedure as in Example 11, but with an increased spacer concentration. A slight increase in the swelling capacity was observed.

**Example 13**

**A:** 3% CMCNa; 12% Sorbitol; 0.15% Citric Acid (5% w/w of CMCNa)
Cross-linked for 90 min @ 80°C (ambient pressure)

Washed in methanol / distilled water (70%/30%) three times (for ≈ 24h)
Dried/washed in 100% acetone for 2 times then dried in oven (45°C) for 3h for complete acetone removal.


Swelling Ratio in SGF after 15 min = 75.38
Swelling Ratio in SGF after 30 min = 76.67
Swelling Ratio in SGF after 60 min = 124.20
Swelling Ratio in SGF after 120 min = 138.60


**B:** 3% CMCNa; 12% Sorbitol; 0.15% Citric Acid (5% w/w of CMCNa)
Cross-linked for 150 min @ 80°C (ambient pressure)
Washed in methanol / distilled water (70%/30%) three times (for ≈ 24h)
Dried/washed in 100% acetone for 2 times then dried in oven (45°C) for 3h for complete acetone removal.


Swelling Ratio in SGF after 15 min = 61.73
Swelling Ratio in SGF after 30 min = 80.47
Swelling Ratio in SGF after 60 min = 99.86
Swelling Ratio in SGF after 120 min = 116.45


**Discussion**

Here, the washing stage was performed in a methanol-water mixture. After the washing, the material was desiccated directly in acetone without any washing stage in water. Because the material was in a partially swollen state before the acetone desiccation procedure, the volume of acetone required for the desiccation was low, and the costs associated to safety issues and process management were low. In turn, product performance, in terms of final swelling capacity was excellent.

**Example 14**

3% CMCNa; 6% Sorbitol; 0% Citric Acid
25ml of acetic acid for 100ml of water (pH ≈ 3.74)
Cross-linked for 30, 60, 90, or 150 min @ 80°C (ambient pressure)
Washed in distilled water three times (for about 24h)


Swelling Ratio @ 15 min = na
Swelling Ratio @ 30 min = na
Swelling Ratio @ 60 min = na
Swelling Ratio @ 120 min = na
(na = did not swell)


**Discussion**

This sample has been synthesized without the use of citric acid, and this synthesis has been performed with the aim of demonstrating that no cross-linking is achieved without the cross-linker. This hypothesis has been demonstrated by the fact that the material dissolves when immersed in water after the desiccation

**Example 15**

**A:** 6% CMCNa; 18% Sorbitol; 0.3% Citric Acid (5% w/w of CMCNa)
Cross-linked for 90 min @ 80°C (ambient pressure)
Washed in tap water followed by a desiccation in Acetone and finally dried in air atmosphere at 45°C
Swelling Ratio in SGF/water 1/8 after 30 min = 94
Swelling Ratio in SGF/water 1/8 after 60 min = 98
Elastic modulus of 1% CMC/CA in SGF/water 1/8 @ 10 rad / sec = 1238 Pa
Viscosity of 1% CMC/CA in SGF/water 1/8 @ 0.5 $S^{-1}$ = 68

**B:** 6% CMCNa; 18% Sorbitol; 0.06% Citric Acid (1% w/w of CMCNa)
Cross-linked for 18 h @ 80°C (ambient pressure)
Washed in tap water followed by a desiccation in Acetone and finally dried in air atmosphere at 45°C
Swelling Ratio in SGF/water 1/8 after 30 min = 100
Swelling Ratio in SGF/water 1/8 after 60 min = 105
Elastic modulus of 1% CMC/CA in SGF/water 1/8 @ 10 rad / sec = 1300 Pa
Viscosity of 1% CMC/CA in SGF/water 1/8 @ 0.5 $S^{-1}$ = 140

## Example 16 Stability Testing of edible polymer hydrogel

Samples of the edible polymer hydrogel prepared by the method of Example 15B were placed in sealed vials at elevated- and room-temperature. For each sample, the swelling of the hydrogel in SGF:water (1:8) was measured at pre-determined time points. The results shown below in dicate that the edible polymer hydrogel is stable at room temperature and elevated temperatures.

6 days @ **25°C** - swelling in SGF/water 1/8 = 102
12 days @ **25°C** - swelling in SGF/water 1/8 = 107
20 days @ **25°C** - swelling in SGF/water 1/8 = 104
25 days @ **25°C** - swelling in SGF/water 1/8 = 99

3 days @ **70°C** - swelling in SGF/water 1/8 = 87
6 days @ **70°C** - swelling in SGF/water 1/8 = 75
10 days @ **70°C** - swelling in SGF/water 1/8 = 82
20 days @ **70°C** - swelling in SGF/water 1/8 = 81
25 days @ **70°C** - swelling in SGF/water 1/8 = 79

## Examples 17-23 Modified Foods and Foodstuffs

The present invention encompasses foods and foodstuffs that are preferably capable of providing satiety and/or providing a portion of the recommended daily allowance of vitamins and minerals as set forth by the U.S. Department of Agriculture. Each of these foods comprises carboxymethylcellulose cross-linked with citric acid ("CMC/CA hydrogel").
Examples of foods under the five listed groups below are given to illustrate different applications of the present invention.

Pasta
Food bars
Hot and cold cereals
Breads and cakes
Beverages

In one type of preparation, the edible polymer hydrogel within the food swells either during food preparation (e.g., pasta, yogurt, deserts, beverages) or in the stomach/GI tract (food bars, corn flakes). In a second type of preparation, the hydrogel is formed during the process of food processing (pasta, breads).

## Example 17 Modified Nutritional Food Bars

This part of the present invention provides a nutritional snack that is capable of both providing satiety and includes a portion of the recommended daily allowance of all vitamins and minerals as set forth by the U.S. Department of Agriculture.
The nutritional bar contains an edible polymer hydrogel which is not degraded in the stomach. When it swells in the stomach, it provides additional satiety due to mechanical means. Upon ingestion and contact with gastric fluid or a combination of gastric fluid and water, the edible polymer hydrogel will swell. Thus, the volume of the stomach taken up by the hydrogel can be significantly greater than the volume of the edible polymer hydrogel ingested by the subject. The edible polymer hydrogels of the invention can also take up volume and/or exert pressure on the wall of the small intestine by moving from the stomach into the small intestine and swelling.

**A. Protein Rich Bar**

Ingredients:

> 473 mL (2 cups) quick oats
> 355 mL (11/2 cups) powdered non-fat milk
> 7 g edible polymer hydrogel (as prepared in Example 10D)
> 4 scoops low carb chocolate or vanilla protein powder
> 236 mL (1 cup) sugar-free maple syrup
> 2 egg whites, beaten
> 59 mL (1/4 cup) orange juice
> 5 mL (1 teaspoon) vanilla
> 59 ML (1/4 c.) natural applesauce

> 1. Preheat oven to 163 C (325 °F) and spray a baking sheet or 9x12 baking dish with non-stick spray.
> 2. Mix oats, powdered milk and protein powder in bowl and blend well.
> 3. In separate bowl, combine egg whites, orange juice, applesauce, and the sugar-free syrup and blend well.
> 4. Stir liquid mixture into dry ingredients until blended. The consistency will be thick and similar to cookie dough.
> 5. Add the edible polymer hydrogel and homogenize for 5 min.
> 6. Spread batter onto pan and bake until edges are crisp and browned.
> 7. Cut into 10 bars and store in airtight container or freeze.

**B. Lean Bar**

Ingredients:

> 236 mL (1 cup) almond or peanut butter
> 177 mL (3/4 cup) honey
> 2.5 mL (1/2 teaspoon) vanilla extract
> 1.25 mL (1/4 teaspoon) cinnamon
> 473 mL (2 cups) old fashioned rolled oats
> 237 mL (1 cup) toasted slivered almonds
> 5 g edible polymer hydrogel (as prepared in Example 10D)
> 59 to 118 mL (1/4 to 1/2 cup) raisins or other dried fruit

> 1. Preheat oven to (350 °F) 177 C. Spray a 23 cm (9 inch) square pan with canola cooking spray.
> 2. Combine almond butter, honey in a heavy bottomed sauce pan over medium-high flamed. Whisk until melted - three to five minutes.
> 3. Stir in vanilla and cinnamon.
> 4. Add in oat, almonds and raisins.
> 5. Add the edible polymer hydrogel and mix for 5 minutes.
> 6. Bake for 15 minutes. Let cool completely and cut into nine equal squares.

**C. Coated Edible polymer hydrogel**

> 1. 100 g of edible polymer hydrogel as prepared in Example 10D were placed in a worcester fluidized bed and a solution of acetoglyceride is sprayed on the polymeric particles and allowed to dry.
> 2. edible polymer hydrogel particles (200-600 um), sugar, nonfat milk solids and sodium caseinate are blended in a 2.841 (3 quart) Hobart mixer held at (120 °F) 49-C. Fat, preheated to approximately 60 C (140 °F) is added to the dry blend and mixing was continued for 15 minutes using a dough arm at slow speed.

**D. No-Bake Granola Bars**

Ingredients:

118 mL (1/2 c.) firmly packed brown sugar
118 mL (1/2 c.) light corn syrup
237 mL (1 c.) peanut butter
5 mL (1 tsp.) vanilla
355 mL (1 1/2 c.) quick cooking rolled oats
355 mL (1 1/2 c.) crisp rice cereal
10 g of coated edible polymer hydrogel (as prepared in Example 10D) with particle size range of 200 - 1000 um
237 mL (1 c.) raisins
118 mL (1/2 c.) coconut
118 mL (1/2 c.) sunflower nuts
30 mL (2 tbsp.) sesame seeds

1. In medium saucepan, combine brown sugar and corn syrup. Bring to a boil, stirring constantly.
2. Remove from heat, stir in peanut butter and vanilla; blend well.
3. Add oats, cereal, edible polymer hydrogel, raisins, coconut, sunflower nuts and sesame seeds. Mix well.
4. Press into ungreased 23 cm (9 inch) square pan. Cool. Cut into 20 bars.

The particles were coated with the corn syrup and fats and did not swell. After a week at room temperature the bar was analyzed, it looked as the edible polymer hydrogel did not swell. The bar with the edible polymer hydrogel and a bar without the edible polymer hydrogel were placed into a beaker of water (150 mL). The bar without the edible polymer hydrogel disintegrated after 1 h and the water was free flowing. On the other hand, the bar with the edible polymer hydrogel particles disintegrated, the edible polymer hydrogel particles swelled to over 200 fold and the water became viscous.

**E. Strawberry-Filled Cereal Bars**

Prepare the strawberry filling;

591 mL (2 1/2 cups) coarsely chopped hulled strawberries
118 mL (1/2 cups) sugar
37 mL (2 1/2 tablespoons) cornstarch
177 mL (3/4 c.) butter, softened
237 mL (1 c.) packed brown sugar
473 mL (2 c.) all-purpose flour
2.5 mL-baking soda
(1/2 tsp.) 25 355 mL (11/2 c.) granola cereal
10 g of coated edible polymer hydrogel (as prepared in Example 10D)

Directions

1. Bring all ingredients to a boil in a heavy small sauce pan, stirring constantly and crushing berries slightly with back of spoon
2. Boil 2 minutes to thicken; stirring constantly (mixture will be slightly chunky).
3. Cream butter and sugar. Stir together flour and soda. Add to creamed mixture with granola and polymer; mix well.
4. Pat half into greased and floured 33 x 23 x 5 cm baking pan. Spread with filling.
5. Add 1 tablespoon water to remaining crumb mixture; sprinkle atop filling. Lightly press with hand chill and cut into bars while warm.

The strawberry seeds masked the granular mouth feel of the edible polymer hydrogel.

**F. Granola Bar with edible polymer hydrogel-Containing Chocolate Pieces**

3 g of edible polymer hydrogel (as prepared in Example 10D) with particle size 200-1000 um 5 g of chocolate (dark chocolate, Hershey, PA)

The chocolate was melted over low heat using a double boiler and the edible polymer hydrogel with particle size 200-600 um was mixed in slowly. The oils in the chocolate coated the particles and prevented the swelling. After creating a homogenous mix, the melted chocolate contains the edible polymer hydrogel particles was poured onto a cold marble counter and formed into a cube (2x1x1cm). The chocolate cube was placed in the refrigerator overnight. The next morning, the cube was cut into smaller pieces of 2 -3 mm sized to prepare the food bar of example 16D.

After a week at room temperature the chocolate particles were analyzed, the edible polymer hydrogel did not swell. Yet after crumbling the chocolate pieces and placing them into water, the edible polymer hydrogel swelled -200 fold.

**Example 18 Coated Food Bar**

A Kellogg™ Special K™ nutritional food bar bar (Kellogg NA Co., Battle Creek, MI) was crumbled to small pieces and the pieces were softened by heating for 10 minutes at 50°C. The edible polymer hydrogel prepared as in Example 15B was added to the soften pieces (3g edible polymer hydrogel was added to 21g of food bar pieces). The mixture was kneaded by hand and shaped into a bar. After the bar cooled, 10 g of the bar was placed in a glass of water (200 mL). 10 g of an original unaltered bar was also placed in a glass of water. The hydrogel-containing bar disintegrated within a minute and after 8 min absorbed all the water to form a semi-solid gel. The original bar was still intact after 10 min.

**Example 19 Modified Cereals**

Corn Flakes

Unsweetened or sweetened cornflakes (corn meal, concentrated fruit Juice, sea salt) are mixed with CMC/CA and the mixture is sprayed with a solution of sugars, minerals, vitamins, proteins, flavorings and colorants to create a coating on the corn flakes to allow attachment of the edible polymer hydrogel to the surface of the flakes.

The coated edible polymer hydrogel particles when placed in milk swell slightly also due to the proteins in the milk, but will further swell upon exposure to gastric fluids.

Granola Cereal

The CMC/CA is granulated to form granular or predetermined shapes such as dried fruits shapes, such as raisins, nuts, or any other shape. The CMC/CA granulars are added with sugars, honey, maple syrup and other semi-solid sweeteners are mixed under gentle heating with the dried cereal clusters to form clusters upon cooling to room temperature.

Typical cereal clusters are made from: whole grain wheat, sugar, rice, whole grain oats, corn syrup, wheat flakes, rice flour, honey, salt, brown sugar syrup, wheat bits (whole wheat flour, corn starch, corn flour, sugar, salt, trisodium phosphate, baking soda, color added), oat flour, natural and artificial flavor, trisodium phosphate, color added, zinc and iron (mineral nutrients), Vitamin C (sodium ascorbate), a B Vitamin (niacinamide), Vitamin B6 (pyridoxine hydrochloride), Vitamin B2 (riboflavin), Vitamin B1 (thiamin mononitrate), Vitamin A (palmitate), a B Vitamin (folic acid), Vitamin B12, Vitamin D, nonfat milk, natural almond flavor, walnut meal. Vitamin E (mixed tocopherols) and BHT to preserve freshness.

The formed granola clusters when placed in milk will swell only slowly due to the proteins in the milk, but will further swell upon exposure to gastric fluids.

Oatmeal Cereal

Oatmeal cereal is mixed with coated or uncoated CMC/CA prior to serving. Steal Cut oats are soaked a few hours in cold water, salt, and maple syrup, ground nutmeg, ground cinnamon, and ground ginger. The mixture is heated and cooked for up to 90 minutes, and to the warm mixture CMC/CA granules are added and allowed to partially swell before serving with cream, milk, or additional water.

**Example 20. Modified Pasta**

This part of the present invention describes a novel type of pasta made up of traditional pasta ingredients and the hydrogel described above in concentrations which can be varied as a function of the product's caloric content.

Because the hydrogel will not be absorbed in the gastric tract, its function will only be that of bulking product. This bulking function will be limited in a dry form and more pronounced in the swollen state. The hydrogel will exhibit this swollen state in two places: first when in contact with liquids (mainly water and water solutions) during cooking and second when in the stomach and small intestines (gastric and intestinal fluids).

For this particular application, the required swelling capacity of the hydrogel component in the final product is lower when compared to the same bulking application in capsules. Additionally, the amount of product ingested in this application can be significantly higher. The hydrogel's rheological properties (e.g. high viscosity, high G' modulus, etc) are key factors to produce a good taste and homogeneity during final product development.

Pasta is mostly associated with the production of different types of pasta with various shapes, sizes, additives types and concentrations (e.g. semolina, vegetables, flavors, etc). The product can be categorized into either artisan manufacturing (small scale, traditional plants and procedures, low pressures and high production times) or large scale production (high pressures, low production time).

Pasta production in the dry form requires proper equipment. The equipment consists of the following two components: 1. a particular system for the volumetric dosage specific for this application and 2. a cochlea bath for the mixing of all the components of the process. The cochlea bath mixing will be performed under vacuum to manufacture product without inside air and air bubbles. This will lead to a more compact and transparent product and more importantly, a product with a more brilliant color than that of traditional mixtures.

The starting mixture will contain the CMCNa and citric acid with the addition of flour in a concentration which can controlled as a function of the final product's desired caloric content. Sorbital will not be added in the starting mixture because the flour itself acts as a molecular spacer. Other components such as vegetables, spices, olive oil or other foods can also be added to the starting mix.

Once the semolina flour is added, the amber yellowish colloidal mass (mixture) is transferred in a cylinder with a variable section to mold the transferred mixture. After molding into the desired shape (e.g. spaghetti, tagliatelle, etc.), the next stage is desiccation, where the total humidity amount in the product is reduced to values slightly below of 12.5% (maximum humidity allowed by law). In the case where other food components have been added, both color and humidity of the resulting mass can change accordingly to the color and humidity of the added foods.

The desiccation step is the most sensitive part of the whole process. It allows for prolonged storage of the product, stabilizes the quality of the material, increases the product's taste characteristics, and creates an equilibrium amongst the product's components for optimal quality. The desiccation process will be performed using traditional ovens.

A second approach for the production of the pasta resembles the one described above, except for the fact that the hydrogel is not formed directly during the pasta production process, but is synthesized before, accordingly to Examples 4-13, and is then added to the normal pasta production process in a concentration which can be modulated as a function of the desired caloric content of the final product.

Two examples, related to these two different approaches, are described below.

**A. Spaghetti- Hydrogel Formation during Cooking**

Ingredients:

    2 parts of semolina flour
    1 part of CMCNa
    Water (33% of the CMC-flour mixture)
    Citric acid (5% of the CMC-flour-water mixture)

    1. Insert the components in the extruder, excluding water.
    2. Slowly add water at the different stages of the extrusion process.
    3. Extrude the mixture throughout the die.
    4. Desiccate the extrudate product at 45°C overnight.

**B. Spaghetti- Hydrogel Addition During the Process**

Ingredients:

    2 parts of CMC/CA hydrogel (as from example 12)

1 part of semolina flour
Water (33% of the initial flour content)

1. Insert the flour in the extruder.
2. Slowly add water at the different stages of the extrusion process.
3. Add the edible polymer hydrogel at the final portion of the extrusion process.
4. Extrude the mixture through the die.
5. Desiccate the product at 45°C overnight.

**Example 21 Modified Bread**

This part of the present invention describes a novel type of bread, and two types: soft and 'grissini-like' bread. Here, the working concept is similar to what was described for the pasta and food bars applications described above. An absorbent hydrogel is added to the bread products in different concentrations to achieve a composite structure which will be able to resemble structure and taste of normal bread, but with the addition of a hydrogel able to swell once in the stomach.

The hydrogel concentration in the bread product will be variable as a function of the desired caloric content of the final product, and the satiety effect to be generated. Optionally, vegetables, olive oil, spices and other foods can be added to improve the taste of the final product.

**A. Soft Bread**

Ingredients:

2 parts of flour
1 part of CMC
Citric acid (5% of the CMC by weight)
Sodium salt (5% of the flour by weight)
Water (40% of the flour by weight)
Olive oil (5% of the flour by weight)

1. Mix all the ingredients at room conditions without water.
2. Add warm (37°C) water while mixing.
3. Mold the colloidal mixture in the desired shape.
4. Stop mixing and keep at 37°C for 3 hours.
5. Cook in an oven at 121 C for a time depending by the dimensions (1Kg of cylindrical shape requires approx. 1 hour).

**B. Grissini Bread**

Ingredients:

400g of flour
20g of baking powder
2.5 mL (1/2 teaspoon) vanilla extract
200ml of milk (37°C)
2 spoons of olive oil
1 spoon of salt

1. Mix the flour, CMC, citric acid and the salt.
2. Place the flour and salt in a way which forms a circle.
3. Dissolve the baking powder into the milk and place it in the middle of the circle, together with the olive oil.
4. Mix everything until a colloidal mass is obtained.
5. Stop mixing and leave the colloidal mass for 40 minutes at 37°C.
6. Cut and mold it in thin cylindrical pieces.

7. Cook it in oven at 200°C for 20 minutes.

## Example 22 Modified Beverages

This part of the present invention describes a novel type of beverage capable of providing long-lasting water and mineral delivery to the small intestine for prolonged hydration. This result is achieved by adding swollen edible polymer hydrogel microspheres to the beverage. The edible polymer hydrogel is ingested together with the beverage. Once in the small intestine, the liquid and excess salts are delivered under a concentration gradient and eventually expelled with the feces.

To provide this product, the hydrogel microspheres must remain in a dry form within the bottle, possibly stored under the cap. The hydrogel microspheres are optionally charged with additives such as proteins, salts and/or molecules intended to be administered orally. One minute prior to drinking, the container under the cap is broken, releasing the edible polymer hydrogel into the liquid where it begins to swell. The additives will start to be released, first in the liquid mass, and then during the whole passage through the gastrointestinal tract).

The amount of edible polymer hydrogel stored changes as a function of the hydration time and desired salt and nutrients concentrations. However, the maximum quantity of edible polymer hydrogel stored in the bottle will be modulated so that it will be not able to absorb all the liquid phase. This is intended to create a microbead suspension rather than a bulk gel.

A second approach to this particular field of application consists of the use of the beverage as the carrier for the edible polymer hydrogel material, creating a bulking agent effect. To this aim, the edible polymer hydrogel, in dry form, is coated by a protein or macromolecular film or other suitable protective barrier which does not dissolve in water or water solutions, thus preventing the hydrogel from swelling in the liquid before ingestion. Once the edible polymer hydrogel reaches the stomach, the coating dissolves or is digested, and the edible polymer hydrogel starts to swell, thus increasing the viscosity of the liquid present in the stomach. Moreover, by means of this coating protection, the material can be ingested in high amounts, obviating the need to swallow large number of xerogel-filled capsules.

### A. Long lasting Hydration Water

Ingredients:

    400ml of mineral water
    3g of CMC/CA hydrogel as from example 10D

As represented in Figure 1, the edible polymer hydrogel is stored in a container under the bottle cap, in a membrane that is not permeable to the water. Before drinking, exerting a pressure on the cap, break the membrane and release the hydrogel into the water (Figure 2). Hydrogel swells in water, creating a suspension of microbeads floating in water. The product is now ready for consumption.

### B. Bulking agents in beverages

Ingredients:

    400ml of mineral water
    3g of CMC/CA hydrogel as from example 10D
    0.25 g of Eudragit (by Degussa)

The edible polymer hydrogel is placed in a fludizer bed and a solution of Eudragit is sprayed on the edible polymer hydrogel particles. It is then allowed to dry before removing from the fluidizer bed.

As presented in Figure 3, the coated edible polymer hydrogel is stored in a dry form under the bottle cap before use. Immediately before use, it is released in the liquid, remaining in dry suspension until the liquid is ingested and reaches the stomach. Once it arrives in the stomach, the coating disappears and the hydrogel is free to swell. Significant amounts of dry material can be ingested without any problem for the patient by means of this technique.

C. Protein Shake

CMC/CA particles (10 - 250 um) are used as is or coated with proteins and/or fats are added to a protein shake (8 oz. unsweetened vanilla milk, 1 scoop of protein powder, a dash of lemon, heaping spoonful of yogurt, and strawberries, blueberries, raspberries or blackberries) mixed and served.

The shake containing the CMC/CA particles will be converted in the stomach semi-solid and therefore will stay in the stomach longer period of time and will enhanced satiety feel as compared to the regular protein shake.

**Example 23 Modified Cakes and Pastries**

This part of the present invention describes a novel type of cakes that is able to provide long-lasting satiety with low calories while still preserving a cake-like appearance and taste. This includes cakes and a particular type of ice creams: the ghiaccioli.

For cake production, the superabsorbent hydrogel described above will be used in the already partially or fully swollen state, with the potential addition of flavours or colorants.

**A. Modified Cannoli**

Ingredients

> 500 g flour 00;
> 2 yolks
> 25 g of alcohol
> 20 g of suet (fat)
> red wine
> CMC/CA hydrogel (Example 15A.)
> colorant
> lemon flavor

1) Cannoli shell:

> a) Place the flour in a large bowel; add the yolks, the alcohol and the wine to the middle of the flour and mix till the whole mass has a strong viscosity.
> b) Cover the mass with a towel for ½ h.
> c) Roll the dough to form of a sheet (2/3mm thickness), and cut it to circular pieces (10cm diameter).
> d) Wrap the circular sheets around cylindrical molds, and fry it in a pan in suet.
> e) Once the pieces are fried, let them cool down and then remove the moulds.

2) Cannoli Filling:

> a) Immerse the dry hydrogel powder in a water solution containing the lemon flavoring and the colorant. The hydrogel absorbs the solution, passing from a glassy-like dry state to a gel-like swollen state.
> b) Insert the swollen hydrogel inside the external cylindrical portions and serve cold.

**B. Chocolate Sponge Cake 1**

Ingredients

> 4 eggs, whole
> 237 mL (1 cup) granulated sugar
> 22 mL (1 1/2 tablespoon) margarine, melted
> 59 mL (1/4) cups cocoa, sifted
> 59 mL (4 tablespoons) boiling water
> 5.5 mL (11/8 teaspoon) self rising flour

79 mL (1/3 cup) Hi-maize® 260 resistant starch

**Preparation:**

1. Pre-heat oven to (350 °F) 177 C.
2. Lightly grease a 23 cm (9 inch) cake pan and line the base with waxed paper.
3. Beat eggs with an electric mixer until fluffy, then gradually add the sugar and beat for 15 minutes.
4. Combine the butter, cocoa and boiling water, and fold into egg mixture.
5. Sift flour and then sift again over the egg mixture.
6. Add the Hi-maize resistant starch and gently fold them together.
7. Spoon the mixture into the prepared cake pan.
8. Bake for approximately 50 minutes or until just firm to touch.
9. Cool.

The cake was flavorful and dense.

**C. Chocolate Sponge Cake 2**

Ingredients

4 eggs, whole
237 mL (1 cup) granulated sugar
22 mL (11/2 tablespoon) margarine, melted
59 mL (1/4 cup) cocoa, sifted
59 mL (4 tablespoons) boiling water
5.6 mL (11/8 teaspoon) self rising flour
39 mL (1/6 cup) Hi-maize® 260 resistant starch
15 mL (1 tablespoon) of CMC/CA hydrogel (Example 15A)

**Preparation:**

1. Pre-heat oven to (350 °F) 177 C.
2. Lightly grease a 23 cm (9 inch) cake pan and line the base with waxed paper.
3. Beat eggs with an electric mixer until fluffy, then gradually add the sugar and beat for 15 minutes.
4. Combine the butter, cocoa and boiling water, and fold into egg mixture.
5. Sift flour and then sift again over the egg mixture.
6. Swell the carboxymethyl cellulose cross-linked with citric acid polymer in 1/6 cup of warm water.
7. Add the Hi-maize resistant starch and gently mix.
8. Fold the Hi-maize resistant starch / carboxymethyl cellulose cross-linked with citric acid polymer mix with the above ingredients slowly.
9. Spoon the mixture into the prepared cake pan.
10. Bake for approximately 50 minutes or until just firm to touch.
11. Cool.

The chocolate cake was flavorful and had a good texture.

**D. Frozen Confection**

Ingredients:

CMC/CA hydrogel
colorant;
flavoring

Place the dry hydrogel powder in a bowl with a water solution containing the flavoring and the colorant. The hydrogel absorbs the solution, passing from a glassy-like dry state to a gel-like swollen state. Pour the swollen hydrogel into a mold and place the mold in a freezer at a temperature between -4 and -10°C. Remove the product from the mold and serve frozen.

This frozen confection will not drip when it reaches room temperature as the hydrogel will trap the water.

## Example 24. Comparison between CMC/CA Hydrogel and Common Food Fibers

A CMC/CA hydrogel was made as detailed in Example 15B 6% CMCNa; 18% Sorbitol; 0.06% Citric Acid Cross-linked for 210 min @ 80°C, ambient pressure). The rheological properties of this hydrogel were compared using standard methods with those of psyllium, guar gum and glucomannan.

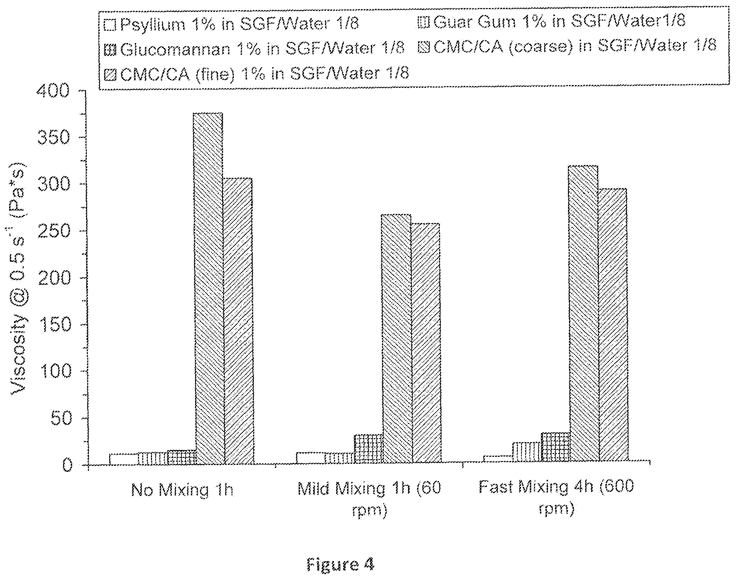

Figure 4 demonstrates that the swollen super absorbant hydrogel creats significantly higher viscosity in SGF (and SIF - data not shown) compared to the food fibers: Psyllium, guar gum and glucomannan.

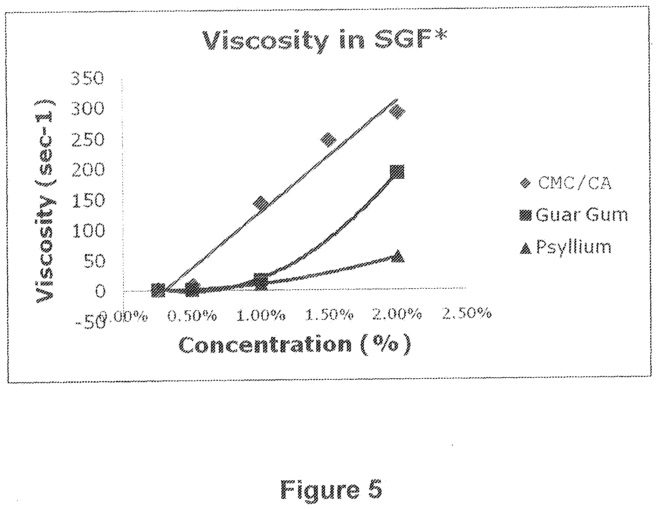

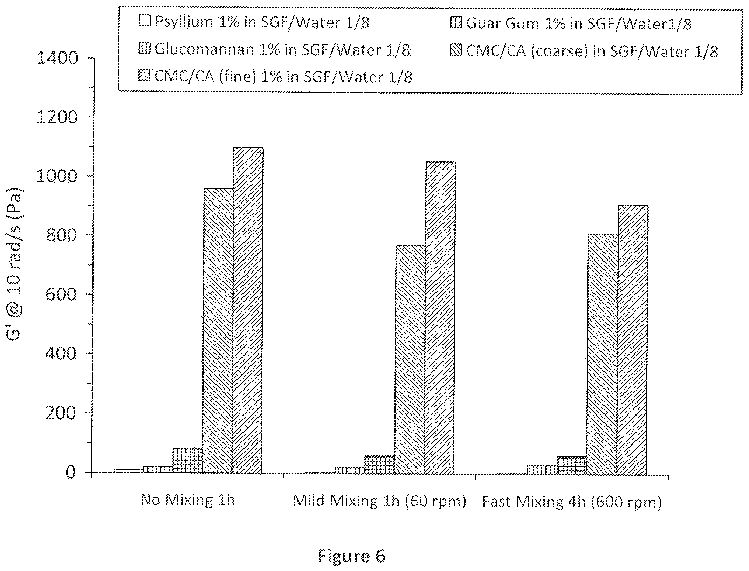

Figures 5 and 6 describe the concentration effect in SGF where unlike the food fibers, the Hydrogel had a significant effect on viscosity even in small concentrations (similar data was obtained in SIF - data not shown). This is significant as the consumption of marketed fibers in limited by their gastrointestinal adverse affects which limits their daily dose to ~10 g (which is 1% of a full stomach) while at similar concentration the hydrogel has a substantial viscosity. The increased viscosity coupled with increased elasticity of the edible polymer hydrogel will create satiety due to mechanical stretching, slow rate of gastric emptying, slow rate of glucose and fat absorption and will increase satiation, reduce food intake and would lead to better weight management and glycemic control

## Example 25 Rheometry of CMC/CA and Glucomannan in water

The rheometry (G'and G") of 2 types of CMC/CA (shorter and longer cross-linking times: 15 h - CMC/CA-A005 and 36 h - CMC/CA-A001) and glucomannan was measured in distilled water.

The CMC/CA hydrogel was prepared using a method similar to that of Example 15B.

| Material | G' @ 10 rad/s (Pa) | G" @ 10 rad/s (Pa) |
|---|---|---|
| Glucomannan 1% in water | 39,75 | 35,50 |
| Glucomannan 2% in water | 218,57 | 157,77 |
| CMC/CA-A005 1% in water (15h cross-link) | 1307,18 | 184,31 |
| CMC/CA-A005 2% in water (15h cross-link) | 2222,54 | 323,19 |
| CMC/CA-A001 1% in water (36h cross-link) | 2095,64 | 814,09 |
| CMC/CA-A001 2% in water (36h cross-link) | 2970,92 | 983,82 |

The results indicated that also at standard condition (in water) and at similar concentrations the rheology of CMC/CA was superior to that of glucamannan.

## Examples 26-29 *In Vivo* Studies

All the animal studies in the examples were approved by the respective Institutional Animal Care and Use Committee (IACUC) and the Committee for Animal Protection. Procedures used in the following studies were designed to conform to accepted practices and to minimize or avoid causing pain, distress, or discomfort to the animals. In those circumstances in which required study procedures likely caused more than momentary or slight pain or distress, the animals received appropriate analgesics or anesthetics unless the withholding of these agents has been justified in writing by the Study Director and approved by the Institutional Animal Care and Use Committee (IACUC).

## Example 26 Decrease in Food Intake of Rats Administered SAP with Different Groups of Rats

CMC/CA hydrogel was prepared and experimental conditions were the same as outlined in example two.

However, three different groups of rats were used as compared to example one. The first group of rats was fed a high fat diet (e.g., 20% of chow was fat by weight) in order to promote weight gain of the animals. The second group consisted of older animals which also had gained weight over time. The third group consisted of age matched rats to the first group and were younger compared to the second group, but were fed a normal diet.

As was observed in the second example, the CMC/CA hydrogelproduced a significant decrease in food intake compared to the water control in a within-subject design

## Example 27 Decrease in Food Intake in Rats upon Administration of CMC/CA Hydrogel

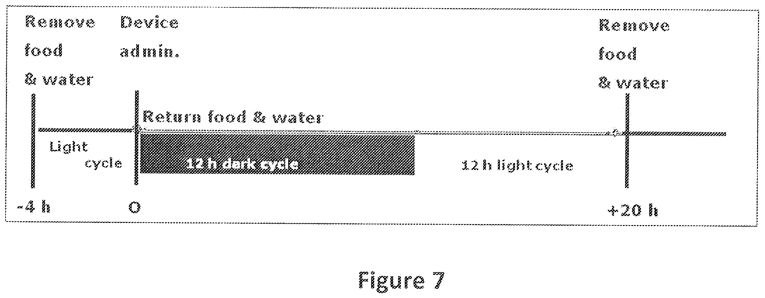

A total of 21 male Sprague-Dawley rats were randomized into two weight-matched groups (10-11 per group) prior to hydrogel or vehicle administration (the hydrogel was pre-swollen in water, 100 mg in 10 mL water). Rats weighing approximately 400 grams were housed in standard caging and fed a standard diet of rat chow. The animals were kept on a 12 hour light and dark cycle. Four hours prior to the lights being shut off, food was removed from the rats (Figure 7). On days in which the rats were subject to an experimental treatment, the animals were orally gavaged with either hydrogel which was swollen with water prior to gavage or a similar volume of water (e.g., 8 mL of polymer or 8 mL of water were used) prior to the lights being shut off. Three days later, in a classic within subject design, the animals which received water received polymer and *vice versa.* Food and water intake (digital balance) as well as locomotor activity (consecutive beam brakes) were monitored online every 5 minutes for 40 hours post-dosing. Food and water intake data were collected using MaNi FeedWin, an online computerized feeding system using digital weighing cells. Two types of baseline food intake (digital balance) and lick counts were monitored. All data were entered into Excel spread-sheets and subsequently subjected to relevant statistical analyses. The results in Figure 6 are presented as mean $\pm$ SEM unless otherwise stated. Statistical evaluation of the data was carried out using one-way or two-way analysis of variance (ANOVA).

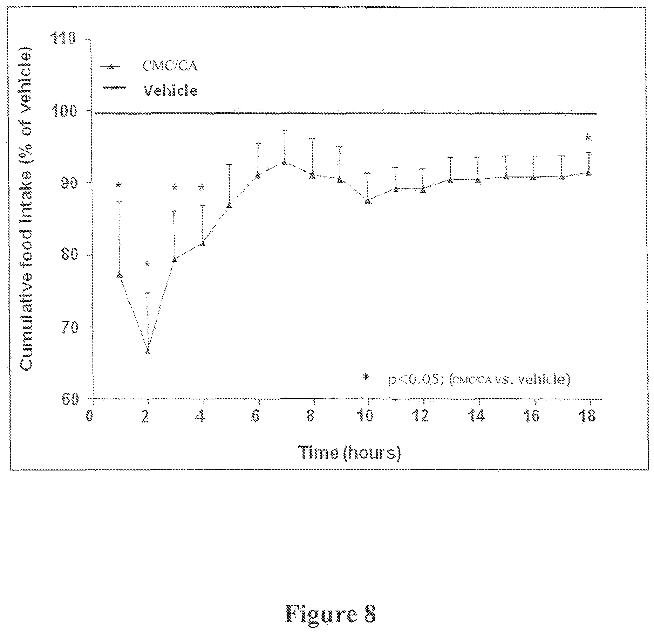

Figure 8 represents a typical study result. Cumulative food intake is graphed over time. There was no difference between the groups at baseline (time = 0). Gavage of 8 mL of hydrogel led to a significnt decrease in food intake. As shown, the hydrogel induced a marked decrease in food intake that persisted over 18 hours. These data suggest that the administraiton of hydrogel leads to a decrease in food intake due to a stomach filling effect, slower gastric emptying time, and a small intestinal effect, all of these effects combined can induce satiety in mammals over a longer period of time than a stomach filler alone will provide. Furthermore, from previous experiment (Example 20) we noted that rats' stomach was empty after 60-120 minutes. (also see, for example, Tomlin *et al.* wherein half emptying time was reported as less than 20 min; Tomlin. J. et al. Gut. 1993, 34(9): 1177-1181). The extended effect was achieved by slower emptying time and a satiety caused by the polymer re-swelling in the small-small intestine.

## Example 28 Decrease in Food Intake of Rats Administered CMC/CA Hydrogel with Different Groups of Rats

CMC/CA hydrogel was prepared and experimental conditions were the same as outlined in Example 26. However, three different groups of rats were used as compared to Example 26. Three different groups of rats were used in this experiment. The first group of rats was fed a high fat diet (e.g., 20% of chow was fat by weight) in order to promote weight gain of the animals. The second group consisted of older animals which also had gained weight over time. The third group consisted of age matched rats to the first group and were younger compared to the second group, but were fed a normal diet.

The CMC/CA hydrogel produced a significant decrease in food intake compared to the water control in a within-subject design

## Example 29 Acute Effects of CMC/CA Hydrogel on Energy Consumption, Urine Production, and Feces Production

The behavioral specificity of hydrogel was evaluated by simultaneous examination of energy consumption, urine production, and feces production. The study was conducted in male Sprague-Dawley rats, by sub-chronic per oral administration of hydrogel (10 mL, by gavage, once daily).

Sub-chronic administration of hydrogel for four days did not influence the production of urine or feces or the percentage of fecal water content. These data indicates that the adminsitered hydrogel is being degraded in the GI tract and it is not being expelled intact.

The rats consumed less food (Figure 8),. This result indicates that the administration of these hydrogel should lead to weight loss over sufficient time periods.

## Example 30 In Vitro Modeling of GI Transit of CMC/CA Hydrogel

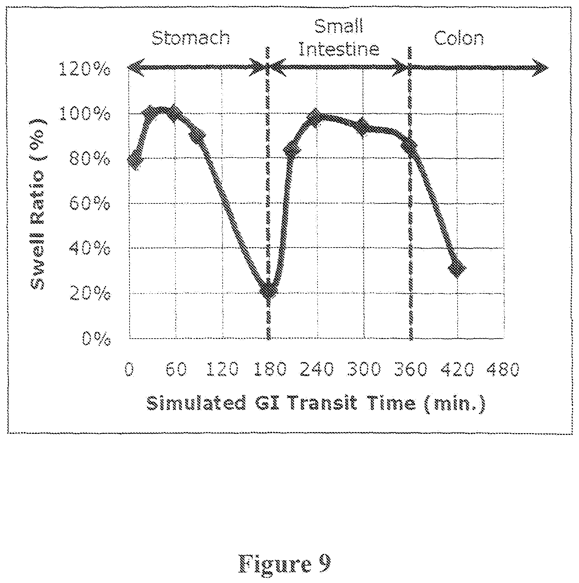

Figure 9 illustrates the swell-collapse--re-swell--degrade cycle that was observed in laboratory experiments *in*

*vitro*. The polymer used was CMC/CA hydrogel.

Simulated gastric fluid (SGF) was prepared by dissolving 2.0 g of sodium chloride, 3.2 g of pepsin and 7.0 ml of concentrated (37%) HCl in distilled water to obtain a solution having a total volume of 1 L. (USP Test Solution Method)

The above SGF solution was mixed with water at a ratio of SGF:water 1:8, respectively, to mimic a person taking the material on an empty stomach (50 mL gastric fluid) with two glasses of water (400 mL).

Simulated intestinal fluid (SIF) was prepared by adding 190 ml of 0.2 N NaOH, 400 ml of distilled water and 10 g of pancreatin to an aqueous potassium hydrogen phosphate solution, adjusting the pH of the resulting solution to 7.5 and adding distilled water to obtain a solution having a total volume of 1 L. *(USP Test Solution Method).* Simulated colonic fluid (SCF) is prepared by substituting pectinase for pancreatin in the above simulated intestinal fluid preparation.

Figure 9 demonstrates the unique properties of the hydrogel to response to environment pH. The hydrogel swells in the stomach and then collapse in response to gastric fluids excretion; the collapsed hydrogel re-swell in the small intestine and only degrades in the colon. The fibers do not swell (although they affect the viscosity see Example 20) while the hydrogel swells between 50 and 150 fold in GI tract environment (and between 500 to 3000 in water)

## Example 31 Human Satiety Study with CMC/CA Hydrogel

In order to measure the efficacy of CMC/CA hydrogel, a human study was conducted. The trial involved a total of 97 patients, who were blindly divided into three groups. At each mealtime, one group was administered a 2-g dose of the hydrogel under the study, while the rest were given placebo (cane sugar) of the same weight, texture and color as the hydrogel.

As shown in Table 9, following the method used, the subjects of each of the three groups were administered the CMC/CA hydrogel at only one of the three daily meals (breakfast, lunch or dinner) and were given placebo with the two other meals. For each meal the group receiving the hydrogel is indicated in Table 9 with an asterisk. The study was carried out on three days of the week, or more precisely once every four days (Day 1, Day 4, Day 7), so that each group received the hydrogel at each mealtime.

Table 9. Study Design

|           | Day 1                            | Day 4                            | Day 7                            |
|-----------|----------------------------------|----------------------------------|----------------------------------|
| Breakfast | Group A* Group B Group C         | Group B* Group A Group C         | Group C * Group A Group B        |
| Lunch     | Group B * Group A Group C        | Group C * Group A Group B        | Group A* Group B Group C         |
| Dinner    | Group C Group A Group B          | Group A* Group B Group C         | Group B* Group C Group A         |

The subjects were healthy volunteers (students, doctors, internal hospital personnel) and outpatients not affected by severe obesity. The study was conducted in Italy. In general, the subjects ate a very small breakfast and a small lunch. Dinner was the main meal of the day.

i) Study Design summary:

(1) 97 subjects with average BMI about 31
(2) CMC/CA hydrogel - 2 g
(3) Double blind, placebo controlled, cross-over design.

In order to examine the results from the trial, a descriptive statistical analysis was performed. This analysis showed that in some cases, the presence of outliers distorted the results. To isolate the effect of the outliers and to bring order to the results from the descriptive analysis, an inferential analysis was carried out.

In particular, a linear regression was performed to study the dependence between the incremental efficacy of hydrogel in relation to placebo and independent variables such as: BMI, sex, age, degree of obesity, time of administration (breakfast, lunch, or dinner) and degree of hunger prior to each mealtime.
The regression model therefore took the form:

$$\Delta E = \alpha + \beta_1 BMI + \beta_2 GEN + \beta_3 ETA' + \beta_4 OBE + \beta_5 PASTO + \beta_6 FAME + \varepsilon$$

Where:

- $\Delta E$ = incremental efficacy of hydrogel in comparison to placebo, calculated as the difference between the feeling of satiety immediately, 30 minutes and 60 minutes after the administration of hydrogel, and in comparison to the feeling of satiety after the administration of placebo;

- $\alpha$ = model intercept;

- $\beta_1 BMI$ = effect of BMI on incremental efficacy;

- $\beta_2 GEN$ = effect of the female gene on incremental efficacy. The GEN variable was created as a "dummy" which assumes the value 0 for male subjects and 1 for female subjects;

- $\beta_3 AGE$ = effect of age on incremental efficacy;

- $\beta_4 OBE$ = effect of degree of obesity on incremental efficacy;

- $\beta_5 MEAL$ = effect of the time of hydrogel administration on incremental efficacy. This variable assumes the value 1 if administered before breakfast, 2 if before lunch, and 3 if before dinner;

- $\beta_6 HUNGER$ = effect of the hunger sensation at the time of administration on incremental efficacy; and

- $\varepsilon$ = unexplained residual part of the model.

ii) Results:

(1) Excellent Safety profile
(2) Based on a self-assessment questionnaire in which each patient was asked to state their feeling of hunger before each meal and, subsequently, their feeling of satiety immediately, 30 minutes and 60 minutes after each meal, we noted a statistical significant increased satiety post meal time by ~16% in compare to placebo as measured by visual analog scales (VAS).

## Example 32 Determination of Elastic Modulus, Viscosity and Swelling Ratio

A. Determination of Elastic Modulus

The elastic modulus is determined using the method set forth below. The standard swelling media is distilled water unless specified otherwise. The swelling media could also be simulated gastric fluid (SGF) water mixture 1:8 or simulated intestinal fluid (SIF).
Unless specified otherwise, the concentration of the tested polymeric material in the media is 0.67%. In a 150 ml beaker 300mg of the tested polymeric material is added followed by the swelling media (45 mL). The beaker is covered with a Parafilm and the solution is stirred with a magnetic stirrer at room temperature (25 °C) for 60 min at high shear rate (600 RPM).
The tests are performed at room temperature by means of a parallel plate (25mm diameter) rheometer (ARES 509953791T, Rheometric Scientific, Inc.). An abrasive paper is fixed on the surface of each plate in order to prevent any slipping between the material and the plates during the test.
The swollen samples are placed between the plates of the rheometer in a cylindrical shape driven by means of a stainless steel ring of inner diameter of 25 mm (then the ring is removed).
The frequency sweep tests are performed at 1% of strain in a range between 0.1 and 50 rad/s.

The software (RSI Orchestrator by Rheometric Scientific Inc.) is able to acquire and store on the PC hard disk the signals coming from the rheometer transducer. G' and G" are calculated via the software by the following equations:

$$G'(\omega) = \frac{\tau_0}{\gamma_0} \cos \delta$$

$$G''(\omega) = \frac{\tau_0}{\gamma_0} \sin \delta$$

Where @ is the applied frequency, $\tau_0$ is the acquired torque, $\gamma_0$ is the applied strain and $\delta$ is the displacement.

B. Determination of Viscosity

Viscosity is determined using the method set forth below.

The standard swelling media is distilled water unless specified otherwise. The swelling media could also be simulated gastric fluid (SGF) water mixture 1:8 or simulated intestinal fluid (SIF). Unless specified otherwise, the concentration of the tested polymeric material in the media is 0.67%. In a 150 ml beaker 300mg of the tested polymeric material is added followed by the swelling media (45 mL). The beaker is covered with Parafilm and the solution is stirred with a magnetic stirrer at room temperature (25 °C) for 60 min at high shear rate (600 RPM).

The tests are performed at room temperature (25 °C) by means of a parallel plate (25mm diameter) rheometer (ARES 509953791T, Rheometric Scientific, Inc.). An abrasive paper is fixed on the surface of each plate in order to prevent any slipping between the material and the plate during the test.

The swollen samples are placed between the plates of the rheometer in a cylindrical shape driven by means of a stainless steel ring of inner diameter of 25mm (then the ring is removed).

The viscosity measurements are performed at the frequency sweep range of 0.05% to 10% strain. Unless specified otherwise, viscosity values presented herein are the measured result at 0.5% strain.

C. Determination of Swelling Ratio

The standard swelling media is distilled water unless specified otherwise. The swelling media could also be simulated gastric fluid (SGF) water mixture 1:8 or simulated intestinal fluid (SIF).

Unless specified otherwise, the concentration of the tested polymeric material in the media is 0.67%. In a 150 ml beaker 300mg of the tested polymeric material is added followed by the swelling media (45 mL). The beaker is covered with a Parafilm and the solution is stirred with a magnetic stirrer at room temperature (25 °C) for 60 min at high shear rate (600 RPM). The content of the beaker is transferred into a filter funnel and vacuum for 3 min at about 300 mBar is applied.

References

[1] Sannino A. et al., polymer 2005; 46:4676
[2] Silverstein R.M., et al. Spectrometric Identification of Organic Compounds, Wiley, 1991, pp 120-130.
[3] Peppas NA, edible polymer hydrogels in Medicine and Pharmacy; CRC Press, Boca Raton, Florida, 1987, p.29
[4] Coma V et al., Carbohydrate polymers 2003;51:265-271
[5] Xie XS and Liu Q, Starch 2004, 56:364

**Claims**

1. An edible polymer hydrogel comprising carboxymethylcellulose crosslinked with citric acid , for use in enhancing glycemic control in a subject, wherein said subject is diabetic, insulin resistant or at risk for developing insulin resistance or diabetes, and wherein the edible polymer hydrogel is orally administered to the subject

2. The edible polymer hydrogel for use according to claim 1 wherein the edible polymer hydrogel is administered to the subject with food or up to two hours prior to eating.

3. The edible polymer hydrogel for use according to claim 1 or 2 wherein the edible polymer hydrogel is administered with an enteric coating.

4. The edible polymer hydrogel for use according to any of claims 1 to 3 wherein the edible polymer hydrogel is administered in an amount sufficient to increase the viscosity and elastic modulus of the gastrointestinal content and slow gastric emptying and absorption of carbohydrates and fats in the subject's small intestine.

5. The edible polymer hydrogel for use according to any of claims 1 to 4 wherein the edible polymer hydrogel is administered in a tablet, a capsule, a sachet or as a component of a food.

6. The edible polymer hydrogel for use according to any one of claims 1 to 5, wherein the weight ratio of citric acid to carboxymethylcellulose is about 0.5% to about 5%.

7. A modified food or foodstuff comprising an edible polymer hydrogel, wherein said hydrogel comprises carboxymethylcellulose crosslinked with citric acid, and is in the form of dehydrated particulates individually coated with a moisture barrier.

8. The modified food or foodstuff of claim 7, wherein the food or foodstuff comprises carbohydrate and the edible polymer hydrogel replaces at least a portion of the carbohydrate content relative to the corresponding conventional food or foodstuff.

9. The modified food or foodstuff of claim 8, wherein the food or foodstuff comprises carbohydrate from a grain, a cereal or a starchy vegetable.

10. The modified foodstuff of claim 7 wherein the modified foodstuff is a modified flour.

11. The modified flour of claim 10, wherein the flour is selected from the group consisting of wheat flour, rice flour, corn flour, oat flour, potato flour, sorghum flour, millet flour, rye flour, barley flour, semolina flour, Atta flour, buckwheat flour, tapioca flour, brown rice flour, glutinous rice flour, noodle flour, pasta flour, chestnut flour, nut flours, chickpea flour, bean flour, pea flour, spelt flour and potato starch flour.

12. The modified food or foodstuff of claim 7, wherein the food or foodstuff is selected from the group consisting of hot and cold cereals, food bars, baked goods, baked good mixes, doughs for baked goods, pasta, dairy products, processed meat, beverages, meal replacement beverages, shakes and wet or dry pet food.

13. The modified food of claim 7, wherein the food is a beverage.

14. The beverage of claim 13, comprising citric acid, ascorbic acid, succinic acid, tartaric acid, phosphoric acid or monopotassium phosphate and optionally having a pH of 4 or less.

15. The beverage of claim 13 or 14, wherein said beverage is effervescent.

16. The modified food of claim 7 wherein the modified food is a beverage, a protein shake, ice cream, yogurt, frozen yogurt, frozen custard or soup.

17. The modified food of any one of claims 7 to 16 wherein said moisture barrier comprises fat, protein, sugar or a combination thereof.

18. The modified food or foodstuff of any one of claims 7 to 17, wherein the weight ratio of citric acid to carboxymethylcellulose is about 0.5% to about 5%.

19. The edible polymer hydrogel for use according to any of claims 1 to 6 or the modified food or foodstuff of any of claims 7 to 18, wherein the edible polymer hydrogel has

    a. a swelling ratio of 40 or greater; and
    b. an elastic modulus of at least 200 Pa.

**Patentansprüche**

1. Zum Verzehr geeignetes Polymerhydrogel, umfassend Carboxymethylcellulose, vernetzt mit Zitronensäure, zur Verwendung im Erhöhen der glykämischen Kontrolle bei einem Individuum, wobei das Individuum diabetisch, insulinresistent oder ein Risikoträger für die Entwicklung von Insulinresistenz oder Diabetes ist, und wobei das zum Verzehr geeignete Polymerhydrogel an das Individuum oral verabreicht wird.

2. Zum Verzehr geeignetes Polymerhydrogel zur Verwendung gemäß Anspruch 1, wobei das zum Verzehr geeignete Polymerhydrogel dem Individuum mit der Nahrung oder bis zu zwei Stunden vor dem Essen verabreicht wird.

3. Zum Verzehr geeignetes Polymerhydrogel zur Verwendung gemäß Anspruch 1 oder 2, wobei das zum Verzehr geeignete Polymerhydrogel mit einer enterischen Beschichtung verabreicht wird.

4. Zum Verzehr geeignetes Polymerhydrogel zur Verwendung gemäß einem der Ansprüche 1 bis 3, wobei das zum Verzehr geeignete Polymerhydrogel in einer ausreichenden Menge verabreicht wird, um die Viskosität und den elastischen Modul des gastrointestinalen Inhalts zu erhöhen und die Magenentleerung und Absorption von Kohlenhydraten und Fetten im Dünndarm des Individuums zu verlangsamen.

5. Zum Verzehr geeignetes Polymerhydrogel zur Verwendung gemäß einem der Ansprüche 1 bis 4, wobei das zum Verzehr geeignete Polymerhydrogel in einer Tablette, einer Kapsel, einem Beutelchen oder als einer Komponente eines Nahrungsmittels verabreicht wird.

6. Zum Verzehr geeignetes Polymerhydrogel zur Verwendung gemäß einem der Ansprüche 1 bis 5, wobei das Gewichtsverhältnis von Zitronensäure zu Carboxymethylcellulose etwa 0,5% zu etwa 5% beträgt.

7. Modifiziertes Nahrungsmittel oder Nährmittel, umfassend ein zum Verzehr geeignetes Polymerhydrogel, wobei das Hydrogel Carboxymethylcellulose, vernetzt mit Zitronensäure, umfasst und in Form dehydrierter Partikel vorliegt, die individuell mit einer Feuchtigkeitsbarriere beschichtet sind.

8. Modifiziertes Nahrungsmittel oder Nährmittel nach Anspruch 7, wobei das Nahrungsmittel oder Nährmittel Kohlenhydrat umfasst und das zum Verzehr geeignete Polymerhydrogel zumindest einen Teil des Kohlenhydratgehalts relativ zu dem entsprechenden herkömmlichen Nahrungsmittel oder Nährmittel ersetzt.

9. Modifiziertes Nahrungsmittel oder Nährmittel nach Anspruch 8, wobei das Nahrungsmittel oder Nährmittel Kohlenhydrat von einem Korn, einer Getreideflocke oder einer stärkehaltigen Gemüsepflanze umfasst.

10. Modifiziertes Nährmittel nach Anspruch 7, wobei das modifizierte Nährmittel ein modifiziertes Mehl ist.

11. Modifiziertes Mehl nach Anspruch 10, wobei das Mehl ausgewählt ist aus der Gruppe, bestehend aus Weizenmehl, Reismehl, Maismehl, Hafermehl, Kartoffelmehl, Sorghummehl, Hirsemehl, Roggenmehl, Gerstenmehl, Grießmehl, Atta-Mehl, Buchweizenmehl, Tapiokamehl, Vollkornreismehl, Klebreismehl, Nudelmehl, Pastamehl, Maronenmehl, Nussmehl, Kichererbsenmehl, Bohnenmehl, Erbsenmehl, Dinkelmehl und Kartoffelstärkemehl.

12. Modifiziertes Nahrungsmittel oder Nährmittel nach Anspruch 7, wobei das Nahrungsmittel oder Nährmittel ausgewählt ist aus der Gruppe, bestehend aus heißen und kalten Getreideflocken, Nahrungsriegeln, Backwaren, Backmischungen, Hefen für Backwaren, Pasta, Milchprodukten, verarbeitetem Fleisch, Getränken, Nahrungsmittelersatzgetränken, Mixgetränken und feuchtem oder trockenem Tierfutter.

13. Modifiziertes Nahrungsmittel nach Anspruch 7, wobei das Nahrungsmittel ein Getränk ist.

14. Getränk nach Anspruch 13, umfassend Zitronensäure, Ascorbinsäure, Succinsäure, Weinsäure, Phosphorsäure oder Monokaliumphosphat und optional einen pH von 4 oder weniger aufweisend.

15. Getränk nach Anspruch 13 oder 14, wobei das Getränk sprudelnd ist.

16. Modifiziertes Nahrungsmittel nach Anspruch 7, wobei das modifizierte Nahrungsmittel ein Getränk, ein Protein-Mixgetränk, Speiseeis, Joghurt, Joghurteis, Cremeeis oder Suppe ist.

**17.** Modifiziertes Nahrungsmittel nach einem der Ansprüche 7 bis 16, wobei die Feuchtigkeitsbarriere Fett, Protein, Zucker oder eine Kombination daraus umfasst.

**18.** Modifiziertes Nahrungsmittel oder Nährmittel nach einem der Ansprüche 7 bis 17, wobei das Gewichtsverhältnis von Zitronensäure zu Carboxymethylcellulose etwa 0,5% zu etwa 5% beträgt.

**19.** Zum Verzehr geeignetes Polymerhydrogel zur Verwendung gemäß einem der Ansprüche 1 bis 6, oder das modifizierte Nahrungsmittel oder Nährmittel nach einem der Ansprüche 7 bis 18, wobei das zum Verzehr geeignete Polymerhydrogel ausweist:

    a. ein Quellverhältnis von 40 oder größer; und
    b. einen Elastizitätsmodul von mindestens 200 Pa.

**Revendications**

**1.** Hydrogel polymère comestible comprenant de la carboxyméthylcellulose réticulée par de l'acide citrique, pour une utilisation dans l'amélioration du contrôle glycémique chez un sujet, ledit sujet étant diabétique, insulinorésistant ou à risque de développer une insulinorésistance ou un diabète, et ledit hydrogel polymère comestible étant administré par voie orale au sujet.

**2.** Hydrogel polymère comestible pour une utilisation selon la revendication 1, dans lequel l'hydrogel polymère comestible est administré au sujet avec un aliment ou jusqu'à deux heures avant la prise d'alimentation.

**3.** Hydrogel polymère comestible pour une utilisation selon l'une des revendications 1 ou 2, dans lequel l'hydrogel polymère comestible est administré avec un enrobage entérique.

**4.** Hydrogel polymère comestible pour une utilisation selon l'une quelconque des revendications 1 à 3, dans lequel l'hydrogel polymère comestible est administré dans une quantité suffisante pour augmenter la viscosité et le module élastique du contenu gastrointestinal et pour ralentir la vidange gastrique et l'absorption de glucides et de graisses dans l'intestin grêle du sujet.

**5.** Hydrogel polymère comestible pour une utilisation selon l'une quelconque des revendications 1 à 4, dans lequel l'hydrogel polymère comestible est administré sous la forme d'un comprimé, d'une gélule, d'un sachet ou comme composant d'un aliment.

**6.** Hydrogel polymère comestible pour une utilisation selon l'une quelconque des revendications 1 à 5, dans lequel le rapport en poids de l'acide citrique à la carboxyméthylcellulose est d'environ 0,5 % à environ 5 %.

**7.** Aliment ou produit alimentaire modifié comprenant un hydrogel polymère comestible, ledit hydrogel comprenant de la carboxyméthylcellulose réticulée par de l'acide citrique, et se trouvant sous la forme de particules déshydratées enrobées individuellement par une barrière contre l'humidité.

**8.** Aliment ou produit alimentaire modifié selon la revendication 7, dans lequel l'aliment ou le produit alimentaire comprend un glucide et l'hydrogel polymère comestible remplace au moins une partie de la teneur en glucide par rapport à l'aliment ou au produit alimentaire classique correspondant.

**9.** Aliment ou produit alimentaire modifié selon la revendication 8, dans lequel l'aliment ou le produit alimentaire comprend un glucide provenant de grains, de céréales ou de légumes amylacés.

**10.** Produit alimentaire modifié selon la revendication 7, dans lequel le produit alimentaire modifié est une farine modifiée.

**11.** Farine modifiée selon la revendication 10, dans lequel la farine est choisie dans le groupe consistant en farine de blé, farine de riz, farine de maïs, farine d'avoine, farine de pomme de terre, farine de sorgho, farine de millet, farine de seigle, farine d'orge, semoule de blé tendre, farine non classée, farine de sarrasin, farine de tapioca, farine de riz brun, farine de riz gluant, farine de nouilles, farine de pâtes alimentaires, farine de châtaigne, farines de noix, farine de pois chiche, farine de haricot, farine de pois, farine d'épeautre et fécule de pomme de terre.

**12.** Aliment ou produit alimentaire modifié selon la revendication 7, dans lequel l'aliment ou le produit alimentaire est choisi dans le groupe consistant en céréales chaudes et froides, barres alimentaires, produits de boulangerie-pâtisserie, mélanges de produits de boulangerie-pâtisserie, pâtes pour produits de boulangerie-pâtisserie, pâtes alimentaires, produits laitiers, viande transformée, boissons, boissons de substitut de repas, milk-shakes et aliments pour animaux de compagnie humides ou secs.

**13.** Aliment modifié selon la revendication 7, dans lequel l'aliment est une boisson.

**14.** Boisson selon la revendication 13, comprenant de l'acide citrique, de l'acide ascorbique, de l'acide succinique, de l'acide tartrique, de l'acide phosphorique ou du phosphate monopotassique et facultativement ayant un pH de 4 ou moins.

**15.** Boisson selon l'une des revendications 13 ou 14, dans lequel ladite boisson est effervescente.

**16.** Aliment modifié selon la revendication 7, dans lequel l'aliment modifié est une boisson, un milk-shake protéiné, une crème glacée, un yaourt, un yaourt glacé, une glace aux oeufs ou une soupe.

**17.** Aliment modifié selon l'une quelconque des revendications 7 à 16, dans lequel ladite barrière contre l'humidité comprend une graisse, une protéine, un sucre ou une combinaison de ceux-ci.

**18.** Aliment ou produit alimentaire modifié selon l'une quelconque des revendications 7 ou 17, dans lequel le rapport en poids de l'acide citrique à la carboxyméthylcellulose est d'environ 0,5 % à environ 5 %.

**19.** Hydrogel polymère comestible pour une utilisation selon l'une quelconque des revendications 1 à 6 ou aliment ou produit alimentaire modifié selon l'une quelconque des revendications 7 à 18, dans lequel l'hydrogel polymère comestible a :

    a. un rapport de gonflement de 40 ou plus ; et
    b. un module élastique d'au moins 200 Pa.

Cap

Hydrogel powder

Water

Figure 1

Cap

Water
+
Hydrogel

Figure 2

Figure 3

Figure 4

- Psyllium 1% in SGF/Water 1/8
- Guar Gum 1% in SGF/Water1/8
- Glucomannan 1% in SGF/Water 1/8
- CMC/CA (coarse) in SGF/Water 1/8
- CMC/CA (fine) 1% in SGF/Water 1/8

Viscosity @ 0.5 s⁻¹ (Pa*s)

No Mixing 1h    Mild Mixing 1h (60 rpm)    Fast Mixing 4h (600 rpm)

Figure 5

Figure 6

Figure 7

Figure 8

Figure 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5336486 A, Acharya
- US 5063073 A, Kratochvil
- US 5654028 A, Christensen
- US 6426077 B, Grace
- US 5405616 A
- US 6103269 A, Wounderlich
- US 5100688 A
- US 5211976 A
- US 5219599 A
- US 5665234 A
- US 5665419 A
- US 7601705 B
- US 5776887 A
- US 5695803 A
- US 6018033 A, Park K.
- US 5750585 A
- US 6271278 B
- US 2004192582 A, Burnett D.R.
- US 20050245957 A
- US 20060142794 A
- WO 2006047882 A
- WO 2006070337 A
- WO 2009021701 A

- US 20080227944 A
- US 4570629 A, Widra
- US 5153174 A, Band
- US 5489261 A, Franzblau
- US 5863984 A, Doillon
- US RE33997 E, Kuamz
- US 4264155 A, Miyata
- US 5948429 A, Bell
- US 5859077 A
- US 6168762 B, Reichman
- US 5676967 A, Williams
- US 5869080 A, McGregor
- US 4837285 A, Berg
- US 4950485 A, Akhtar
- US 4971954 A, Brodsky
- GB 756082 A
- US 4820533 A
- EP 0090559 A
- WO 9523520 A
- US 20060286264 A
- US 20020146495 A
- EP 0471558 A

**Non-patent literature cited in the description**

- Technical report series 894: Obesity: Preventing and managing the global epidemic. World Health Organization, 2000
- Clinical Guidelines on the Identification, Evaluation, and Treatment of Overweight and Obesity in Adults--The Evidence Report. Obes Res. National Institutes of Health, September 1998, vol. 6, 51S-209S
- Clinical Guidelines on the Identification, Evaluation, and Treatment of Overweight and Obesity. National Institutes of Health, September 1998
- Clinical Guideline 43: Obesity: The prevention, identification, assessment and management of overweight and obesity in adults and children. National Institute for Health and Clinical Excellence, 2006
- BELL et al. *Am J Clin Nutr,* 1998, vol. 67, 412-20
- ROLLS. *Am J Clin Nutr,* 1999, vol. 70, 448-455
- ELLO-MARTIN. *Am J Clin Nutr,* 2007, vol. 85, 1465-7
- GREENE. *Obesity,* 2006, vol. 14, 1795-1801

- BARBARA ROLLS. Volumetrics Eating Plan. *Harper Collins,* 2007
- I.T. NORTON ; W.J. FRITH ; S. ABLETT. Fluid gels, mixed fluid gels and satiety. *Food Hydrocolloids,* March 2006, vol. 20, 229-239
- MARCIANI, L. ; GOWLAND, P. A. ; SPILLER, R. C. ; MANOJ, P. ; MOORE, R. J. ; YOUNG, P. ; FILLERY-TRAVIS, A. J. Effect of meal viscosity and nutrients on satiety, intragastric dilution, and emptying assessed by MRI. *American Journal of Physiology Gastrointestinology and Liver Physiology,* 2001, vol. 280, G1227-G1233
- JUVONEN, K. R. et al. Viscosity of Oat Bran-Enriched Beverages Influences Gastrointestinal Hormonal Responses in Healthy Humans. *Journal of Nutrition,* 2009, vol. 139 (3), 461-466
- HLEBOWICZ, J. et al. Effect of commercial breakfast fiber cereals compared with corn flakes on postprandial blood glucose, gastric emptying and satiety in healthy subjects: a randomized blinded crossover trial. *Nutrition Journal,* 2007, vol. 6, 22

- **TARNOW, L ; GROOP ; HADJADJ ; KAZEEM ; CAMBIEN ; MARRE ; FORSBLOM ; PARVING et al.** European rational approach for the genetics of diabetic complications-EURAGEDIC: patient populations and strategy. *Nephrology, dialysis, transplantation,* 2008, vol. 23 (1), 161-8
- **ADAMS, D.D.** Autoimmune destruction of pericytes as the cause of diabetic retinopathy. *Clinical ophthalmology,* 2008, vol. 2 (2), 295-8
- **HUANG, ES ; BROWN ; EWIGMAN ; FOLEY ; MELTZER.** Patient perceptions of quality of life with diabetes-related complications and treatments. *Diabetes care,* 2007, vol. 30 (10), 2478-83
- **HEATON K W ; MARCUS S N ; EMMETT P M ; BOLTON C H.** Particle size of wheat, maize, and oat test meals: effects on plasma glucose and insulin responses and on the rate of starch digestion in vitro. *Am. J. Clin. Nutr.,* 1988, vol. 47, 675-682
- Shokumotsu Sen-i (Dietary Fiber). Asakura-shoten, 1997, 412-420
- *Kagaku to Seibutsu (Chemistry and Biology),* 1980, vol. 18, 95-105
- **CHEN JUN et al.** Gastric retention properties of superporous hydrogel composite. *J. Controlled Release,* 2000, vol. 64, 39-51
- **BARDOS.** *Behav Neurosci.,* 1997, vol. 111, 834-844
- **BARDOS.** *Physiol Behav.,* 2001, vol. 74, 407-413
- **SAMELSON SL et al.** *J R Soc Med,* 1985, vol. 78, 230-233
- **MALJAARS PW ; PETERS HP ; MELA DJ ; MASCLEE AA.** Ileal brake: a sensible food target for appetite control. *Physiol Behav.,* 20 October 2008, vol. 95 (3), 271-81
- **JACOBS DR, JR. ; MEYER KA ; KUSHI LH ; FOLSOM AR.** Whole-grain intake may reduce the risk of ischemic heart disease death in postmenopausal women: the Iowa Women's Health Study. *Am J Clin Nutr.,* 1998, vol. 68 (2), 248-257
- **RIMM EB ; ASCHERIO A ; GIOVANNUCCI E ; SPIEGELMAN D ; STAMPFER MJ ; WILLETT WC.** Vegetable, fruit, and cereal fiber intake and risk of coronary heart disease among men. *JAMA,* 1996, vol. 275 (6), 447-451
- **KEENAN JM ; PINS JJ ; FRAZEL C ; MORAN A ; TURNQUIST L.** Oat ingestion reduces systolic and diastolic blood pressure in patients with mild or borderline hypertension: a pilot trial. *J Fam Pract.,* 2002, vol. 51 (4), 369
- **TROCK B ; LANZA E ; GREENWALD P.** Dietary fiber, vegetables, and colon cancer: critical review and meta-analyses of the epidemiologic evidence. *J Natl Cancer Inst.,* 1990, vol. 82 (8), 650-661
- **KORZENIK JR.** Case closed? Diverticulitis: epidemiology and fiber. *J Clin Gastroenterol.,* 2006, vol. 40 (3), 112-116
- **KUMAR, C. M. et al.** Modulatory effect of butyric acid-a product of dietary fiber fermentation in experimentally induced diabetic rats. *The Journal of Nutritional Biochemistry,* vol. 13, 522-527
- **TOMLIN. J. et al.** *Gut.,* 1993, vol. 34 (9), 1177-1181
- **SANNINO A. et al.** *polymer,* 2005, vol. 46, 4676
- **SILVERSTEIN R.M. et al.** Spectrometric Identification of Organic Compounds. Wiley, 1991, 120-130
- edible polymer hydrogels. **PEPPAS NA.** Medicine and Pharmacy. CRC Press, 1987, 29
- **COMA V et al.** *Carbohydrate polymers,* 2003, vol. 51, 265-271
- **XIE XS ; LIU Q.** *Starch,* 2004, vol. 56, 364